# FASCICULE DE BREVET EUROPEEN

(11) **EP 1 163 522 B1**
(45) Date de publication et mention de la délivrance du brevet: **22.11.2006**
(21) Numéro de dépôt: 00912720.0
(22) Date de dépôt: 20.03.2000
(51) Int. Cl.: G01N 33/569, G01N 33/68, G01N 33/50, G01N 33/564, G01N 33/566, G01N 33/574, A61K 39/00

(54) **DETECTION D'UNE ACTIVITE SUPERANTIGENIQUE INDUITE PAR MSRV-1 DANS UN ECHANTILLON BIOLOGIQUE**
NACHWEIS EINER MSRV-1 INDUZIERTEN SUPERANTIGENAKTIVITÄT IN EINER BIOLOGISCHEN PROBE
METHOD FOR DETECTING MSRV-1 INDUCED SUPERANTIGEN ACTIVITY IN A BIOLOGICAL SAMPLE

(30) Priorité: 19.03.1999 FR 9903622; 28.10.1999 FR 9913755
(43) Date de publication de la demande: 19.12.2001
(73) Titulaire: BIO MERIEUX, 69280 Marcy l'Etoile (FR)
(72) Inventeur: PERRON, Hervé, F-69005 Lyon (FR); LAFONT, Monique, F-75015 Paris (FR)
(74) Mandataire: Guerre, Dominique
(86) Numéro de dépôt international: PCT/FR2000/000691
(87) Numéro de publication internationale: WO 2000/057185

(56) Documents cités:
- WO-A-98/26747
- WO-A1-94/28138
- WO-A2-99/02666
- BIOLOGICAL ABSTRACTS, Philadelphia PA USA; abstract no. PREV199800529131, abrégé XP002139048 & H. PERRON ET AL. : "Retrovirus, cytotoxic molecules including superantigen and multiple sclerosis: Epiphenomenon or new avenue of research " JOURNAL OF NEUROIMMUNOLOGY, vol. 90, no. 1, 23 août 1998 (1998-08-23), page 68 Montreal Canada
- BIOLOGICAL ABSTRACTS Philadelphia PA USA; abstrcat no. PREV199699194041, abrégé XP002139428 & B.A. TORRES ET AL.: "HIV encodes for its own CD4 T-cell superantigen mitogen " BIOCHEMICAL AND BIOPHYSICAL RESEARCH COMMUNICATIONS, vol. 225, no. 2, 1996, pages 672-678, New York NY USA
- CHEMICAL ABSTRACTS, vol. 119, no. 7, 16 août 1993 (1993-08-16) Columbus, Ohio, US; abstract no. 69990, XP002123380 & E. JOUVIN-MARCHE ET AL.: "Identification of endogenous superantigens reacting with T cell receptor of V.beta.17 chains" EOS RIV IMMUNOL IMMUNOFARMACOL, vol. 13, no. 1, 1993, pages 74-75, Paris
- CHEMICAL ABSTRACTS, vol. 119, no. 21, 22 novembre 1993 (1993-11-22) Columbus, Ohio, US; abstract no. 223499, XP002123381 & N. LABRECQUE ET AL.: "T-cell recognition of superantigens: another view." RES IMMUNOL, vol. 144, no. 3, 1993, Montreal
- YOON K.S. ET AL: 'EXPERIMENTAL ACUTE HEMATOGENEOUS OSTEOMYELITIS IN MICE. II. INFLUENCE OF STAPHYLOCOCCUS AUREUS INFECTION ON T-CELL IMMUNITY' JOURNAL OF ORTHOPAEDIC RESEARCH vol. 17, no. 3, Mai 1999, pages 382 - 391

## Description

La sclérose en plaques (SEP) est une maladie chronique du système nerveux central de l'homme, évoluant par succession de phases de rémission et de poussée ou selon une progression régulière, dont la caractéristique anatomopathologique consiste en la formation de zones de démyélinisation bien délimitées dans la substance blanche du cerveau et de la moelle épinière.

Au niveau histologique, ces zones présentent au stade précoce du processus lésionnel, une dégradation de la myéline péri-axonale associée à une atteinte des cellules gliales responsable de cette démyélinisation. Une activation macrophagique inflammatoire impliquant les cellules microgliales (macrophages tissulaires résidants du système nerveux central), ainsi que, probablement, des macrophages provenant de monocytes sanguins infiltrés, est associée à ce processus de démyélinisation et contribue à la destruction des feuillets myélinisés. Au centre de la zone démyélinisée, une déplétion relative en cellules gliales est retrouvée alors qu'une prolifération d'astrocytes se développe à la périphérie et peut envahir la plaque démyélinisée pour générer une plaque fibreuse ou gliotique. Ces structures sclérotiques sont à l'origine du nom donné à la maladie.

Une autre caractéristique de ces plaques est leur association quasi systématique avec un élément vasculaire autour duquel elles se développent.

Au niveau histologique, on observe une altération fréquente de la barrière hémato-encéphalique (BHE) constituée par l'endothélium capillaire. Un des éléments déterminants dans le maintien de la BHE est constitué par la présence sous-jacente d'extensions cytoplasmiques des astrocytes, appelées pieds astrocytaires. Vraisemblablement, les pieds astrocytaires induisent la formation ou permettent le maintien de structures de jonction étanches qui assurent la cohésion de la barrière endothéliale capillaire concrétisant la BHE. Or, différents modèles pathologiques font état de l'altération de la BHE et d'une déplétion des pieds astrocytaires.

Par ailleurs, dans le processus lésionnel de la SEP, l'altération de la BHE contribue à amplifier la réponse inflammatoire associée, par l'afflux de cellules lymphoïdes provenant de la circulation sanguine. La contribution de l'inflammation associée aux cellules immunitaires est importante dans la SEP et participe au processus lésionnel.

L'étiologie de la SEP est la source d'un débat d'actualité parce que la maladie pourrait avoir des causes multiples. Des arguments ont été avancés en faveur d'une hypothèse bactérienne, virale ou auto-immune.

Les rétrovirus sont des candidats potentiellement intéressants pour l'étude étiologique de la sclérose en plaques, par analogie avec une maladie ovine très proche de la SEP, induite chez le mouton par un rétrovirus exogène : le virus MAEDI-VISNA. L'infection expérimentale de moutons par inoculation intra-ventriculaire de souches neurovirulentes du virus VISNA a permis d'établir la responsabilité de ce virus dans la génèse de l'infection démyélinisante du mouton.

De nombreux travaux ont été réalisés pour étayer l'hypothèse d'une étiologie virale de la maladie et la découverte que HTLV-1, un oncovirus, est associé à une myélopathie chronique et progressive, la Paraparésie Spastique Tropicale (PST), a relancé l'intérêt pour les virus, sans qu'il ait pu être établi un lien de causalité entre virus et sclérose en plaques.

Récemment, les travaux de H. Perron et al. (Res. Virol. 1989 ; 140, 551-561 ; " Current Concepts in Multiple Sclerosis " Wiethöler et al., eds. Amsterdam, Elsevier, 1991, 111-116 et the Lancet 1991 ; 337, 862-863) ont permis à partir d'une ponction lombaire de liquide céphalorachidien d'un patient SEP d'isoler une lignée non immortalisée de cellules non lymphoïdes et de mettre en évidence la présence d'un virus, présentant les caractéristiques d'un rétrovirus et montrant en particulier un pic correspondant à une activité transcriptase inverse, dans le surnageant de culture de cette lignée. Plus récemment encore, ces mêmes auteurs ont obtenu à partir de ce pic d'activité transcriptase inverse un ADN complémentaire qui correspondait au gène *pol* codant pour l'enzyme RT (transcriptase inverse ou reverse transcriptase). Ce rétrovirus, appelé MSRV par les auteurs, a notamment été caractérisé au niveau génomique dans la demande de brevet PCT WO 99/02666. L'analyse phylogénique, par comparaison de la séquence de la région *pol* de MSRV avec d'autres séquences *pol* disponibles dans les bases de données a permis de montrer que MSRV est proche de la famille ERV-9 (endogenous retrovirus-9). De plus, Perron et al. (J. Neuro immunol. 1998; 90, 68, poster abstract 380) divulgue la notion que le rétrovirus MSRV pourrait avoir une activité superantigénique associée à la sclérose en plaques.

Par ailleurs, F. Beseme et al. dans la demande de brevet PCT WO 99/02696 ont criblé une banque d'ADNc à l'aide d'une sonde Ppol-MSRV et détecté des clones chevauchants qui leur ont permis de reconstruire un ARN génomique putatif de 7582 nucléotides. Cet ARN génomique présente une structure R-U5-*gag*-*pol*-*env*-U3-R caractéristique des rétrovirus et une interrogation de plusieurs bases de données a permis de montrer qu'il existe une quantité importante de séquences génomiques (ADN) apparentées dans le génome humain qui sont retrouvées sur plusieurs chromosomes. Les auteurs ont ainsi mis en évidence l'existence de structures partielles de type rétroviral dans le génome humain et envisagé leur rôle potentiel dans des maladies auto-immunes, comme la sclérose en plaques. Cette nouvelle famille de rétrovirus endogènes a été dénommée HERV-W, en raison de ses caractéristiques structurelles. L'analyse phylogénique dans la région *pol* a montré que la famille HERV-W est phylogéniquement proche des familles ERV-9 et RTVL-H et appartient donc à la famille des rétrovirus endogènes de type 1. Par ailleurs, l'analyse phylogénique de la trame de lecture ouverte de *env* montre qu'elle est plus proche des rétrovirus simiens de type D et des rétrovirus de la réticuloendothéliose aviaire que des rétrovirus mammifères de type C, suggérant une structure de génome chimérique C/D. Les analyses des arbres phylogéniques montrent que les familles ERV-9 et HERV-W dérivent de deux vagues d'insertion indépendantes.

La protéine de l'enveloppe de MSRV-1 codée par le gène *env* de MSRV-1 et ses variants présente une très forte homologie avec la protéine env de HERV-W codée par l'ARN exprimé dans le placenta humain tel que décrit dans la demande de brevet WO-99/02696 au nom de la Demanderesse.

Le rétrovirus MSRV est génétiquement apparenté à la famille HERV-W.

Les variants de MSRV-1 ont un gène env qui présente avantageusement une homologie d'au moins 90%, de préférence au moins 95%, voire de 98% avec celui de MSRV-1.

Tous ces éléments plaident en faveur de l'implication d'éléments rétroviraux dans la sclérose en plaques.

Il semble, par ailleurs, maintenant probable que des manifestations auto-immunes peuvent être induites par l'expression de superantigènes (SAgs).

Les superantigènes sont des molécules susceptibles de se lier à des molécules de classe II du Complexe Majeur d'Histocompatibilité (CMH) et à des séquences peptidiques caractéristiques de certaines familles de récepteurs des cellules T (Vβ). Ces superantigènes activent un grand nombre de clones T, indépendamment du peptide antigénique reconnu par leur TCR (récepteur des cellules T) en association avec le CMH de la cellule présentatrice d'antigènes. La conséquence de cette activation est une prolifération polyclonale ou une induction d'anérgie, voire d'apoptose, dans la population lymphocytaire T porteuse de ce Vβ. Les superantigènes sont des produits d'expression de micro-organismes, tels que bactéries et rétrovirus exogènes ou endogènes.

Certaines molécules ayant des propriétés proches de certains effets connus pour les superantigènes peuvent aussi être responsables de processus immunopathologiques majeurs et sont appelées superantigènes-like. Dans la suite de la présente description, ils sont inclus dans le terme superantigène.

Le récepteur T (TCR) présent à la surface des lymphocytes et impliqué dans la reconnaissance d'un antigène ou d'un superantigène est constitué de deux chaînes, une chaîne α de 40 à 50 kDa et une chaîne β de 35 à 47 kDa, liées entre elles par un pont disulfure. Chaque chaîne polypeptidique comprend deux domaines d'environ 110 acides aminés chacun, qui sont organisés comme les domaines des immunoglobulines. Ils sont ancrés dans la membrane plasmique par un peptide transmembranaire et possèdent une courte queue cytoplasmique. La différence de poids moléculaire entre les deux chaînes α et β est due à la présence d'une chaîne carbohydratée en position N-terminale de la chaine α. La variabilité de séquence en acides aminés réside dans le domaine N-terminal de chaque polypeptide α et β, homologues des domaines variables des immunoglobulines. Chaque domaine est codé par un réarrangement de gènes V, D et J pour la chaîne β et V et J pour la chaîne α. L'analyse des séquences de ces différents domaines TCR V révèle une grande variabilité qui correspond aux régions hypervariables des immunoglobulines (CDRs). (Roitt 1. et al., Immunology 3^{rd} edition, Mosby, England).

Le réarrangement des gènes du TCR est similaire à celui des gènes des immunoglobulines. La diversité des TCRs provient d'une recombinaison génétique entre les segments V, D et J. Les gènes Vβs, incluant les gènes D, J et C, sont groupés ensemble à l'exception de Vβ14 qui est présent à l'extrémité 3' du locus. Une diversité extensive est générée pendant les procédés de regroupement des régions V-D-J, mais aussi V-J et V-D-D-J.

Un superantigène est capable d'activer les lymphocytes T de façon non spécifique, contrairement à un antigène. Un superantigène est capable de se fixer conjointement aux molécules du CMHII présentes à la surface des cellules présentatrices de l'antigène et aux molécules Vβs du récepteur T présent à la surface des cellules T. La chaîne Vβ du TCR fixe le superantigène à l'extérieur du site spécifique d'antigène du TCR, mais cela est suffisant pour activer la cellule T. Cette fixation entraîne une induction de signaux intracellulaires dans la cellule T. Ces cascades de signaux intracellulaires induisent soit la prolifération de la cellule T portant le Vβ d'intérêt, soit une anergie ou une apoptose de la cellule. Ainsi, en fonction des conditions expérimentales, l'effet sur la sous population des lymphocytes T activée peut être un effet de prolifération, d'anergie ou d'apoptose polyclonale. Contrairement à une réponse T à un antigène classique, la stimulation des lymphocytes T est donc polyclonale et non oligoclonale, voire monoclonale (Scherer et al., 1993 Annu. Rev. Cell Biology 9 : 101-128). Cette induction de prolifération cellulaire, d'anergie ou d'apoptose dépend de plusieurs paramètres qui agissent en combinaison. Elle dépend en particulier de la concentration du superantigène et de l'existence de rencontres préalables de stimulations analogues ciblant le même Vβ par le système immunitaire du donneur de lymphocytes T. La mise en anergie ou en apoptose peut suivre entre autre une stimulation ou activation prolongée des cellules T. Ainsi un superantigène a un effet induisant une variation positive ou négative d'une sous-population de cellules T d'un Vβ" x " défini. Un superantigène ciblant un déterminant antigénique Vβ" x "donné va interagir avec toute la sous-population lymphocytaire portant ce Vβ. L'effet induit par cette interaction couplée avec celle ciblant le CMHII des cellules présentatrices de l'antigène (APCs) est une variation significative du pourcentage des lymphocytes Vβ" x " par rapport aux autres lymphocytes T Vβ non " x ". Cette variation est typiquement soit une prolifération, soit une déplétion (Bernal A et al., 1999 J Clin Immunol 19 : 149 ; Li H et al., 1999 Annu Rev lmmunol 17 : 435 ; Girgis et al., 1999 J Exp Med 189 : 265 ; Lavoie et al., Immunol Rev 1999 168 : 257 ; Cornwell WD et Rlgers TJ, Immunology 1999 96 : 193 ; Wang ZQ et al., Immunology 1998 94 : 331 ; Maier CC et al., PNAS 1998 95 : 4499 ; Michie CA et Cohen J, Trends Microbiol 1998 6 : 61 ; La Bon A et al., Int Immunol 1999 11 : 373 ; Shen X et Konig R, Int Immunol 1998 10 : 247 ; Noble A et al., J Immunol 1998 160 : 559 ; Yang Y et al., lnt Immunol 1998 10 : 175 ; Renno T et al., J Immunol 1999 162 : 6312 ; Roitt I et al., Immunology third edition, Mosby).

Par ailleurs, lorsque l'agent codant pour un superantigène donné est mis en présence de lymphocytes T, et non seulement en présence de la molécule purifiée portant l'activité superantigénique, l'effet immunologique résultant correspond à la superposition d'un effet superantigénique et de stimulations antigéniques variées causées par les autres antigènes de l'agent entier. Il est à noter que, contrairement à la stimulation superantigénique, ces dernières peuvent différer en fonction du HLA de classe II du donneur de lymphocytes ou du patient dans un contexte pathologique. Ceci a pour conséquence que la prolifération ou la déplétion T Vβ" x " s'accompagne d'un profil de réactivité (prolifération ou inhibition) d'autres sous populations Vβs non " x ", éventuellement variables selon le HLA II ; ce profil étant défini par la nature des antigènes associés à l'agent ou à plusieurs agents (dans le cas d'une cascade conduisant à l'activation d'un rétrovirus endogène codant pour le superantigène exprimé dans ces conditions). Dans ce dernier cas, deux superantigènes peuvent être exprimés, si l'agent inducteur en produit un et si l'infection d'une cellule cible par celui ci réactive un rétrovirus endogène qui en produit alors un second. Ces données confirment donc la nécessité d'évaluer un profil complet de réponse T en fonction du Vβ lorsque l'on étudie un contexte " naturel " d'infection/réactivation et non une molécule superantigénique purifiée hors contexte.

La Demanderesse a maintenant montré que l'expression d'un superantigène ou d'une protéine ayant certains effets de type superantigène (superantigène-like) est associée à un état pathologique, par exemple un état associé à la sclérose en plaques.

L'activité superantigénique est induite directement ou indirectement par un agent effecteur, tel qu'une protéine ou un micro-organisme ou un agent pathogène, en particulier un rétrovirus (MSRV-1) et/ou un agent pathogène (MSRV-2), et en particulier un épitope compris dans une protéine env d'un rétrovirus MSRV-1.

De plus, la Demanderesse a également mis en évidence une production stimulée de cytokines, telles que l'IL-6, dans des cellules mononucléées sanguines provenant de donneurs sains et mises en contact avec un extrait de surnageant ou une fraction d'un extrait de surnageant de culture cellulaire SEP.

La demanderesse a aussi mis en évidence un profil de réponse T, en fonction des Vβs à un ou des agent(s) associé(s) à l'expression d'une molécule de type superantigénique.

Enfin, la Demanderesse a observé que les mêmes extraits provenant de patients SEP et/ou infectés par le rétrovirus MSRV-1 induisait une mort par apoptose significativement élevée dans les mêmes cellules mononucléées sanguines.

La Demanderesse a donc développé un procédé pour mettre en évidence les effets précités dans un échantillon biologique.

Les critères requis pour établir qu'une protéine ou qu'un micro-organisme est ou contient un superantigène ou une protéine ayant certains effets de type superantigène sont :
(i) la capacité de la protéine ou du micro-organisme à induire l'expansion ou la perte de certaines familles de lymphocytes porteuses au niveau de leur TCR d'une chaîne Vβ particulière, et
(ii) une activation des Vβs indépendante de l'haplotype du CMH de classe II.

II convient de noter que lorsque l'on parle d'activité superantigénique dans la présente invention, ceci signifie indifféremment l'expression d'un superantigène ou d'une protéine ayant certains effets de type superantigène (SAg-like).

L'invention concerne un procédé de détection de la sclérose en plaques ou d'une prédisposition à développer la sclérose en plaques, dans un échantillon biologique, procédé selon lequel

on détecte une activité superantigénique induite par le produit d'expression du gène *env* de MSRV-1 ou d'un fragment du gène *env* de MSRV-1, ledit gène *env* de MSRV-1 comprenant ou consistant en la séquence référencée en SEQ ID NO :1, ou

on détecte une activité superantigénique induite par le produit d'expression du gène *env* d'un variant de MSRV-1 ou d'un fragment du gène *env* dudit variant, ledit gène *env* dudit variant présentant une homologie d'au moins 90% avec le gène *env* de MSRV-1,
en mettant en évidence une expansion ou une perte de lymphocytes choisis parmi les lymphocytes porteurs d'un déterminant Vβ16 et/ou Vβ17 et les lymphocytes porteurs d'un déterminant Vβ7, ladite expansion ou ladite perte étant d'au moins 31%.

Selon une variante avantageuse, on met en évidence une expansion de lymphocytes porteurs d'un déterminant Vβ 16.

Selon une variante avantageuse, on met en évidence un profil d'une expansion de lymphocytes porteurs d'un déterminant Vβ16 et d'une co-expansion de lymphocytes porteurs de Vβs choisis parmi au moins l'un quelconque des Vβs 2, 3, 7, 8, 12, 14, 17 et 22, et préférentiellement des Vβs 3 et 12.

Selon une autre variante avantageuse, on met en évidence une perte de lymphocytes porteurs d'un déterminant Vβ16.

Selon une variante avantageuse, on met en évidence une perte de lymphocytes porteurs d'un déterminant Vβ16 et une co-diminution de lymphocytes porteurs de Vβs choisis parmi au moins l'un quelconque des Vβs 2, 3, 7, 8, 12, 14, 17 et 22, de préférence des Vβs 7, 14, et 17, et avantageusement des Vβs 7 et 17.

La détection de l'expansion et éventuellement de la co-expansion ou de la perte et éventuellement co-diminution est effectuée par mise en évidence des molécules Vβs membranaires associées aux cellules mononucléées sanguines, qui de préférence sont les lymphocytes T. On calcule ensuite un pourcentage de variation des molécules Vβs détectées et provenant de tout ou partie du surnageant de culture de patients atteints de SEP par rapport au nombre de molécules Vβs détectées dans tout ou partie d'un surnageant de culture provenant d'un donneur sain. Il est également possible de prévoir un mélange de surnageants de culture provenant de donneurs sains.

L'ensemble des molécules Vβs est appelé répertoire Vβ des lymphocytes T. Les différentes molécules Vβs sont détectées de manière spécifique par utilisation d'au moins une des techniques décrites ci-dessous :
(a) soit par utilisation d'au moins un ligand, c'est à dire toute molécule capable de reconnaître spécifiquement le Vβ à détecter, par exemple un anticorps monoclonal ou polyclonal anti-Vβ ou un fragment d'anticorps monoclonal ou polyclonal ou une molécule inhibitrice de la fonction du Vβ considéré. Le pourcentage des molécules Vβs du répertoire est alors rapporté au pourcentage des cellules présentant la molécule CD3 à leur surface, cette dernière étant aussi détectée de manière spécifique par l'utilisation d'au moins un ligand. Par ligand, on entend notamment un anticorps monoclonal ou polyclonal ou un fragment desdits anticorps, de préférence un anticorps monoclonal. Les anticorps monoclonaux dirigés contre un Vβ d'intérêt sont produits par des techniques conventionnelles utilisées pour produire des anticorps contre des antigènes de surface. Des souris ou des lapins sont immunisés (i) soit avec une protéine naturelle ou recombinante, (ii) soit avec un peptide immunogène, (iii) soit avec des cellules murines qui expriment la protéine ou le peptide d'intérêt et les molécules du CMHII. La lignée murine Balb/c est la plus fréquemment utilisée. L'immunogène peut être également un peptide choisi parmi les peptides définis à partir des séquences primaires des Vβs d'intérêt. Les protéines ou peptides sont couplés à l'hémocyanine de Lymphet Keyhole (peptide-KLH), comme support pour leur utilisation en immunisation, ou couplé à de l'albumine sérique humaine (peptide-HSA). Les animaux sont soumis à une injection de peptide -KLH ou de peptide -HSA en utilisant de l'adjuvant complet de Freund (IFA). Les sérums et les surnageants de culture d'hybridome issus des animaux immunisés avec chaque peptide sont analysés pour la présence d'anticorps par un test ELISA utilisant les molécules initiales. Les cellules spléniques de ces souris sont récupérées et fusionnées avec des cellules de myélome. Le polyéthylèneglycol (PEG) est l'agent de fusion le plus fréquemment utilisé. Les hybridomes produisant les anticorps les plus spécifiques et les plus sensibles sont sélectionnés. Les anticorps monoclonaux peuvent être produits *in vitro* par culture cellulaire des hybridomes produits ou par récupération de liquide d'ascite murin après injection intrapéritonéale des hybridomes chez la souris. Quel que soit le mode de production en surnageant ou en ascite, il importe ensuite de purifier l'anticorps monoclonal. Les méthodes de purification utilisées sont essentiellement la filtration sur gel échangeur d'ions ou par chromatographie d'exclusion, voire l'immunoprécipitation. Pour chaque anticorps il faut choisir la méthode qui permettra d'obtenir le meilleur rendement. Un nombre suffisant d'anticorps est criblé dans des tests fonctionnels pour identifier les anticorps les plus performants pour fixer la molécule d'intérêt et/ou pour bloquer l'activité de la molécule d'intérêt. Les anticorps monoclonaux sélectionnés sont humanisés par des méthodes standard de " CDR grafting " (protocole réalisé par de nombreuses compagnies, sous forme de service). Ces anticorps humanisés peuvent être testés cliniquement chez le patient. L'efficacité de ces anticorps peut être suivie par des paramètres cliniques. La production *in vitro* d'anticorps, de fragments d'anticorps ou de dérivés d'anticorps, tels que les anticorps chimères humanisés ou non, produits par génie génétique, dans des cellules eucaryotes a été décrite (EP 120 694 ou EP 125 023) et est aussi applicable à la présente invention ;
(b) soit par biologie moléculaire après extraction des acides nucléiques des cellules mononucléées sanguines, entres autres les lymphocytes T, mises en présence de surnageant ou d'une fraction de surnageant de culture SEP et en présence d'une culture de surnageant ou d'une fraction de surnageant de culture témoin, amplification de leurs ARN Vβ transcrits par RT-PCR, étude du profil des produits d'amplification sur gel et/ou séquençage et/ou électrophorèse. Pour l'étape de RT-PCR et/ou pour détecter de façon spécifique les produits d'amplification, on utilise des fragments d'ADN et/ou d'ARN codant pour le déterminant Vβ étudié ou pour un fragment de ce déterminant, et/ou des fragments d'ADN et/ou d'ARN capables de s'hybrider et d'amplifier des fragments d'acides nucléiques codant pour le déterminant Vβ étudié par complémentarité des bases nucléiques. Le profil des fragments amplifiés dont la taille varie en fonction du réarrangement des chaînes spécifiques de chaque clone est déterminé par analyse électrophorétique et permet d'objectiver une polyclonalité. La détection des fragments amplifiés d'ADN et/ou d'ARN codant pour le déterminant Vβ étudié peut aussi être réalisée par hybridation nucléotidique selon les techniques bien connues de l'homme de l'art (Southern Blot, Northern Blot, ELOSA " Enzyme-Linked Oligosorbent Assay " (Katz JB et al., Am. J. Vet. Res. 1993 Dec ; 54 (12) : 2021-6 et François Mallet et al., Journal of Clinical Microbiology, June 1993, p1444-14491)).

Aussi, la présente invention a pour objet un procédé avantageux pour mettre en évidence l'activité superantigénique précitée qui comprend les étapes suivantes :
(i) on prélève un surnageant de culture de cellules mononucléées sanguines ou de cellules de plexus choroïdes ou de cellules leptoméningées, lesdites cellules provenant de patients atteints de sclérose en plaques, ou suspectés d'avoir une prédisposition à développer la sclérose en plaques, ou d'une lignée cellulaire établie, telles que les cellules des lignées cellulaires PLI-2 déposée à l'ECACC le 22 juillet 1992, sous le numéro 92072201 et LM7PC déposée à l'ECACC le 8 janvier 1993, sous le numéro 93010817, conformément aux dispositions du Traité de Budapest, et
(ii) on met en contact ledit surnageant de culture ou une partie du surnageant de culture avec une série de cultures, de préférence au moins trois, de cellules mononucléées sanguines provenant de donneurs sains, et
(iii) on détecte ladite expansion et éventuellement une co-expansion ou ladite perte et éventuellement co-diminution des cellules mononucléées sanguines de l'étape (ii).

Les cellules mononucléées sanguines productrices de molécules superantigéniques et provenant de patients atteints de SEP sont notamment choisies parmi les lymphocytes B et les monocytes.

Les cellules mononucléées sanguines répondant à la stimulation et provenant de donneurs sains sont notamment choisies parmi les lymphocytes T.

Un autre procédé avantageux de l'invention consiste à
(i) prélever des cellules mono nucléées sanguines, lesdites cellules provenant de patients atteints de sclérose en plaques ou de patients suspectés d'avoir un risque de développer la sclérose en plaques et d'individus sains,
(ii) mettre en contact lesdites cellules mononucléées sanguines provenant des patients ou d'individus sains avec des surnageants de culture ou une fraction de surnageant de culture de cellules choisies parmi les cellules mononucléées sanguines, les cellules de plexus choroïdes et les cellules leptoméningées, les cellules issues de lignées cellulaires établies, telles que les cellules des lignées cellulaires PLI-2 déposée à l'ECACC le 22 juillet 1992, sous le numéro 92072201 et LM7PC déposée à l'ECACC le 8 janvier 1993, sous le numéro 93010817, conformément aux dispositions du Traité de Budapest, et
(iii) détecter ladite expansion et éventuellement co-expansion ou ladite perte et éventuellement co-diminution à partir des cellules mononucléées sanguines de l'étape (i).

La mise en évidence de ladite expansion et éventuellement co-expansion ou de ladite perte et éventuellement co-diminution est réalisée
(a) soit par utilisation de ligands, en particulier un anticorps et de préférence un anticorps monoclonal ou un fragment d'anticorps, chaque ligand étant spécifique d'un déterminant choisi parmi les Vβs 16, 2, 3, 7, 8, 12, 14, 17 et 22, de préférence des Vβs 16, 3 et 12, ou d'un ligand en particulier un anticorps et de préférence un anticorps monoclonal, chaque ligand étant spécifique d'un déterminant choisi parmi les Vβs 16, 2, 3, 7, 8, 12, 14, 17 et 22, de préférence les Vβs 16, 7, 14 et 17,
(b) soit selon le protocole décrit ci-dessous en réalisant :
   (i) une extraction des ARN totaux des cellules mononucléées sanguines mises en présence de surnageant ou d'une fraction de surnageant de culture SEP et en présence de surnageant ou d'une fraction de surnageant de culture témoin,
   (ii) une transcription inverse desdits ARN,
   (iii) une amplification spécifique de chaque famille de Vβs à l'aide d'un couple d'amorces déterminé,
   (iv) un marquage par tout marqueur approprié des produits d'amplification obtenus,
   (v) une électrophorèse desdits produits d'amplification et analyse des profils d'électrophorèse obtenus, à l'aide d'un détecteur approprié.

La prédisposition à un état pathologique est évaluée en testant expérimentalement la sensibilisation immunologique d'un patient en mettant en évidence une expansion ou une perte majoritaire de lymphocytes porteurs d'un déterminant Vβ16 et/ou 17 et éventuellement une co-expression ou une co-diminution de lymphocytes porteurs de Vβs choisis parmi au moins l'un quelconque des Vβ 2 , 3, 7, 8, 12, 14, 17 et 22, et préférentiellement des Vβs 3 et 12 ou des Vβs 7, 14 et 17, de préférence 7 et 17.

La prédisposition détectée peut être une prédisposition naturelle ou une prédisposition acquise, par exemple après une forte stimulation chez un individu des lymphocytes T porteurs des déterminants Vβ16 ou Vβ17, préférentiellement Vβ16, par exposition de l'organisme de l'individu à un antigène. Cet antigène peut être par exemple au moins une protéine ou un peptide du virus de l'Hépatite B.

Ainsi, la présente invention concerne un procédé de détection de la SEP ou d'une prédisposition à la SEP selon lequel :
on met en évidence une expansion ou une perte majoritaire de lymphocytes porteurs d'un déterminant Vβ16 et/ou Vβ17, préférentiellement Vβ16 ou expansion ou perte majoritaire de lymphocytes porteurs d'un déterminant Vβ16 et une co-expansion ou co-diminution de lymphocytes porteurs de Vβs des lymphocytes T choisis parmi au moins l'un quelconque des Vβs 2, 3, 7, 8, 12, 14, 17 et 22, et préférentiellement les Vβs 3 et 12 ou les Vβs 7, 14 et 17, de préférence 7 et 17, ladite expansion ou perte étant d'au moins 31%,
on calcule un pourcentage de variation du nombre des Vβs détectées chez un patient atteint de SEP par rapport à celui obtenu avec les lymphocytes T d'un donneur sain, dans des conditions telles que déterminées précédemment.

Si le pourcentage de variation, en valeur absolue, est significativement différent de 0, on peut en déduire que le patient présente une prédisposition pour la maladie SEP et/ou développe la maladie. Si ce pourcentage est en valeur absolue égal à 0, cela peut signifier que le donneur ne présente pas de prédisposition pour la maladie SEP et/ou n'a pas développé la maladie, au moment auquel le procédé a été appliqué.

L'invention a également pour objet, un procédé de détection d'un état pathologique ou d'une prédisposition à un état pathologique, dans un échantillon biologique, selon lequel on met en évidence les paramètres suivants :
une activité superantigénique, telle que définie précédemment, et au moins l'un d'une stimulation de la production de cytokines, telles que l'IL-6, et d'une induction d'apoptose cellulaire.

De préférence, on détecte les trois paramètres en association.

L'état pathologique est en particulier associé à une maladie auto-immune, telle que la sclérose en plaques.

L'activité superantigénique détectée selon les procédés de l'invention peut notamment être induite directement ou indirectement par un agent effecteur choisi parmi les protéines, les microorganismes, les agents pathogènes et par exemple les bactéries et/ou les rétrovirus, de préférence les rétrovirus humains, tels que MSRV-1 et/ou les agents pathogènes tels que MSRV-2.

En particulier, l'activité superantigénique est induite par la protéine d'enveloppe de MSRV-1 référencée en SEQ ID NO :2 ou par un fragment de ladite protéine, ou bien par le produit d'expression du gène *env* de MSRV-1 référencée en SEQ ID NO :1 ou d'un fragment dudit gène.

Pour mieux comprendre l'invention, on définit certains termes :
- Un rétrovirus humain, en particulier rétrovirus endogène, ayant une activité superantigénique et étant associé à une maladie auto-immune, selon lequel le rétrovirus est MSRV-1 et l'activité superantigénique est induite par l'expression du gène *env* de MSRV-1 ou d'un fragment dudit gène, en particulier un fragment dudit gène codant pour au moins un cadre de lecture de la protéine env de MSRV-1 (SEQ ID NO :2) ou bien elle est induite par la protéine env de MSRV-1 ou par un fragment de ladite protéine, en particulier par un fragment correspondant à au moins un cadre de lecture de ladite protéine (SEQ ID NO :2).
- Une molécule d'acide nucléique comprenant au moins un fragment ou des fragments de l'ARN ou de l'ADN du gène *env* de MSRV-1, identifié par SEQ ID NO :1, ledit fragment ayant une longueur d'au moins 18 nucléotides et de préférence d'au moins 24 nucléotides ; notamment elle comprend au moins un fragment codant pour au moins un cadre de lecture et contenant éventuellement un codon stop ; cette molécule pouvant coder pour une activité superantigénique.
- une molécule polypeptidique, en particulier protéine ou fragment de protéine comprenant au moins un fragment ou des fragments de ladite protéine env de MSRV-1 identifiée par SEQ ID NO :2, ledit fragment ayant une longueur d'au moins 6 acides aminés et de préférence d'au moins 8 acides aminés ; avantageusement la molécule polypeptique comprend au moins un cadre de lecture, et possède éventuellement une activité superantigénique.

Selon une variante de l'invention, additionellement
(i) on produit ou synthétise une protéine recombinante, telle qu'identifiée par SEQ ID NO :2, ou un fragment de ladite protéine,
(ii) on met en contact ladite protéine avec une série de cultures, de préférence au moins trois, de cellules mononucléées sanguines provenant de donneurs sains, et
(iii) on détecte ladite expansion et éventuellement une co-expansion ou ladite perte et éventuellement co-diminution des cellules mononucléées sanguines de l'étape (ii).

Ou bien
(i) on prélève des cellules mononucléées sanguines, lesdites cellules provenant de patients atteints de la SEP ou de patients suspectés d'avoir un risque de développer la SEP, et d'individus sains,
(ii) on met en contact lesdites cellules mononucléées sanguines provenant des patients ou d'individus sains avec un polypeptide ou une protéine recombinante, telle qu'identifiée en SEQ ID NO:2, ou un fragment dudit polypeptide ou de ladite protéine, et
(iii) on détecte ladite expansion et éventuellement co-expansion ou ladite perte et éventuellement co-diminution à partir des cellules mononucléées sanguines de l'étape (i).

De préférence, on utilise un polypeptide de l'invention tel que défini précédemment. Avantageusement, il est codé par un gène env, tel que défini précédemment.

Les procédés tels que définis précédemment peuvent être utilisés pour le suivi thérapeutique de patients et/ou l'évaluation de l'efficacité de molécules à usage thérapeutique vis à vis de paramètres identifiés.

Pour évaluer l'efficacité de molécules à usage thérapeutique, c'est à dire une ou des molécule(s) susceptible(s) d'inhiber l'expansion ou la perte des lymphocytes T d'un Vβ déterminé on prélève un surnageant de culture de cellules mononucléées sanguines ou de cellules de plexus choroïdes ou de cellules leptoméningées, lesdites cellules provenant de patients atteints d'une maladie auto-immune, en particulier de SEP, ou de cellules issues de lignées cellulaires établies, telles que les cellules des lignées cellulaires PLI-2 et LM7PC.
(i) on prélève des cellules mononucléées sanguines, lesdites cellules provenant d'individus sains,
(ii) on met en contact lesdites cellules mononucléées sanguines avec ledit surnageant de culture ou une fraction de surnageant de culture, et
(iii) on détecte l'inhibition de ladite expansion et éventuellement co-expansion ou l'inhibition de ladite perte et éventuellement co-diminution à partir des cellules mononucléées sanguines de l'étape (i), en présence dudit agent ou de ladite composition à des doses déterminées, des lymphocytes porteurs d'au moins un déterminant choisi parmi les Vβs 16, 7 et 17, par utilisation d'un ligand comme décrit précédemment ou d'une amplification associée à une électrophorèse comme décrit ci dessus.

On calcule ainsi un pourcentage de variation du nombre des Vβs détectés en présence de la molécule ou des molécules à usage thérapeutique et en l'absence d'une telle ou de telles molécule(s).

La molécule ou les molécules à usage thérapeutique sont également testée(s) *in vivo* selon un procédé qui consiste
à utiliser un surnageant de culture de cellules mononucléées, de préférence les lymphocytes B et les monocytes, ou de cellules de plexus choroïdes ou de cellules leptoméningées, lesdites cellules étant isolées à partir d'un échantillon biologique d'un patient atteint de SEP, ou à utiliser le surnageant d'une lignée cellulaire établie, telle que la lignée cellulaire PLI-2 et la lignée cellulaire LM7PC ou à utiliser tout ou partie d'au moins une molécule obtenue à partir d'au moins un des deux surnageants précités,
à mettre en contact tout ou partie d'au moins l'un des surnageants précités ou au moins une molécule contenue dans l'un au moins d'entre eux, avec une culture de cellules mono nucléées sanguines, de préférence des lymphocytes T, prélevées chez un individu et/ou un animal avant et après administration de la ou des molécules à tester, et parallèlement après administration d'un agent et/ou composition placebo chez un patient SEP et chez un animal,
à mettre en évidence, comme décrit ci-dessus, l'expansion ou la perte des lymphocytes ayant le déterminant Vβ, avant et après l'administration de la molécule ou des molécules à usage thérapeutique ou de l'agent et/ou composition placebo, chez l'individu et/ou l'animal,
à détecter ladite expansion et éventuellement une co-expansion et/ou la perte et éventuellement une co-diminution des lymphocytes T exprimant à leur surface une molécule Vβ donnée par utilisation d'au moins un ligand ou par biologie moléculaire comme décrit précédemment, et
à calculer un pourcentage (X) d'expansion ou de perte de déterminants Vβ détectées dans les cellules mononucléées mises en culture et issues de l'individu ou de l'animal ayant reçu une ou plusieurs administrations de la molécule ou des molécules à tester par rapport au nombre de déterminants Vβ détectés dans les cellules mises en culture et issues de l'individu ou de l'animal n'ayant reçu aucune administration et/ou un pourcentage (Y) d'expansion ou de perte de déterminants Vβ détectées dans les cellules mononucléées mises en culture et issues de l'individu ou de l'animal ayant reçu une ou plusieurs administrations de la molécule ou des molécules à tester par rapport au nombre de déterminants Vβ détectés dans les cellules mises en culture et issues du patient ou de l'animal ayant reçu une ou des administration de molécule(s) et/ou d'une composition ou d'un agent placebo en respectant les mêmes conditions d'administration que celle utilisées pour la ou les molécules à évaluer.

Si |X| et/ou |Y| et/ou |X|+|Y| sont significativement différents de 0, cela peut signifier que la molécule ou les molécules inhibent totalement ou partiellement l'expansion ou la perte de cellules mononucléées avec le déterminant Vβ considéré; si |X| ou |Y| ou |X| + |Y| sont égaux ou proches de 0, cela peut signifier que l'agent n'inhibe pas totalement ou que partiellement l'expansion ou la perte de cellules mononucléées avec le déterminant Vβ considéré.

On entend par agent et/ou composition placebo, tout agent et/ou composition qui n'entraîne pas une diminution des cellules mononucléées sanguines ayant le déterminant Vβ déterminé.

L'invention concerne aussi un procédé d'évaluation de l'efficacité d'un agent ou d'une composition pour inhiber une activité superantigénique dans un échantillon biologique, comprenant les étapes suivantes :
(i) on produit ou synthétise un polypeptide, en particulier une protéine recombinante telle qu'identifiée par SEQ ID NO :2, ou un fragment dudit polypeptide ou de ladite protéine,
(ii) on met en contact ledit polypeptide ou protéine recombinante avec une série de cultures, de préférence au moins trois, de cellules mononucléées sanguines provenant de donneurs sains en présence dudit agent ou de ladite composition à des doses prédéterminées, et
(iii) on détecte l'inhibition de ladite expansion et éventuellement co-expansion ou l'inhibition de ladite perte et éventuellement co-diminution des lymphocytes porteurs d'au moins un déterminant choisi parmi les Vβs 16, 2, 3, 7, 8, 12, 14, 17 et 22, en particulier les Vβs 16 et/ou 17, les Vβs 16, 3 et 12 ou les Vβs 16, 7, 14, et 17, en particulier les Vβs 16, 7 et 17, par utilisation d'un ligand ou d'une amplification associée à une électrophorèse comme décrit précédemment.

L'efficacité thérapeutique de plusieurs agents et/ou compositions peut être évaluée en combinaison dans un même essai.

Un procédé *in vivo* avantageux pour tester l'efficacité d'un agent thérapeutique dans la sclérose en plaques comprend les étapes suivantes :
on détermine les pourcentages X et Y comme décrit et défini ci-dessus. X et Y sont déterminés après administration d'un agent et/ou composition à évaluer dans un patient atteint de la SEP,
on détermine de même X' et Y'; ils sont déterminés comme X et Y respectivement, mais après administration du même composé dans un individu sain,
on calcule les pourcentages x et y, avec x = X/X' et y = Y/Y'. Ces pourcentages reflètent la diminution du nombre de cellules mononucléées sanguines présentant le déterminant Vβ due à l'administration du composé thérapeutique à tester chez un patient atteint de sclérose en plaques par rapport à un individu sain.

Si |x| et/ ou |y| et/ou |x| + |y| sont significativement différents de 0, cela peut signifier que l'agent et/ou la composition testés à un effet thérapeutique vis-à-vis de la pathologie SEP; Si |x| et/ou |y| et/ou |x| + |y| sont égaux ou proches de 0, cela peut signifier que l'agent et/ou la composition testés n'a pas ou très peu d'effet thérapeutique vis-à-vis de la pathologie SEP.

Par composition à usage thérapeutique et/ou prophylactique, on entend une composition qui comprend, entre autres, un agent thérapeutique capable d'inhiber, directement ou indirectement, une activité superantigénique dans un échantillon biologique, telle que définie précédemment, éventuellement en association avec un véhicule et/ou un excipient et/ou un adjuvant et/ou un diluant pharmaceutiquement acceptables, c'est à dire qui permettent l'administration à l'animal et à l'être humain. Toutes ces données font partie des connaissances générales de l'homme du métier. (voir par exemple Remington's Pharmaceutical Sciences 16^{th} ed. 1980, Mack Publishing Co). Le véhicule pharmaceutiquement acceptable est préférentiellement isotonique, hypotonique ou présente une faible hypertonicité et présente une force ionique relativement basse, tel que par exemple une solution de sucrose. Par ailleurs, ladite composition peut contenir des solvants, des véhicules aqueux ou partiellement aqueux tels que de l'eau stérile, libre d'agents pyrogène et des milieux de dispersion par exemple. Le pH de ces compositions pharmaceutiques est convenablement ajusté et tamponné selon les techniques conventionnelles.

On entend par efficacité thérapeutique le bénéfice clinique et biologique acquis après administration d'un agent thérapeutique en vue d'une amélioration, voire d'une guérison de la maladie. Ce bénéfice se traduit entre autre par une diminution des signes cliniques, biologiques et des effets pathologiques de la maladie après une analyse clinique par le médecin et/ou des analyses biologiques, telles que l'imagerie par résonance magnétique, analyse des bandes oligoclonales dans liquide céphalorachidien, analyse de potentiels... Cette diminution de signes cliniques et effets pathologiques doit entraîner un bénéfice pour le patient (Schwartz et Lazar, 1995, Elements de statistique médicale et biologique, eds Flammarion ; Lazar et Schwartz, 1995, Elements de statistique médicale et biologique, eds Flammarion). La maladie étudiée de préférence est la sclérose en plaques. Ces agents thérapeutiques sont capables (i) d'influencer de manière qualitative et/ou quantitative l'activité superantigénique telle que décrite dans la présente invention et/ou (ii) de moduler et/ou d'inhiber l'expression des déterminants Vβs identifiés dans la présente invention. Différents agents thérapeutiques sont produits en suivant des approches classiques largement décrites dans la littérature. Les différents groupes d'agents thérapeutiques envisageables dans la présente invention sont décrits ci-dessous.

Comme défini précédemment, l'agent thérapeutique est capable d'inhiber ou de diminuer, directement ou indirectement, une activité superantigénique, telle que définie dans la présente invention, pour un ou des Vβs déterminés et/ou pour un ou des rétrovirus MSRV. L'agent thérapeutique consiste en un matériel chimique ou biologique ou en une composition qui présente une efficacité thérapeutique qui peut être mise en évidence selon les modalités décrites précédemment.

Pour la simplicité de l'exposé, on parlera généralement de molécules d'intérêt de l'invention aussi bien pour désigner le ou les différents Vβs associé(s) à l'activité superantigénique, que pour désigner les protéines de MSRV-1, en particulier la protéine env de MSRV-1.

Par agent thérapeutique on entend :
- au moins une molécule naturelle et/ou une molécule recombinante ou leurs fragments dont la séquence correspond à tout ou partie de la séquence des molécules d'intérêt, c'est à dire :
   - au moins une protéine naturelle et/ou une protéine recombinante et/ou un polypeptide de synthèse choisi parmi les molécules d'intérêt de l'invention,
   - au moins un fragment naturel et/ou synthétique de ces molécules d'intérêt, par exemple un fragment immunogène capable d'induire une réponse immune contre un polypeptide issu d'au moins une des molécules d'intérêt de l'invention,
   - au moins un peptide mimotope défini à partir des séquences des molécules d'intérêt de l'invention, ou une combinaison de mimotopes, capable d'induire une réponse immune contre un polypeptide issu d'au moins une des molécules d'intérêt de l'invention,
   - au moins une protéine ou un peptide pouvant réguler *in vivo* la transcription et/ou la traduction des molécules d'intérêt de l'invention et les séquences peptidiques ou les fragments desdites séquences.

La réponse immune dirigée contre un antigène spécifique peut être divisée en deux catégories distinctes, l'une mettant en jeu les anticorps (réponse immune de type humorale), l'autre les cellules effectrices cytotoxiques telles que par exemple les macrophages, les lymphocytes cytotoxiques (CTL) ou les cellules tueuses (NK) ainsi que les lymphocytes T " helper ", notamment les lymphocytes T CD4+ (réponse immune de type cellulaire). Plus particulièrement, les deux types de réponse se distinguent en ce que les anticorps reconnaissent les antigènes sous leur sous leur forme tridimensionnelle alors que les lymphocytes T, par exemple, reconnaissent des portions peptidiques desdits antigènes, associés à des glycoprotéines codées par les gènes du complexe majeur d'histocompatibilité (CMH), notamment les gènes du complexe majeur d'histocompatibilité de type 1 qui sont exprimés de façon ubiquitaire à la surface des cellules ou les gènes du complexe majeur d'histocompatibilité de type II qui sont exprimés de façon spécifique à la surface des cellules impliquées dans la présentation des antigènes (APC). 1) Selon un premier aspect, la réponse immune de type cellulaire est caractérisée en ce que les cellules T de type CD4+ (cellules T " helper "), suite à un phénomène d'activation bien connu (pour une revue voir Alberola-lia 1997, Annu Rev Immunol 15, 125-154) produisent des cytokines qui à leur tour induisent la prolifération de cellules APC capables de produire lesdites cytokines, la différenciation cellulaire des lymphocytes B capables de produire des anticorps spécifiques de l'antigène, et la stimulation des lymphocytes T cytotoxiques (CTL). 2) Selon un second aspect de la réponse immune cellulaire, les cellules effectrices cytotoxiques telles que par exemple les lymphocytes de type CD8 + (CTL) sont activés a) après interaction avec des peptides antigéniques fixés sur et présentés par les glycoprotéines portées par les cellules ubiquitaires et codées par les gènes appartenant au système CMHI, et b) éventuellement par les cytokines produites par les CD4+.

A partir de la séquence en acides aminés des molécules d'intérêt de l'invention, des séquences peptidiques de ces molécules ou des fragments de séquences peptidiques de ces molécules, correspondant à tout ou partie de la séquence primaire de ces molécules peuvent être synthétisés par des méthodes classiques de synthèse peptidique ou obtenus par recombinaison génétique.

Des protéines recombinantes correspondantes aux molécules d'intérêt de l'invention, produites dans un système cellulaire procaryote ou eucaryote, peuvent être produite par l'homme du métier à partir de la connaissance des séquences des gènes correspondants décrits dans la littérature et en tenant compte de la dégénérescence du code génétique. Toutes les séquences protéiques identifiées dans la présente invention sont ainsi susceptibles d'être obtenues par recombinaison génétique. Les gènes sont clonés dans des vecteurs adaptés. Des vecteurs différents sont utilisés pour transformer des cellules procaryotes (par exemple *E. coli*) et des cellules eucaryotes (par exemple cellules COS, CHO et cellules Simliki). Les protéines recombinantes correspondant aux molécules d'intérêt de l'invention ou à des fragments de ces protéines peuvent être ainsi produits dans des systèmes cellulaires procaryotes et/ou encaryotes. Dans les cellules *E. coli,* les protéines recombinantes sont produites avec une queue poly-histidine. La fraction protéique insoluble est solubilisée dans de l'urée 8M. L'enrichissement du produit est effectué sur résine chélatée au nickel (Qiagen). La colonne est lavée avec des concentrations décroissantes d'urée. L'élution est réalisée avec de l'imidazole en l'absence d'urée. La séquence complète des molécules d'intérêt de l'invention peut être également clonée dans un plasmide adapté puis transférée dans le virus de la vaccine pour obtenir un virus recombinant ;
- au moins un ligand spécifique d'au moins une desdites molécules d'intérêt de l'invention ou de leurs fragments qui est capable de se fixer aux molécules d'intérêt ou aux récepteurs desdites molécules d'intérêt ou encore d'interférer pour la liaison de la molécule d'intérêt à la cellule présentatrice d'antigène et d'inhiber l'activité superantigénique, c'est à dire :

- au moins un anticorps ou fragment d'anticorps polyclonal ou monoclonal spécifique d'au moins une desdites molécules d'intérêt de l'invention ou de leurs fragments. Cet anticorps peut être ou non neutralisant, c'est-à-dire capable ou non d'inhiber l'activité de la protéine d'intérêt. Le ligand peut être choisi parmi toute molécule ou fragment de molécule capable de se fixer aux molécules d'intérêt, par exemple des récepteurs, des co-facteurs de ces molécules, des anticorps polyclonaux ou monoclonaux capables de se fixer aux molécules d'intérêt ou à tout fragment.

Ces anticorps sont très utiles notamment pour permettre la mise en oeuvre de compositions thérapeutiques car ils conduisent par exemple, à des réactions immunes, dirigées spécifiquement à l'encontre d'épitopes immunodominants ou contre des antigènes présentant une grande variabilité. On administre chez le patient soit des anticorps solubles neutralisants pour inhiber leur fonction, soit des anticorps solubles spécifiques pour éliminer le peptide par formation de complexes immuns. L'invention décrit l'utilisation d'anticorps capables de reconnaître spécifiquement au moins une molécule d'intérêt décrite dans la présente invention pour le traitement et /ou pour le suivi thérapeutique de maladie, de préférence la sclérose en plaques. Ces anticorps sont polyclonaux et de préférence monoclonaux. De préférence ces anticorps inhibe la fonction de la protéine en se fixant. La capacité de l'anticorps à se fixer spécifiquement à la protéine est analysée par des techniques conventionnelle décrites, comme par exemple par des tests ELISA ou de Western Blot en utilisant la molécule ou le peptide immunogène naturel ou synthétique. Le titre de l'anticorps est alors déterminé. La capacité de l'anticorps à neutraliser la fonction de la protéine peut être analysée par différents moyen, par exemple en déterminant la diminution de l'activité de la molécule ou du peptide immunogène en présence de l'anticorps.

Par exemple, les anticorps monoclonaux dirigés contre une molécule d'intérêt de l'invention ou une partie de cette molécule sont produits par des techniques conventionnelles utilisées pour produire des anticorps contre des antigènes de surface Des souris ou des lapins sont immunisées (i) soit avec la protéine naturelle ou recombinante d'intérêt, (ii) soit avec tout peptide immunogène de cette protéine d'intérêt, (iii) soit avec des cellules murines qui expriment la protéine ou le peptide d'intérêt et les molécules du CMHII. La lignée murine Balb/c est la plus fréquemment utilisée. L'immunogène peut être également un peptide choisi parmi les peptides définis à partir des séquences primaires des molécules d'intérêt. Les protéines ou peptides sont couplés à l'hémocyanine de Lymphet Keyhole, en abrégé peptide-KLH, comme support pour son utilisation en immunisation, ou couplé à de l'albumine de sérique humaine, en abrégé peptide-HSA. Les animaux sont soumis à une injection de peptide -KLH ou de peptide -HSA en utilisant de l'adjuvant complet de Freund (IFA). Les sérums et les surnageants de culture d'hybridome issus des animaux immunisés avec chaque peptide sont analysés pour la présence d'anticorps anti-molécules par un test ELISA utilisant les molécules initiales. Les cellules spléniques de ces souris sont récupérées et fusionnées avec des cellules de myélome. Le polyéthylèneglycol (PEG) est l'agent de fusion le plus fréquemment utilisé. Les hybridomes produisant les anticorps les plus spécifiques et les plus sensibles sont sélectionnés. Les anticorps monoclonaux peuvent être produits *in vitro* par culture cellulaire des hybridomes produits ou par récupération de liquide d'ascite murin après injection intrapéritonéale des hybridomes chez la souris. Quel que soit le mode de production en surnageant ou en ascite, il importe ensuite de purifier l'anticorps monoclonal. Les méthodes de purification utilisées sont essentiellement la filtration sur gel échangeur d'ions ou par chromatographie d'exclusion, voire l'immunoprécipitation. Pour chaque anticorps il faut choisir la méthode qui permettra d'obtenir le meilleur rendement. Un nombre suffisant d'anticorps anti-protéines sont criblés dans des tests fonctionnels pour identifier les anticorps les plus performants pour fixer la molécule d'intérêt et/ou pour bloquer l'activité de la molécule d'intérêt. Les anticorps monoclonaux sélectionnés sont humanisés par des méthodes standard de " CDR grafting " (protocole réalisé par de nombreuses compagnies, sous forme de service). Ces anticorps humanisés peuvent être testés cliniquement chez le patient. L'efficacité de ces anticorps peut être suivie par des paramètres cliniques.

La production *in vitro* d'anticorps, de fragments d'anticorps ou de dérivés d'anticorps, tels que les anticorps chimères humanisés ou non, produits par génie génétique, dans des cellules eucaryotes a été décrite (EP 120 694 ou EP 125 023) et est aussi applicable à la présente invention,
- au moins une molécule inhibitrice de la fonction d'au moins une molécule choisie parmi les molécules d'intérêt de l'invention ou leurs fragments,
- au moins une molécule régulatrice de l'expression d'au moins une molécule choisie parmi les molécules d'intérêt de l'invention ou leurs fragments, par exemple pour bloquer la transcription ou la traduction de ces molécules,
- au moins une molécule régulatrice du métabolisme d'au moins une protéine choisie parmi les molécules d'intérêt de l'invention ou leurs fragments,
- au moins une molécule régulatrice de l'expression et/ou du métabolisme d'un ligand d'au moins une protéine choisie parmi les molécules d'intérêt de l'invention ou leurs fragments, par exemple un récepteur ou un co-facteur ;
- au moins une séquence d'acides nucléiques comprenant au moins un gène d'intérêt thérapeutique dont la séquence nucléique est déduite des séquences d'ADN et d'ARN codant pour tout ou partie des molécules d'intérêt de l'invention, en association avec des éléments assurant l'expression dudit gène d'intérêt thérapeutique *in vivo* dans des cellules cibles destinées à être génétiquement modifiées par la séquence nucléique du gène d'intérêt thérapeutique. Les gènes peuvent être non mutés ou mutés. Ils peuvent également consister en des acides nucléiques modifiés de sorte qu'il ne leur est pas possible de s'intégrer dans le génome de la cellule cible, ou d'acides nucléiques stabilisés à l'aide d'agents, tels
que la spermine. Un tel gène d'intérêt thérapeutique code notamment pour :
- au moins une protéine choisie parmi les molécules d'intérêt identifiées dans la présente invention ou leurs fragments, et/ou
- au moins un ligand ou toute partie d'un ligand capable de se fixer à au moins une protéine ou un fragment de protéine choisi parmi les molécules d'intérêt identifiées dans la présente invention ou leurs fragments, pouvant inhiber ou non la fonction de la molécule d'intérêt, et/ou
- au moins tout ou partie d'un anticorps polyclonal ou monoclonal capable de se fixer à au moins une protéine ou un fragment de protéine choisi parmi les molécules d'intérêt identifiées dans la présente invention ou leurs fragments, pouvant inhiber ou non la fonction de la molécule d'intérêt. Il peut notamment s'agir d'anticorps transmembranaire natif, ou de fragment ou dérivé d'un tel anticorps, pour autant que ledit anticorps, fragment ou dérivé d'anticorps soit exprimé à la surface de la cellule cible du mammifère génétiquement modifiée et soit capable de se fixer à un polypeptide présent à la surface d'une cellule effectrice cytotoxique ou d'un lymphocyte T " helper " impliqué dans le procédé d'activation d'une telle cellule, et/ou
- au moins une molécule inhibitrice d'au moins une protéine ou de ses fragments, ladite protéine étant choisie parmi les molécules d'intérêt identifiées dans la présente invention, pouvant inhiber la fonction et/ou le métabolisme et/ou la fixation des molécules d'intérêt ou de leurs fragments.

Par ailleurs, ledit acide nucléique peut comprendre au moins deux séquences, identiques ou différentes, présentant une activité de promoteur transcriptionnel et/ou au moins deux gènes, identiques ou différents, situés l'un par rapport à l'autre de manière contiguë, éloignée, dans le même sens ou dans le sens inverse, pour autant que la fonction de promoteur transcriptionnel ou la transcription desdits gènes ne soit pas affectée.

De même dans ce type de construction d'acide nucléique, il est possible d'introduire des séquences nucléiques " neutres " ou introns qui ne nuisent pas à la transcription et sont épissées avant l'étape de traduction. De telles séquences et leurs utilisations sont décrites dans la littérature (référence : demande de brevet PCT WO 94/29471).

Ledit acide nucléique peut également comprendre des séquences requises pour le transport intracellulaire, pour la réplication et/ou pour l'intégration, pour la transcription et/ou la traduction. De telles séquences sont bien connues de l'homme de l'art.

Par ailleurs, les acides nucléiques utilisables selon la présente invention peuvent également être des acides nucléiques modifiés de sorte qu'il ne leur est pas possible de s'intégrer dans le génome de la cellule cible, ou des acides nucléiques stabilisés à l'aide d'agents, tels que par exemple la spermine, qui en tant que tels n'ont pas d'effet sur l'efficacité de la transfection.

La séquence d'acide nucléique est de préférence une séquence d'ADN ou ARN nue, c'est à dire libre de tout composé facilitant son introduction dans les cellules (transfert de séquence d'acide nucléique). Toutefois, selon un second mode de réalisation de l'invention, afin de favoriser son introduction dans les cellules cibles et afin d'obtenir les cellules génétiquement modifiées de l'invention, cette séquence d'acide nucléique peut être sous la forme d'un " vecteur ", et plus particulièrement sous la forme d'un vecteur viral, tel que par exemple un vecteur adénoviral, rétroviral, un vecteur dérivé d'un poxvirus, notamment dérivé du virus de la vaccine ou du Modified Virus Ankara (MVA) ou d'un vecteur non viral tel que, par exemple, un vecteur consistant en au moins une dite séquence d'acide nucléique complexée ou conjuguée à au moins une molécule ou substance porteuse sélectionnée parmi le groupe consistant en un amphiphile cationique, notamment un lipide cationique, un polymère cationique ou neutre, un composé polaire protique notamment choisi parmi le propylène glycol, le polyéthylène glycol, le glycérol, l'éthanol, la 1-méthyl L-2-pyrrolidone ou leurs dérivés, et un composé polaire aprotique notamment choisi parmi le diméthylsulfoxide (DMSO), le diéthylsulfoxide, le di-n-propylsulfoxide, le diméthylsulfone, le sulfolane, la diméthylformamide, le diméthylacetamide, la tetraméthylurée, l'acétonitrile ou leurs dérivés. La littérature procure un nombre important d'exemples de tels vecteurs viraux et non viraux.

De tels vecteurs peuvent en outre et de préférence comprendre des éléments de ciblage pouvant permettre de diriger le transfert de séquence d'acide nucléique vers certains types cellulaires ou certains tissus particuliers tels que les cellules T helpers, les cellules T cytotoxiques et les cellules présentatrices de l'antigène. Ils peuvent également permettre de diriger le transfert d'une substance active vers certains compartiments intracellulaires préférés tel que le noyau, les mitochondries ou les peroxysomes, par exemple. Il peut en outre s'agir d'éléments facilitant la pénétration à l'intérieur de la cellule ou la lyse de compartiments intracellulaires. De tels éléments de ciblage sont largement décrits dans la littérature. II peut par exemple s'agir de tout ou partie de lectines, de peptides, notamment le peptide JTS-1 (voir demande de brevet PCT WO 94/40958), d'oligonucléotides, de lipides, d'hormones, de vitamines, d'antigènes, d'anticorps, de ligands spécifiques de récepteurs membranaires, de ligands susceptibles de réagir avec un anti-ligand, de peptides fusogènes, de peptides de localisation nucléaire, ou d'une composition de tels composés,
ou encore
- au moins une séquence d'acides nucléiques capable de s'hybrider à une séquence d'acides nucléiques codant pour les molécules d'intérêt de l'invention ou leurs fragments. Les fragments correspondent en particulier à des molécules anti-sens ou ribozyme et peuvent être synthétisés à l'aide de synthétiseurs automatiques, tels que ceux commercialisés par la société Applied Biosystem. Les oligonucléotides anti-sens sont capables d'interférer spécifiquement avec la synthèse d'une protéine cible d'intérêt, par inhibition de la formation et/ou du fonctionnement du polysome selon le positionnement de l'ARNm dans la cible. Donc le choix fréquent de la séquence entourant le codon d'initiation de la traduction comme cible pour une inhibition par un oligonucléotide anti-sens vise à prévenir la formation du complexe d'initiation. D'autres mécanismes dans l'inhibition par des oligonucléotides anti-sens impliquent une activation de la ribonucléase H qui digère les hybrides oligonucléotide anti-sens/ARNm ou une interférence au niveau de sites d'épissage par des oligonucléotides anti-sens dont la cible est un site d'épissage de l'ARNm. Les oligonucléotides anti-sens sont également complémentaires de séquences ADN et peuvent donc interférer au niveau de la transcription par la formation d'une triple hélice, l'oligonucléotide anti-sens s'appariant par des liaisons hydrogène dites de Hoogsteen au niveau du grand sillon de la double hélice d'ADN. Dans ce cas particulier, on parle plus précisément d'oligonucléotides antigènes. Il est bien entendu que les oligonucléotides anti-sens peuvent être strictement complémentaires de la cible ADN ou ARN à laquelle ils doivent s'hybrider, mais aussi non strictement complémentaires à la condition qu'ils s'hybrident à la cible. De même, il peut s'agir d'oligonucléotides anti-sens non modifiés ou modifiés au niveau des liaisons inter-nucléotidiques. Toutes ces notions font partie des connaissances générales de l'homme de l'art. Bien entendu, de telles molécules anti-sens peuvent constituer en tant que telles des vecteurs. On peut également utiliser des vecteurs qui comprennent une séquence d'acides nucléique qui code pour un anti-sens. Ces molécules d'acides nucléiques sont capables de s'hybrider dans des conditions stringentes à l'ADN ou/et ARN codant pour les protéines de l'invention ou pour leur(s) fragment(s). Des conditions de stringence caractéristiques sont celles qui correspondent à une combinaison de la température et de la concentration saline choisie approximativement entre 12 à 20°C sous le Tm (" melting temperature ") de l'hybride à l'étude. De telles molécules sont synthétisées et peuvent être marquées en utilisant des méthodes de marquage conventionnelles utilisées pour les sondes moléculaires, ou peuvent être utilisées comme amorces dans les réactions d'amplification. Les séquences qui présentent au moins 90% d'homologie par rapport à une séquence de référence font également partie de l'invention, de même que les fragments de ces séquences qui présentent au moins 20 nucléotides et de préférence 30 nucléotides contigus homologues par rapport à une séquence de référence.

Les molécules ou séquences d'acides nucléiques ADN ou ARN consistent en un fragment d'ADN et/ou d'ARN, double brin ou simple brin, linéaire ou circulaire, naturel et isolé ou de synthèse, correspondant à un enchaînement précis de nucléotides, modifiés ou non, et permettant de définir un fragment ou une région d'un acide nucléique choisi dans le groupe consistant en un ADNc; un ADN génomique ; un ADN plasmidique ; un ARN messager. Les séquences d'acides nucléiques sont déduites de la séquence d'acides aminés des molécules d'intérêt de l'invention ou de leurs fragments, en utilisant le code génétique. En raison de la dégénérescence du code génétique l'invention englobe également des séquences équivalentes ou homologues. Ces séquences définies permettent à l'homme de l'art de définir lui-même les molécules adaptées. II peut ainsi définir et utiliser des molécules d'acides nucléiques complémentaires des séquences ADN et/ou ARN codant pour les molécules d'intérêt de l'invention ou de leur(s) fragment(s).

Ces séquences d'acides nucléiques et/ou vecteurs permettent de cibler les cellules dans lesquelles la protéine ou le fragment de protéine est exprimé, soit par utilisation de molécule de ciblage introduite sur le vecteur, soit par l'utilisation d'une propriété particulière de la cellule ;
- au moins une cellule de mammifère ne produisant pas naturellement au moins une molécule d'intérêt de l'invention ou tout fragment de ces molécules, ou des anticorps spécifiques d'au moins une desdites molécules d'intérêt de l'invention ou de ses fragments, ladite cellule de mammifère étant génétiquement modifiée *in vitro* par au moins une séquence d'acide nucléique ou un fragment d'une séquence nucléique ou une association de séquences d'acides nucléiques correspondant à des fragments d'acides nucléiques issus d'un même gène ou de gènes différents, la ou lesdites séquences nucléiques étant déduite(s) des séquences d'ADN et d'ARN codant pour les molécules d'intérêt de l'invention ou tout fragment, ledit gène d'intérêt thérapeutique codant pour tout ou partie de la molécule d'intérêt de l'invention, d'un fragment de la molécule ou d'un anticorps spécifique de la molécule qui sera exprimé à la surface de ladite cellule de mammifère (Toes et al., 1997, PNAS 94 : 14660-14665). Ainsi, ladite cellule contient au moins un gène qui code *in vivo* pour :
   - au moins une protéine choisie parmi les molécules d'intérêt de l'invention et/ou leurs fragments, et/ou
   - au moins un peptide défini à partir de la séquence primaire d'au moins une protéine choisie parmi les molécules d'intérêt de l'invention et/ou leurs fragments, et/ou
   - au moins toute molécule inhibitrice de l'activité et/ou de la fixation et/ou de l'expression de ces molécules, et/ou
   - au moins un peptide issu de la séquence primaire d'une protéine choisie parmi les molécules d'intérêt de l'invention et/ou leurs fragments, et capable de se fixer sur au moins une glycoprotéine du CMHI et/ou CMHII, et/ou
   - au moins un ligand et/ou tout anticorps et/ou toute partie d'anticorps capable de se fixer à au moins une protéine choisie parmi les molécules d'intérêt de l'invention et/ou les fragments.

Plus particulièrement, ladite cellule cible provient soit du mammifère à traiter, soit d'un autre mammifère que celui à traiter. Dans ce dernier cas, il convient de noter que ladite cellule cible aura subi un traitement la rendant compatible avec le mammifère à traiter. Selon un aspect préféré, par "mammifère" on entend désigner un mammifère humain. Ces cellules sont établies en lignées cellulaires et sont préférentiellement CMHII + ou CMHII- inductibles comme les lymphocytes, les monocytes, les astrocytes, les oligodendrocytes.

L'invention concerne également les cellules modifiées et un procédé de préparation d'une cellule telle que décrite ci dessus caractérisé en ce que l'on introduit dans une cellule de mammifère par tout moyen approprié, au moins une séquence d'acide nucléique contenant au moins un gène d'intérêt thérapeutique et des éléments assurant l'expression dudit gène dans ladite cellule, ledit gène d'intérêt thérapeutique contenant une séquence d'acide nucléique codant pour une molécule ou un fragment de molécule *in vivo,* comme décrit juste ci-dessus. Plus particulièrement, elle concerne des cellules procaryotes, des cellules de levure et des cellules animales, en particulier des cellules de mammifères transformées par au moins une séquence nucléotidique et/ou un vecteur tel que décrit précédemment.

Selon un mode de réalisation particulier, les cellules (cellules dendritiques, macrophages, astrocytes, lymphocytes T CD4+, lymphocytes T CD8+) du patient ou allogéniques sont placées en contact d'une préparation purifiée du polypeptide cible, celui-ci étant internalisé, apprêté et présenté à la surface cellulaire associé aux molécules du CMHI et/ou CMHII. Elles peuvent entre autre induire une réponse immune spécifique contre le peptide. Les cellules " activées " sont ensuite administrées au patient, chez lequel elles peuvent entre autre induire une réponse immune spécifique des antigènes (on utilise une voie naturelle de la réponse immune, mais on contrôle ce que la cellule présentatrice de l'antigène va présenter)

Selon un mode de réalisation particulier, les cellules présentatrices d'antigène (cellule dendritique, macrophage, astrocytes,..) sont modifiées *in vitro* pour exprimer les antigènes dans la cellule transformée, qui peuvent être sécrétées par la cellule et/ou s'associer aux molécules du CMHI et/ou CMHII et être présentées à la surface des cellules pour induire chez le patient chez lequel on administre la cellule modifiée une réaction immune parfaitement ciblée.

Toutes les approches vaccinales ne sont pas toujours satisfaisantes et conduisent par exemple à des réactions immunes limitées dirigées uniquement à l'encontre d'épitopes immunodominants ou contre des antigènes présentant une grande variabilité. De même la présentation incorrecte des antigènes par les glycoprotéines du système CMH à la surface des cellules, ne permet pas de développer chez le patient traité une immunité anti-protéine d'intérêt convenable. Afin de pallier ces problèmes, certains auteurs ont proposé dans le cadre de tels procédés vaccinaux, de sélectionner les fragments minimaux antigéniques correspondant aux portions de peptide susceptibles d'être reconnus spécifiquement par les lymphocytes T cytotoxiques, de les exprimer dans les cellules afin qu'ils s'associent aux molécules du CMHI et soient présentés à la surface des cellules pour induire chez le patient traité une réaction immunitaire parfaitement ciblée (Toes et al. 1997, PNAS 94: 14660-14665). Plus particulièrement, il a été montré que des épitopes de très petites tailles (variant de 7 à environ 13 acides aminés) qui sont exprimés à partir de minigènes introduits dans un virus de la vaccine, pouvaient induire une immunisation de type cellulaire. Il a par ailleurs été montré que plusieurs minigènes pouvaient être exprimés conjointement à partir d'un même vecteur (cette construction particulière est appelée 'string of beads'). Une telle construction présente l'avantage d'induire une réaction immune de type CTL synergique (Whitton et al ;, 1993 J. of Virology 67 : 348-352).

Protocole de mise en contact des cellules et du fragment antigénique :

La présentation des fragments antigéniques par les molécules CMHI et/ou CMHII repose sur un procédé intracellulaire identifié (voir Groettrup et al., 1996 Immunology Today 17 : 429-435 pour une revue) au cours duquel des peptides antigéniques de très courtes tailles (environ 7-13 acides aminés) sont produits par dégradation d'un polypeptide plus complexe contre lequel la réaction immune finale sera dirigée. Ces courts peptides sont ensuite associés aux molécules du CMHI ou du CMHII pour former un complexe protéique qui est transporté à la surface cellulaire afin de présenter lesdits peptides aux lymphocytes T cytotoxiques circulants ou aux lymphocytes T helper circulants, respectivement. II convient en outre de noter que la spécificité des molécules CMH I ou CMH II vis-à-vis des peptides antigéniques varie en fonction des molécules CMH I ou CMH II (exemple pour le CMHI : HLA-A, HLA-B, ...) et de l'allèle (exemple pour le CMH I : HLA-A2, HLA-A3, HLA-A11) considérés. Au sein d'une même espèce animale, d'un individu à l'autre, il existe une grande variabilité des gènes codant pour les molécules du système CMH (à ce sujet, voir notamment George et al., 1995, imunology Today 16 : 209-212).

Selon un mode de réalisation particulier, les cellules, telles que les cellules dendritiques, les macrophages, les astrocytes, les lymphocytes T CD4+, les lymphocytes T CD8+, sont modifiées de manière à exprimer à leur surface des anticorps spécifiques du peptide ciblé. Le peptide est neutralisé par les anticorps exprimés à la surface des cellules. Ces cellules sont de préférence immunes, de préférence du patient, de préférence cytotoxiques, modifiées pour exprimer tout ou partie d'un anticorps spécifique du polypeptide cible.

Isolement de cellules mononucléées à partir de sang périphérique :

En 1968, Boyum décrivit une technique rapide qui permet par centrifugation du sang sur gradient de densité, de séparer les cellules mononucléées (lymphocytes et monocytes) avec un bon rendement (rendement théorique 50%, c'est-à-dire 10⁶ cellules /ml de sang). 50 ml de sang périphérique prélevés stérilement dans des tubes héparinés sont centrifugés 20 minutes à 150g à 20°C. Les cellules récupérées sont diluées dans deux volumes de sang périphérique initial de PBS stérile. 10 ml de cette suspension sont déposés sur 3ml d'une solution de ficoll-Hypaque (milieu de séparation des lymphocytes, Flow). Après centrifugation pendant 20 minutes à 400g et 20°C sans freinage de décélération , les cellules mononucléées sédimentent à l'interface PBS-Ficoll, en une couche dense, opalescente, alors que la quasi-totalité des globules rouges et des polynucléaires sédimentent au fond du tube. Les cellules mononucléées sont récupérées et lavées en PBS stérile.

Internalisation des antigènes par les cellules présentatrices de l'antigène :
Traitement préalable des cellules présentatrices de l'antigène : les cellules présentatrices de l'antigène sont préalablement lavées avec un tampon PBS-BSA à 0.5% (p/v) puis énumérées puis elle sont préincubées en présence de différents inhibiteurs de réduction trois fois en PBS-BSA 0.5% contenant de 10 µM à 10 mM final de DTNB (acide 5,5'-dithio-bis-2-nitrobenzoïque) ou de NEM (N-éthylmaléimide). Les étapes ultérieures de fixation d'antigènes à la surface cellulaire ou d 'internalisation d'antigènes se réalisent aussi en présence des différentes concentrations d'inhibiteurs.

Protocole d'internalisation des antigènes par les cellules présentatrices de l'antigène :
8.10⁶ cellules sont incubées en présence de quantité saturante de protéines radiomarquées à l'iode 125 (1 µg) dans des micropuits dans 70 µl. Après une heure d'incubation à 4°C sous agitation, les antigènes sont fixés à la surface des cellules. La suspension cellulaire est lavée deux fois en PBS-BSA et les culots cellulaires sont repris dans 70 µl de tampon et incubées à 37°C pendant différentes périodes allant jusqu'à 2 heures. Cellules et surnageants sont séparés par centrifugation à 800g pendant 5 minutes 4°C. Pour des plus longues périodes d'incubation, l'étape préliminaire de pré-fixation des antigènes à la surface des cellules est supprimée. Les cellules sont diluées dans un milieu RPMI-10% SVF en présence de 20 mM Hépès, à 10⁶cellules /ml. Les cellules sont incubées en présence d'un excès d'antigène pendant différentes périodes à 37°C (1 µg de molécules /5.10⁷ cellules monocytes/macrophages ou /10⁸ cellules B-EBV).

A partir des connaissances des séquences en acides aminés des protéines d'intérêt de l'invention, il est à la portée de l'homme de l'art de définir et utiliser les molécules décrites ci-dessus et/ou toute molécule capable de se fixer aux dites molécules, et/ou toute molécule capable d'inhiber l'activité desdites molécules d'intérêt.

L'agent thérapeutique est de préférence choisi parmi une molécule naturelle et/ou une molécule recombinante, ou un fragment desdites molécules, dont la séquence protéique correspond à la séquence des molécules Vβs16 et/ou 17, de préférence Vβ16, éventuellement en association avec une ou des molécules naturelles et/ou recombinantes ou un fragment desdites molécules dont la séquence protéique correspond à la séquences des molécules Vβs 2, 3, 7, 8, 12, 14, 17 et 22 et préférentiellement des molécules Vβs 3 et 12 ou des molécules Vβs 7, 14 et 17, et avantageusement 7 et 17 ;
parmi les molécules naturelles, et/ou recombinantes et/ou de synthèse ou un fragment desdites molécules qui codent pour les molécules telles que définies précédemment.

En particulier l'agent thérapeutique et/ou prophylactique est choisi parmi les molécules ADN et/ou ARN ; les oligonucléotides anti-sens et les oligonucléotides anti-gènes ; au moins un ligand susceptible d'interagir avec les Vβs 16 et/ou 17, en particulier le Vβ316, éventuellement en association avec au moins un ligand susceptible d'interagir avec au moins un des Vβs 2, 3, 7, 8, 12, 14, 17 et 22 et préférentiellement les Vβs 3 et 12 ; parmi les anticorps, de préférence les anticorps monoclonaux et les anti-récepteurs des TCRs des différents Vβs ci-dessus ; au moins un ligand susceptible d'interagir avec les Vβ16 et/ou 17, éventuellement en association avec au moins un ligand susceptible d'interagir avec au moins un des Vβs 7, 14 et 17 et 22 et préférentiellement les Vβs 7 et 17, en particulier les anticorps, et de préférence les anticorps monoclonaux ou des fragments desdits anticorps et les anti-récepteurs des TCRs des différents Vβs ci-dessus ; un agent susceptible de bloquer l'interaction du superantigène aux cellules présentatrices d'antigène ; au moins une cellule modifiée génétiquement *in vitro,* de préférence une cellule d'origine mammifère, par un agent thérapeutique qui consiste en au moins une molécule d'acide nucléique codant pour au moins une molécule dont la séquence protéique correspond à la séquence codant pour les molécules telles que définies précédemment; en particulier une molécule ADN et/ou ARN ; au moins une cellule modifiée génétiquement *in vitro,* de préférence une cellule d'origine mammifère, par un agent thérapeutique qui consiste en au moins une molécule d'acide nucléique codant pour au moins un ligand tel que défini précédemment, en particulier une molécule ADN et/ou ARN.

L'agent thérapeutique est de préférence choisi
parmi une molécule naturelle et/ou une molécule recombinante, ou un fragment desdites molécules, dont la séquence protéique correspond
à la séquence des protéines MSRV-1, et avantageusement à la séquence de la protéine env de MSRV-1, et
parmi les molécules naturelles et/ou recombinantes et/ou de synthèse ou un fragment desdites molécules qui codent pour les molécules telles que définies précédemment.

En particulier, l'agent thérapeutique et/ou prophylactique est choisi parmi les molécules ADN et/ou ARN ; les oligonucléotides anti-sens et les oligonucléotides anti-gènes ; au moins un ligand susceptible d'interagir avec les protéines MSRV-1, en particulier la protéine env de MSRV-1, parmi les anticorps, de préférence les anticorps monoclonaux et les anti-protéines de MSRV-1 en particulier les anti-protéines env de MSRV-1 ; au moins une cellules modifiée génétiquement in vitro, de préférence une cellule d'origine mammifère, par un agent thérapeutique qui consiste en au moins une molécule d'ADN codant pour au moins une molécule dont la séquence protéique correspond à la séquence codant pour les molécules telles que définies précédemment, en particulier une molécule ADN et/ou ARN ; au moins une cellule modifiée génétiquement in vitro, de préférence une cellule d'origine mammifère, par un agent thérapeutique qui consiste en au moins une molécule d'ADN codant pour au moins un ligand tel que défini précédemment, en particulier une molécule d'ADN et/ou ARN.

Ainsi, l'agent thérapeutique est de préférence un agent antiviral, plus particulièrement antiviral, en particulier humain, de préférence anti-MSRV1 tel qu'un inhibiteur du cycle de réplication et/ou de l'expression d'un rétrovirus, tel qu'un anticorps anti-protéine rétrovirale, en particulier anti-enveloppe, tel que des oligonucléotides anti-sens, plus particulièrement bloquant l'expression rétrovirale.

Avantageusement, le ligand est susceptible d'interagir avec un rétrovirus, en particulier un rétrovirus humain, tel que MSRV-1, ses protéines et/ou ses acides nucléiques. En particulier, le ligand est choisi parmi les anticorps anti-MSRV-1, de préférence les anticorps monoclonaux.

Les agents thérapeutiques tels que définis précédemment sont utilisés pour la préparation d'une composition prophylactique et/ou thérapeutique et l'invention porte également sur une telle composition comprenant au moins un desdits agents thérapeutiques en association éventuelle avec un véhicule et/ou un excipient et/ou un adjuvant et/ou un diluant pharmaceutiquement acceptable. II peut s'agir entre autres de compositions vaccinales à base de molécules protéine(s) ou de peptide(s) ou de séquences d'acides nucléiques dérivés des molécules d'intérêt ou de leurs fragments, comme décrit précédemment pour la prophylaxie et/ou la thérapie d'une maladie auto-immune, en particulier la sclérose en plaques. Parmi ces molécules, on peut trouver des antiviraux. Ces protéines et peptides administrés sont caractérisés en ce qu'ils ne doivent pas être toxiques pour l'organisme dans lequel ils sont administrés, ou doivent avoir perdu leur capacité à se fixer à un ligand, et peuvent induire significativement une réponse immune médiée par les lymphocytes T et/ou les anticorps dirigés contre cette protéine. De telles protéines sont dites " modifiées ", cependant leur immunogénicité est conservée. De telles molécules immunogéniques modifiées sont obtenues par un nombre de traitements conventionnels, par exemple la dénaturation chimique ou à la chaleur, la troncation ou la mutation avec délétion, insertion ou remplacement d'acides aminés. Un exemple de troncation consiste en la troncation d'acides aminés à l'extrémité carboxy-terminale pouvant aller jusqu'à 5-30 acides aminés. Les molécules modifiées peuvent être obtenues par des techniques synthétiques et/ou recombinantes ou par des traitements chimiques ou physiques des molécules naturelles.

Les molécules d'intérêt naturelles et/ou recombinantes identifiées dans la présente invention et les séquences peptidiques ou les fragments desdites molécules sont utilisées en vaccination prophylactique et thérapeutique contre les maladies, de préférence la sclérose en plaques. Un vaccin comprend une quantité immunogénique effective de la molécule immunogène en association avec un véhicule pharmaceutiquement acceptable et éventuellement un adjuvant et/ou un diluant. Les véhicules, adjuvants et diluants pharmaceutiquement acceptables sont bien connus de l'homme du métier. On peut citer à titre de référence le Remington's Pharmaceutical Sciences. L'utilisation de compositions vaccinales est particulièrement avantageuse en association avec un diagnostic précoce de la maladie. La protéine immunogène est utilisée dans la préparation de médicament pour la vaccination prophylactique ou thérapeutique. Les protéines d'intérêt peuvent être éliminées de l'organisme sans induire d'effets secondaires indésirables. L'identification de telles molécules ou peptides vaccins est réalisée comme suit : on vérifie que les molécules candidates modifiées comme décrit précédemment (protéines naturelles, recombinantes, peptides) ne sont pas toxiques pour l'organisme, puis on vérifie leur immunogénicité (i) en réalisant un test *in vitro* de prolifération de lymphocytes T CD4+ spécifiques de l'antigène administré (T cell assay) ou un test *in vitro* de cytotoxicité des lymphocytes CD8+ spécifiques de l'antigène administré et (ii) en mesurant entre autre le taux d'anticorps circulants dirigés contre la protéine naturelle. Ces formes modifiées sont utilisées pour immuniser des hommes par des procédures standard avec des adjuvants appropriés.

Les vaccins préparés sont injectables, c'est-à-dire en solution liquide ou en suspension. En option, la préparation peut aussi être émulsifiée. La molécule antigénique peut être mélangée avec des excipients qui sont pharmaceutiquement acceptables et compatibles avec l'ingrédient actif. Des exemples d'excipients favorables sont l'eau, une solution saline, le dextrose, le glycérol, l'éthanol ou des équivalents et leurs combinaisons. Si désiré, le vaccin peut contenir des quantités mineures de substances auxiliaires comme des agents " wetting " ou émulsifiants, des agents qui tamponnent le pH ou des adjuvants comme l'hydroxide d'aluminium, le dipeptide muramyl ou leurs variations. Dans le cas des peptides, leur couplage à une plus grosse molécule (KLH, toxine tétanique) augmente quelquefois l'immunogénicité. Les vaccins sont administrés conventionnellement par injection par exemple sous cutanée ou intramusculaire. Des formulations additionnelles favorables avec d'autres modes d'administration incluent des suppositoires et quelquefois des formulations orales.

En général, la concentration en protéine dans la composition utilisée pour une administration *in vivo* est par exemple de 0,1µg /ml à 20 mg /ml.

La présente invention concerne également l'utilisation de vaccins incluant des molécules d'acides nucléiques qui codent pour les molécules d'intérêt de l'invention ou des peptides immunogènes ou leur fragment(s), non actifs. Les vaccins d'acides nucléiques, en particulier les vaccins ADN, sont administrés généralement en association avec un véhicule pharmaceutiquement acceptable en injection intramusculaire.

Un autre aspect de l'invention concerne des compositions prophylactiques et/ou thérapeutiques qui comprennent, entre autres, des substances naturelles et/ou synthétiques et/ou recombinantes (i) capables de bloquer et/ou d'inhiber l'activité des molécules d'intérêt de l'invention et/ou de leurs fragments, et/ou (ii) capables d'inhiber leur métabolisme, et/ou (iii) capables de réguler l'expression des molécules d'intérêt de l'invention ou leurs fragments, et/ou (iv) capables d'inhiber la fonction et/ou l'expression des ligands des molécules d'intérêt de l'invention ou leurs fragments. Ces substances peuvent être utilisées dans des traitements prophylactiques et thérapeutiques de la maladie, par exemple la SEP, en administrant des quantités effectives. Les substances peuvent être de petites molécules synthétiques ou naturelles, des dérivés des protéines identifiées dans cette invention, des lipides, des glycolipides, des composés chimiques, etc... Les petites molécules peuvent être criblées et identifiées en grande quantité en utilisant des librairies combinatoires chimiques. L'invention concerne également des compositions pharmaceutiques comprenant ces substances en association avec des véhicules et/ou adjuvants et/ou excipients et/ou diluants physiologiques acceptables, et des méthodes pour la préparation de médicaments à utiliser en thérapie ou en prévention de maladies auto-immunes dont la SEP en utilisant ces substances.

Pour identifier de telles molécules, on utilise les tests et protocoles in vitro et in vivo comme décrit ci-dessus, en utilisant des échantillons prélevés de patient et/ou d'animaux non traités ou traités. Les molécules sélectionnées sont testées à différentes concentrations. Ces inhibiteurs sont aussi testés dans des essais de toxicité et pharmacocinétique pour savoir s'ils peuvent représenter des drogues candidates valables. Les petites molécules peuvent être criblées et identifiées en grande quantité en utilisant des librairies combinatoires chimiques.

La présente invention concerne aussi l'utilisation de cellules transformées *in vivo* après injection de vecteurs contenant au moins un gène d'intérêt thérapeutique défini à partir des molécules d'intérêt identifiées ou leurs fragments pour la préparation d'une composition. prophylactique et/ou thérapeutique dite de thérapie génique et ladite composition. II est rappelé que pour la simplicité de l'exposé, on parlera généralement de molécules d'intérêt de l'invention pour désigner le ou les différents Vβ associé(s) à l'activité superantigénique et la ou les protéines de MSRV-1, en particulier la protéine env de MSRV-1.

La composition comprend au moins un vecteur contenant un gène thérapeutique comme décrit ci-dessous, capable d'être introduit dans une cellule cible *in vivo* et d'exprimer le gène d'intérêt thérapeutique *in vivo.* L'avantage repose sur la possibilité de maintenir sur le long terme un niveau basal de molécules exprimées chez le patient traité. Des vecteurs ou acides nucléiques codant pour des gènes d'intérêt thérapeutique sont injectés. Ces vecteurs et acides nucléiques doivent être transportés jusqu'aux cellules cibles et transfecter ces cellules dans lesquelles ils doivent être exprimés *in vivo.*

L'invention concerne l'expression *in vivo* de séquences nucléotidiques et/ou de vecteurs tels que décrit précédemment, c'est-à-dire des séquences correspondant à des gènes d'intérêt thérapeutique codant notamment :
- soit au moins pour une protéine choisie parmi les molécules d'intérêt identifiées dans la présente invention ou leurs fragments, et/ou
- soit au moins pour tout ou partie d'un anticorps polyclonal ou monoclonal capable de se fixer à au moins une protéine choisie parmi les molécules d'intérêt identifiées dans la présente invention et leurs fragments. Il peut s'agir d'anticorps transmembranaire natif, ou de fragment ou dérivé d'un tel anticorps, pour autant que ledit anticorps, fragment ou dérivé d'anticorps soit exprimé à la surface de la cellule cible de mammifère génétiquement modifiée et en ce que ledit anticorps est capable de se fixer à un polypeptide présent à la surface d'une cellule effectrice cytotoxique ou d'un lymphocyte T " helper " et d'inhiber l'activité' d'au moins une molécule d'intérêt de l'invention. Il peut s'agir de fragments d'anticorps exprimés par des cellules capables de sécréter lesdits anticorps dans la circulation sanguine d mammifère ou patient porteur des cellules génétiquement modifiées par le gène codant pour l'anticorps, et/ou
- soit au moins pour une molécule inhibitrice d'au moins une protéine choisie parmi les molécules identifiées dans la présente invention ou leurs fragments, et/ou
soit au moins pour un ligand ou toute partie du ligand capable de se fixer sur au moins une protéine choisie parmi les molécules d'intérêt identifiées dans la présente invention ou leurs fragments, et/ou d'inhiber sa fonction. A partir des séquences en acides aminés des molécules d'intérêt de l'invention ou de leurs fragments , il est à la portée de l'homme de l'art de déduire les séquences nucléotidiques ADN et ARN correspondantes aux molécules d'intérêt ou à leurs fragments en se servant du code génétique et en tenant compte de sa dégénérescence. Ainsi la présente invention concerne l'utilisation de ces séquences nucléotidiques sous forme de séquences anti-sens, de séquences codant pour un gène thérapeutique, de séquences pouvant être contenues dans un vecteur pour la réalisation de transformation cellulaire *ex vitro* et/ou *in vivo* (thérapie génique).

Selon un mode de réalisation particulier, il s'agit d'utiliser la thérapie génique de manière à diriger la réponse immune contre la protéine, le peptide ou la molécule d'intérêt cible, c'est-à-dire contre toute molécule choisie parmi les molécules d'intérêt de l'invention et/ou leurs fragments, et/ou contre toute molécule inhibitrice de l'activité et/ou de l'expression et/ou du métabolisme desdites molécules d'intérêt, et/ou des ligands desdites molécules. Pour cela il est évident que les cellules à cibler pour la transformation avec un vecteur sont des cellules appartenant au système immun, soit des cellules présentatrices de l'antigène (cellules dendritiques, macrophages, ...), soit des cellules de type lymphocytes (CD4/CD8).

Selon un mode de réalisation particulier, on modifie génétiquement, notamment *in vivo,* les cellules présentatrices de l'antigène (APC). Les APCs comme les macrophages, les cellules dendritiques, les microgliocytes, les astrocytes jouent un rôle dans l'initiation de la réponse immune. Elles sont les premiers composants cellulaires qui capturent l'antigène, l'apprête dans la cellule et expriment des molécules du CMHI et CMHII transmembranaires impliquées dans la présentation de l'immunogène aux cellules T CD4 + et CD8 +, elles produisent des protéines accessoires spécifiques qui participent à l'activation des cellules T via la reconnaissance des déterminants Vβ du récepteur T à la surface des lymphocytes T (Debrick et al;, 1991, J. Immunol 147 : 2846 ; Reis et al., 1993, J Ep Med 178 : 509 ; Kovacsovics-bankowski et al., 1993, PNAS 90 : 4942; Kovacsovics-bankowski et al., 1995 Science 267 : 243 ; Svensson et al., 1997, J lmmunol 158 : 4229 ; Norbury et al ;, 1997, Eur J Immunol 27 : 280). Pour une vaccination, il peut être avantageux de disposer d'un système de thérapie génique qui peut cibler le transfert de gène dans de telles cellules APC.

On choisit d'exprimer à la surface des cellules APCs *in vivo* tout ou partie d'un anticorps et/ou d'un ligand, capable de réagir avec la protéine ou le peptide cible choisis parmi les molécules d'intérêt de l'invention et/ou leurs fragments. De telles cellules vont alors spécifiquement phagocyter ladite protéine ou ledit peptide, l'apprêter de façon à ce que des fragments de ce peptide soient présentés à la surface des cellules présentatrices de l'antigène.

La littérature propose un grand nombre d'exemples de gènes codant pour des anticorps capables de réagir avec des polypeptides ou récepteurs. II est à la portée de l'homme de l'art d'obtenir les séquences d'acides nucléiques codant pour de tels anticorps. Citons par exemple les gènes codant pour les chaines légères et lourde de l'anticorps YTH 12.5 (anti-CD3) (Routledge et al. 1991, Eur J Immunol 21 : 2717-2725), de l'anti-CD3 selon Arakawa et al ; 1996, J. Biochem. 120 : 657-662. Les séquences d'acide nucléique de tels anticorps sont aisément identifiables à partir des bases de données communément utilisées par l'homme du métier. Il est également possible à partir d'hybridomes disponibles auprès de l'ATCC de cloner les séquences d'acides nucléiques codant pour les chaines lourdes et/ou légères de ces différents anticorps par les méthodes d'amplification telles que la RT-PCR à l'aide d'oligonucléotides spécifiques ou les techniques mettant en oeuvre des banques de cDNA (Maniatis et al ., 1982, Molecular cloning. A laboratory manual CSH Laboratory, Cold Spring Harbor, New York). Les séquences ainsi clonées sont alors disponibles pour leur clonage dans des vecteurs. Selon un cas préféré de l'invention, la séquence d'acide nucléique codant pour la chaîne lourde de l'anticorps est fusionnée par recombinaison homologue avec une séquence d'acide nucléique codant pour un polypeptide transmembranaire (Polydefkis et al., 1990J Exp Med 171 : 875-887). Ces techniques de biologie moléculaire ont été parfaitement bien décrites.

On choisit d'exprimer à la surface des cellules APCs *in vivo* des fragments immunogènes correspondant à au moins une protéine choisie parmi les molécules d'intérêt de l'invention et/ou leurs fragments. Pour cela, on peut choisir de faire exprimer par le vecteur soit le polypeptide complet soit de manière préféré des polypeptides sélectionnés pour réagir avec des ligands et/ou récepteurs spécifiques. Le peptide immunogène codé par le polynucléotide introduit dans la cellule du vertébré *in vivo* peut être produit et/ou sécrété, apprêté puis présenté à une cellule présentatrice de l'antigène (APC) dans le contexte des molécules du CMH. Les APC ainsi transférées *in vivo* induisent une réponse immune dirigée contre l"immunogène exprimé *in vivo.* Les APC possèdent différents mécanismes pour capturer les antigènes : (a) la capture des antigènes par des récepteurs membranaires comme les récepteurs aux immunoglobulines (Fc) ou pour le complément disponibles à la surface des granulocytes, des monocytes ou macrophages permettant une délivrance efficace de l'antigène dans les compartiments intracellulaires après phagocytose médiée par les récepteurs. (b) l'entrée dans les APC par pinocytose en phase fluide, impliquant différents mécanismes : la micropinocytose c'est-à-dire la capture de petites vésicules (0.1µm) par les puits recouverts de clathrine et la macropinocytose c'est-à-dire la capture de plus grosses vésicules (avec une taille variant ente 0.5 µm et environ 6 µm) (Sallusto et al. 1995, J Exp Med 182 : 389-400). Tandis que la micropinocytose existe de façon constitutive dans toutes les cellules, la macropinocytose est limitée à des types cellulaires, comme par exemple les macrophages, les cellules dendritiques, les astrocytes, les cellules épithéliales stimulées par des facteurs de croissance (Racoosin et al., J Cell Sci 1992, 102 : 867-880). Dans cette invention, on entend par cellules capables de macropinocytose, les cellules qui peuvent réaliser les événements décrits ci-dessus et les cellules qui peuvent capturer des macromolécules de préférence entre 0.5 µm et environ 6 µm dans le cytoplasme.

Selon un mode de réalisation particulier, on modifie génétiquement notamment *in vivo,* les cellules effectrices cytotoxiques ou les lymphocytes T " helper " de façon à ce qu'elles expriment à leur surface des ligands d'au moins une desdites molécules d'intérêt de l'invention, naturellement non exprimés par ces cellules, et capables de se fixer à tout ou partie d'au moins une des molécules d'intérêt de l'invention à la surface de la même cellule ou d'une autre cellule et d'inhiber l'activité' d'au moins une molécule d'intérêt de l'invention, par l'introduction dans ces cellules de séquences d'acide nucléique renfermant le gène codant pour un tel polypeptide. Conformément à la présente invention, il est également possible de sélectionner une séquence d'acide nucléique contenant un gène d'intérêt thérapeutique codant pour tout ou partie d'un anticorps dirigé contre une protéine choisie parmi les molécules d'intérêt de l'invention et les séquences peptidiques et/ou les fragments desdites séquences, susceptible d'être exprimé à la surface des cellules cibles du patient à traiter, ledit anticorps étant capable de se fixer à un polypeptide par ces cellules effectdes molécules d'intérêt de l'invention présentes à la surface des lymphocytes cytotoxiques et/ou lymphocytes T "helper", voire d'inhiber l'activité' de ces molécules d'intérêt.

De nombreux outils ont été développés pour introduire différents gènes hétérologues et/ou vecteurs dans des cellules, en particulier des cellules de mammifères. Ces techniques peuvent être divisées en deux catégories : la première catégorie implique des technique physiques comme la micro-injection, l'électroporation ou le bombardement de particules. La seconde catégorie est basée sur l'utilisation de techniques en biologie moléculaire et cellulaire avec lesquelles le gène est transféré avec un vecteur biologique ou synthétique qui facilite l'introduction du matériel dans la cellule *in vivo.* Aujourd'hui, les vecteurs les plus efficaces sont les vecteurs viraux en particulier les adénoviraux et rétroviraux. Ces virus possèdent des propriétés naturelles pour traverser les membranes plasmiques, éviter la dégradation de leur matériel génétique et introduire leur génome dans le noyau de la cellule. Ces virus ont été largement étudiés et certains sont déjà utilisés expérimentalement dans des applications humaines en vaccination, en immunothérapie, ou pour compenser des déficiences génétiques. Cependant cette approche virale a des limitations notamment due à la capacité de clonage restreinte dans ces génomes viraux, le risque de disséminer les particules virales produites dans l'organisme et l'environnement, le risque de mutagénèse artéfactuelle par insertion dans la cellule hôte, dans le cas des rétrovirus, et la possibilité d'induire une forte réponse immune inflammatoire *in vivo* pendant le traitement, ce qui limite le nombre d'injections possibles (Mc Coy et al. 11995, human Gene Therapy 6 : 1553-1560 ; Yang et al., 1996 Immunity 1 : 433-422). D'autres systèmes alternatifs à ces vecteurs viraux existent. L'utilisation de méthodes non virales comme par exemple la co-précipitation avec le phosphate de calcium, l'utilisation de récepteurs qui miment les systèmes viraux (pour un résumé voir Cotten et Wagner 1993, Current Opinion in Biotehcnology, 4 : 705-710), ou l'utilisation de polymères comme les polyamidoamines (haensler et Szoka 1993, Bioconjugate Chem 4 : 372-379). D'autres techniques non virales sont basées sur l'utilisation de liposomes dont l'efficacité pour l'introduction de macromolécules biologiques comme l'ADN, l'ARN des protéines ou des substances pharmaceutiques actives a été largement décrite dans la littérature scientifique. Dans ce domaine des équipes ont proposé l'utilisation de lipides cationiques ayant une forte affinité pour les membranes cellulaires et/ou les acides nucléiques. En fait il a été montré qu'une molécule d'acide nucléique elle-même pouvait traverser la membrane plasmique de certaines cellules *in vivo* (WO 90/110921, l'efficacité étant dépendante en particulier de la nature polyanionique de l'acide nucléique. Dès 1989 (Felgner et al ;, Nature 337 : 387-388) les lipides cationiques ont été proposés pour faciliter l'introduction de larges molécules anioniques, ce qui neutralise les charges négatives de ces molécules et favorise leur introduction dans les cellules. Différentes équipes ont développés de tels lipides cationiques : le DOTMA (Felgner et al., 1987, PNAS 84: 7413-7417), leDOGS ou Transfectam^{™} (Behr et al., 1989, PNAS 86 : 6982-6986), le DMRIE et le DORIE (Felgner et al., 1993 methods 5 : 67-75), le DC-CHOL (Gao et Huang 1991, BBRC 179 : 280-285), le DOTAP^{™} (McLachlan et al., 1995, Gene therapy 2 : 674-622) ou la Lipofectamine^{™}, et les autres molécules décrites dans les brevets WO9116024, WO9514651, W09405624. D'autre groupes ont développés des polymères cationiques qui facilitent le transfert de macromolécules en particulier des macromolécules anioniques dans les cellules. Le brevet WO95/24221 décrit l'utilisation de polymères dendritiques, le document WO96/02655 décrit l'utilisation du polyethyleneimine ou polypropyleneimine et les document US-A-5595897 et FR 2719316, l'utilisation des conjugués polylysine.

Etant donné que l'on souhaite obtenir *in vivo* une transformation ciblée vers un type cellulaire donné, il est évident que le vecteur utilisé doit pouvoir être lui-même " ciblé " (comme décrit ci dessus).

Le matériel biologique défini dans la présente invention peut être administré *in vivo* notamment sous forme injectable. On peut également envisager une injection par voie épidermique, intraveineuse, intra-artérielle, intramusculaire, intracérébrale par seringue ou tout autre moyen équivalent. Selon un autre mode de réalisation par administration orale ou tout autre moyen parfaitement connu de l'homme de l'art et applicable à la présente invention. L'administration peut avoir lieu en dose unique ou répétée, une ou plusieurs fois après un certain délai d'intervalle. La voie d'administration et le dosage les mieux appropriés varient en fonction de différents paramètres tels que par exemple l'individu ou la maladie à traiter, du stade et/ou de l'évolution de la maladie, ou encore de l'acide nucléique et/ou de la protéine et/ou peptide et/ou molécule et/ou cellule à transférer ou de l'organe/tissus cible.

D'autres objets de l'invention sont les suivants :
- un procédé pour identifier des substances capable de bloquer la transcription et/ou la traduction d'un rétrovirus humain, en particulier en rétrovirus MSRV-1 ou un variant de MSRV-1 tel que défini précédemment, et présentant une activité superantigénique, ladite activité superantigénique étant associée à une maladie auto-immune selon lequel,
on met en contact la substance avec des cellules exprimant un polypeptide rétroviral tel que défini précédemment et ayant une activité superantigénique, et
on détecte une perte ou diminution de l'activité superantigénique selon l'invention.
- un kit pour le criblage de substances capables de bloquer l'activité superantigénique d'un rétrovirus, en particulier le rétrovirus MSRV-1 ou un variant de MSRV-1 tel que défini précédemment, associée à une maladie auto-immune, ou capable de bloquer la transcription et/ou la traduction dudit rétrovirus, comprenant :
   des cellules exprimant à leur surface des produits du MHC de classe II, transformées avec et exprimant fonctionnellement un superantigène rétroviral,
   des cellules porteuses de chaînes du récepteur d'un Vβ ou de Vβs stimulées par le superantigène rétroviral, et
   des moyens pour détecter une perte ou diminution de l'activité superantigénique selon l'invention.

### Définitions :

Par fragment d'anticorps on entend les fragments F(ab)2, Fab', Fab, sFv (Blazar et al., 1997, Journal of Immunology 159 : 5821-5833 ; Bird et al., 1988 Science 242 : 423-426) d'un anticorps natif, et par dérivé on entend, par exemple, un dérivé chimérique d'un tel anticorps (voir par exemple les chimères des anticorps antiCD3 Souris/Homme dans Arakawa et al., 1996 J Biochem 120 : 657-662 ou les immunotoxines telles que sFv-toxine de Chaudary et al 1989, Nature 339 : 394-397).

Par anticorps transmembranaire on entend un anticorps dont au moins la région fonctionnelle capable de reconnaître et de se fixer à son antigène spécifique est exprimée à la surface des cellules cibles pour permettre lesdites reconnaissance et fixation. Plus particulièrement, les anticorps selon la présente invention consistent en des polypeptides de fusion comprenant les amino acides définissant ladite région fonctionnelle et une séquence d'amino acides (polypeptide transmembranaire) permettant l'ancrage au sein de la double couche lipidique membranaire de la cellule cible ou à la surface externe de cette bi-couche. Les séquences nucléiques codant pour de nombreux polypeptides transmembranaires sont décrites dans la littérature. Selon un cas tout à fait avantageux, la séquence d'acide nucléique codant pour la chaîne lourde de l'anticorps est fusionnée avec la séquence d'acide nucléique codant pour undit polypeptide transmembranaire.

Par éléments assurant l'expression d'un gène *in vivo,* on fait notamment référence aux éléments nécessaires pour assurer l'expression dudit gène après son transfert dans une cellule cible. II s'agit notamment des séquences promotrices et/ou des séquences de régulation efficaces dans ladite cellule, et éventuellement les séquences requises pour permettre l'expression à la surface des cellules cibles dudit polypeptide. Le promoteur utilisé peut être un promoteur viral, ubiquitaire ou spécifique de tissu ou encore un promoteur synthétique. A titre d'exemple, on mentionnera les promoteurs tels que les promoteurs des virus RSV (Rous Sarcoma Virus), MPSV, SV40 (Simian Virus), CMV (Cytomegalovirus) ou du virus de la vaccine, les promoteurs du gène codant pour la créatine kinase musculaire, pous l'actine. II est en outre possible de choisir une séquence promotrice spécifique d'un type cellulaire donné, ou activable dans des conditions définies. La littérature procure un grand nombre d'informations relatives à de telles séquences promotrices.

Par cellules effectrices cytotoxiques, on entend les macrophages, les astrocytes, les lymphocytes T cytotoxiques (TCL) qui expriment à leur surface les molécules CD8, et les cellules tueuses (NK) ainsi que leurs dérivés telles que par exemple les LAK (Versteeg 1992 Immunology today 13 : 244-247 ;Brittende et al 1996, Cancer 77 :1226-1243). Par 'lymphocytes T helper' on entend désigner notamment les cellules exprimant à leur surface les molécules CD4 qui permettent après activation la sécrétion de facteurs d'activation des cellules effectrices de la réponse immune. Les polypeptides et notamment les récepteurs exprimés à la surface de ces cellules et qui sont impliqués dans l'activation de telles cellules consistent notamment en tout ou partie du complexe TCR comprenant les déterminants Vb et/ou le CD3, tout ou partie des complexes CD8, CD4, CD28, LFA-1, 4-1 BB (Melero et al., 1998, Eur J lmmunol 28 : 1116-1121), CD47, CD2, CD1, CD9, CD45, CD30, CD40, tout ou partie des récepteurs de cytokines (Finke et al., 1998, Gene therapy 5 : 31-39), telles que IL-7, IL-4, IL-2, IL-15 ou GM-CSF, tout ou partie du complexe récepteur des cellules NK tel que par exemple NKAR, Nkp46, .. ; (Kawano et al., 1998 Immunology 95 :5690-5693 ; Pessino et al., 1998 J Exp Med188 :953-960), Nkp44, tout ou partie des récepteurs de macrophages tels que par exemple le récepteur Fc (Deo et al., 1997, Immunology Today 18 : 127-135).

Par efficacité thérapeutique de manière générale, on entend l'effet inhibiteur de l'activité de type superantigénique telle que définie par les profils Vβs de l'invention et la capacité d'inhiber et/ou de bloquer les interactions moléculaires et/ou immunologiques des Vβs de l'invention conduisant à leur expansion ou diminution telle que décrite précédemment.

Par surnageant ou fraction de surnageant on entend le surnageant ou une fraction de ce dernier jusqu'aux molécules, peptides et/ou protéines qu'ils compennent.

Les expériences ont été réalisées à partir :
1) de cellules mononucléées sanguines provenant de onze donneurs sains et purifiées sur un gradient de Ficoll. Le typage HLA DR de chaque donneur a été effectué au préalable.
2) (i) d'un pool de surnageants de culture concentré par ultracentrifugation (100 000g x 2 heures) de lymphocytes B humains provenant de patients SEP (LBSEP) ou de témoins non-SEP (LBTE), et (ii) de surnageant de culture de cellules de plexus choroïdes humains provenant d'un patient SEP (GRE) et d'un témoin non-SEP (LES).
3) de protéines recombinantes.

### Exemple 1 : Préparation d'extraits de surnageants de culture de cellules provenant de patients atteints de SEP ou de témoins non SEP.

Les protocoles de culture des cellules de plexus choroides ont été décrits dans la demande de brevet PCT WO 93/20188.

### 1) Culture des cellules de plexus choroïdes.

Les cellules de plexus choroïdes sont obtenues à partir d'explants prélevés post-mortem et mis en culture, éventuellement après dissociation mécanique et/ou enzymatique des tissus prélevés. Les cellules qui prolifèrent dans la culture sont d'allure fibroblastique et correspondent à des cellules d'origine leptoméningées, parfois appelées "fibroblastes de plexus choroïdes". Elles peuvent être cultivées pendant un certain nombre de passages et être congelées lors de passages intermédiaires et décongelées ultérieurement pour une nouvelle culture.

Les cellules LES (témoin non-SEP) et GRE (SEP) ont été décongelées puis remises en culture dans du milieu F-12 "complet" contenant :
pénicilline 200 U/ml
streptomycine 20 mg/l
L-glutamine 6 mM
pyruvate de sodium 1 %
acides aminés essentiels 1%.
anticorps anti-IFN leucocytaire (anti-interféron alpha polyclonal commercialisé par Sigma) ajouté à 10 U/ml final.

Ce milieu est supplémenté de 30% de SVF (sérum de veau foetal) décomplémenté à 56°C pendant 30 min La culture cellulaire se fait dans une étuve à 37°C humidifiée, en présence de 5% de CO₂. Le changement du milieu s'effectue deux fois par semaine. Si les cellules adhérentes sont à confluence dans le fond du flacon, la culture est dédoublée. Pour ce faire, le surnageant de culture (SC) est éliminé et remplacé par du milieu F12 complet-SVF 30% "frais". A l'aide d'un grattoir, ou d'un mélange trypsine-EDTA, les cellules sont décollées de la paroi du flacon. Puis, elles sont resuspendues soigneusement avant d'être partagées dans deux flacons de culture. On dit que la culture est dédoublée ou qu'elle a subi un passage.

Les SC sont recueillis et congelés à -80°C pour l'ultracentrifugation, après élimination des débris cellulaires par centrifugation à 3000 tours/min pendant 30 min. Pour l'étude, un volume total de 400 ml environ a été décongelé, regroupé et homogénéisé avant ultracentrifugation, pour chaque culture (SEP/GRE et NON-SEP/LES).

### 2) Culture des lignées B lymphoblastoïdes.

- Obtention de lymphocytes B à partir du sang périphérique et établissement de lignées B lymphoblastoïdes immortalisées par l'EBV (Virus d'Epstein Barr).

Les lymphocytes humains sont isolés à partir de 50 ml de sang hépariné dilué au demi avec du RPMI 1640 par centrifugation sur un gradient de Ficoll. Ils sont délicatement recueillis de l'anneau ainsi que des éventuels agrégats cellulaires pouvant flotter juste au dessous de l'anneau. Les cellules sont alors lavées deux fois dans du milieu RPMI 1640. Après ces lavages, les cellules sont resuspendues à la concentration de 2x10⁶ cellules/ml dans du milieu RPMI 1640 contenant :
pénicilline 200 U/ml
streptomycine 20 mg/l
L-glutamine 6 mM
pyruvate de sodium 1 %
acides aminés essentiels 1%.
anticorps anti-IFN leucocytaire (anti-interféron alpha polyclonal-Sigma) ajouté à 10 U/ml final.

Ce milieu est supplémenté de 20% de SVF (sérum de veau foetal) décomplémenté à 56°C pendant 30 min.

Les flacons de culture sont incubés dans des étuves à 37°C dans une atmosphère humidifiée contenant 5% de CO₂ après avoir ajouté au milieu de culture, 1 ml de surnageant filtré de culture productive EBV B95-8 (soit 10⁵ particules virales EBV pour 4 à 5x10⁶ lymphocytes totaux) en présence de 200 µl d'une solution de CSA-Sandoz (2 µg de CSA pour 4 à 5x10⁶ lymphocytes totaux) et ceci, jusqu'au premier changement de milieu de culture. Le changement du milieu de culture s'effectue deux fois par semaine. Les flacons sont entretenus pendant trois mois, au delà desquels, si aucune prolifération lymphocytaire n'a débuté, le flacon est jeté. Lorsque la prolifération cellulaire a commencé dans un flacon, les cellules sont conservées dans le même flacon jusqu'à ce qu'un nombre significatif de colonies prolifératives forment des amas. Le "dédoublement" de la culture (passage ou repiquage) s'effectue après dissociation mécanique des amas par pipetage et refoulement vigoureux. Les cultures ainsi obtenues correspondent à des lignées de lymphocytes B (lignées Lymphoblastoïdes). Quatre lignées provenant de SEP certaines et deux lignées provenant de témoins non-SEP ont été obtenues dans ces conditions.

### - Congélation des cellules.

Les cellules sont lavées dans du milieu RPMI-SVF 20%. Le culot repris dans du SVF (1 ml environ pour 8x10⁶ cellules) est transféré dans un cryotube pour la congélation. 200 µl d'une solution de SVF DMSO préparée à l'avance et conservée à -20°C sont ajoutés à la suspension cellulaire (concentration finale de DMSO=10%) qui est alors légèrement vortexée. Les cellules sont ainsi congelées à -80°C dans du coton cardé, puis stockées le lendemain dans l'azote.
- Décongélation et remise en culture des cellules congelées dans du DMSO.

Les cellules décongelées à température ambiante sont lavées deux fois dans 10 ml de milieu RPMI-SVF 20% avant la remise en culture.

Les quatre cultures SEP et les deux cultures témoins sont remises en culture en même temps, avec les mêmes réactifs et les mêmes lots de milieux, mais cultivées dans des laboratoires de culture séparés (P3 et P2 respectivement). Les cultures individuelles ont été menées en parallèle pendant environ un mois, afin de recueillir un volume, après pool des SC séparés de chaque patient SEP et de chaque témoin, d'environ 600-800 ml de chaque type (LBSEP et LBTE). Ce volume a été réuni par congélation systématique à - 80°C des surnageants prélevés deux fois par semaine après une pré-centrifugation à 1800 tours/min pendant 10 min. pour sédimenter et récupérer les cellules en suspension, suivie d'une deuxième pré-centrifugation à 3000 tours/min pendant 30 min. pour éliminer les débris cellulaires.

Les surnageants SEP et non-SEP ont été décongelés et regroupés séparément et mélangés (SEP = pool LBSEP et témoin = pool LBTE) avant ultracentrifugation (pools de 650 ml). Les culots d'ultracentrifugation d'un même pool centrifugé après répartition dans des tubes séparés de 40 ml, ont aussi été regroupés et mélangés avant aliquotage et congélation de l'extrait à tester, afin d'obtenir un extrait homogène sur tous les aliquotes d'une même origine.

### 3) Détection des mycoplasmes pouvant contaminer la culture.

Un kit (Boehringer) basé sur la technique ELISA (Enzyme-Linked ImmunoSorbent Assay) a été utilisé pour la détection de mycoplasmes afin de garantir l'absence de contamination mycoplasmique dans les cultures.

### 4) Obtention d'un concentrat de surnageant de culture par ultracentrifugation sur coussin de glycérol.

Les surnageants décongelés et préalablement débarrassés des débris cellulaires sont transférés dans des tubes pour ultracentrifugation. Un coussin de glycérol (PBS - glycérol 30%) de quelques cm est alors déposé dans le fond du tube. Après deux heures d'ultracentrifugation à 100 000 x g (calculé au milieu du tube) à 4°C et décélération lente (30 min.), le surnageant est éliminé, le culot repris dans du tampon PBS avec 10% de glycérol, regroupé et homogénéisé avec les culots des autres tubes du même pool d'origine et le tout est aliquoté sous 100 µl. Un aliquot sert pour le dosage d'activité transcriptase inverse (RT) et les autres sont conservés à -80°C. Les aliquots nécessaires sont décongelés pour les tests sur cellules monucléées sanguines.

### Exemple 2 : Préparation de cellules mononucléées sanguines provenant de onze donneurs sains dont le typage HLA DR a été établi.

| Donneurs | Typage HLA DR |
|---|---|
| 1- | DR2-DR4 |
| 2- | DR1-DR1 → DR1 |
| 3- | DR3-DR11 (5) |
| 4- | DR 13-DR8 |
| 5- | DR13(6)-DR14 → DR6 |
| 6- | DR2-DR2 → DR2 |
| 7- | DR3-DR12(5) |
| 8- | DR3-DR7 |
| 9- | DR3-DR13 |
| 10- | DR4-DR5 |
| 11- | DR4-DR4 → DR4 |

Les cellules mononucléées sanguines de 100 ml de sang, provenant des onze donneurs précités, sont séparées des hématies sur gradient de Ficoll (15 ml de Ficoll/25 ml de sang) par centrifugation 15 min. à 800 g. Les cellules sont ensuite lavées avec du milieu RPMI 1640 et recentrifugées dans les mêmes conditions avant d'être aliquotées et congelées dans l'azote gazeux à la concentration finale de 2 x 10⁷ cellules/ml dans une solution de 90% de sérum humain décomplémenté et 10% de DMSO

### Exemple 3 : Mise en culture de cellules mononucléées sanguines de donneurs sains.

Après décongélation rapide, les lymphocytes de donneurs sains (5 x 10⁶ cellules) sont mis en contact 30 min. à 37°C avec 100 µl de surnageants de culture concentrés (i) de LBSEP ou de LBTE ou (ii) de GRE ou de LES.

Après mise en contact, les cellules sont resuspendues dans du milieu de culture (RPMI supplémenté de 10% de sérum humain AB décomplémenté et de gentamycine) et distribuées dans des cupules de plaques de culture à 24 puits à raison de 2 x 10⁶ cellules par ml. La viabilité des cellules et leur immunocompétence ont été vérifiées en introduisant en contrôle une activation par un mitogène classique (PHA à 5 µg / 2 x 10⁶ cellules).

### Exemple 4 : Analyse du répertoire Vβ des lymphocytes CD3 par immuno-détection.

L'analyse du répertoire des CD3 a été réalisée en utilisant un anticorps anti-CD3 couplé à de l'isothiocyanate de fluorescéine et 12 anticorps anti-Vβ couplés à la biotine (Immunotech-Coulter-Beckman). Ces douze anticorps sont répertoriés dans le Tableau 1 ci-dessous.

**Tableau 1**

| Ac anti-Vβ | Référence | Cible Sag | Commentaires |
|---|---|---|---|
| Vβ2 | IM 2081 | TSST-1 | 10% de CD3 + PBL |
| Vβ3 | IM 2109 | SEB | |
| Vβ5-1 | IM 2082 | | 4-7% de CD3 + PBL |
| Vβ7 | IM 2288 | | 1.5-2.4 % de CD3 + PBL |
| | | | Superantigène diabète IDDM (Conrad et al., J Immunol, 1997) |
| Vβ8 | IM 1191 | SEE, N protéine rage | 2.6-5.1 % de CD3 + PBL |
| | | | Superantigène rage (Lafon et al., Nature 1992) |
| Vβ12 | IM 2019 | SEB (12a) | 1.1-1.9% de CD3+PBL |
| Vβ13-1 | IM 2021 | EBV | 1.8-3.3% de CD3 + PBL |
| | | | Superantigène Epstein Barr (Sutkowski et al., J. Exp. Med. 1996) |
| Vβ14 | IM 2022 | | 2.2-5.6% de CD3+PBL |
| Vβ16 | IM 2023 | | 1.1- 2 % de CD3+PBL |
| Vβ17 | IM 1194 | MAM, SEB | 3.3-7% de CD3 + PBL |
| Vβ21-3 | IM 2025 | | 2.2-3.6 % de CD3+PBL |
| Vβ22 | IM 2026 | | 2.4-5.1 % de CD3 + PBL |

Après 24 heures de culture, le contenu des cupules a été cultivé respectivement en présence de LBSEP, LBTE et PHA, ou GRE, LES, et PHA, et est récolté. Le surnageant est aliquoté et conservé à -80°C pour la détection ultérieure de cytokines. Les cellules sont lavées dans du tampon phosphate, réparties dans des microtubes et incubées avec un anticorps anti-CD3 marqué à l'isothiocyanate de fluoréscéine (0,5 µg pour 5 x 10⁵ cellules) et avec chaque anticorps anti-Vβ biotinylé (1 µg d'anticorps pour 2 x 10⁶ cellules), pendant 30 min à 4°C simultanément. Les cellules sont ensuite lavées dans du tampon phosphate et incubées 30 min à 4°C avec de la streptavidine couplée à la phycoérythrine (1 µl dans 50 µl de suspension cellulaire). A l'issue de ce temps d'incubation, les cellules sont lavées et resuspendues dans 250 µl de Cell Fix (nom commercial) avant d'être analysées en cytofluorimétrie. L'immuno-fluorescence est mesurée à l'aide d'un cytofluorimètre FACSCALIBUR (nom commercial) équipé du logiciel CeIIQUEST II (nom commercial) (Becton-Dickinson).

Pour chaque culture et chaque anticorps, les pourcentages de cellules exprimant un Vβ particulier parmi les cellules CD3+ totales sont calculés. La taille des familles est comparée dans les cultures activées par LBSEP, LBTE, LES et GRE). Une augmentation de 31% d'une famille Vβ dans une culture LBSEP ou GRE par rapport aux témoins négatifs est considérée comme une augmentation significative.

### Exemple 5 : Protocole d'analyse du répertoire Vβ des lymphocytes T par biologie moléculaire.

Des extraits de surnageant de culture de cellules provenant de patients atteints de SEP ou de témoins non SEP sont préparés comme décrit dans l'exemple 1, et des cellules PBL de donneurs sains sont préparées comme décrit dans l'exemple 2. Les deux préparations sont mises en culture comme décrit dans l'exemple 3 et les cellules T sont récupérées avec les PBL de la culture. L'analyse du répertoire Vβ du récepteur T présent à la surface de ces cellules est réalisé par biologie moléculaire comme décrit ci-dessous :

Les ARN totaux des cellules lymphoïdes récupérées sont extraits en utilisant des techniques conventionnelles d'extraction, bien connues de l'homme de l'art. Une transcription inverse est réalisée; des ADN complémentaires sont ainsi synthétisés (par exemple en utilisant le kit RT-Expand, Roche). Puis on réalise une amplification spécifique d'une famille de transcrits Vβ (TCRBV) en choisissant des primers spécifiques de la famille Vb étudiée (par exemple en choisissant des primers décrits dans la littérature). (Lue C et al., Am J Clin Pathol 1999 111 : 683 ; Akatsuka Y et al., Tissue Antigens 1999 53 : 122 ; Ryan DK et al., Mol Pathol 1997 50 : 77 ; Lang R et al., J Immunol Methods 1997, 203 : 181 ; Dreitz MJ et al., Vet Immunol Immunopathol 1999 69 : 113 ; Mima T et al., Biochem Biophys Res Commun 1999 263 : 172 ; Aifantis I et al., Immunity 1998 9 : 649 ; Deng X et al., J Biol Chem 1998 273 : 23709 ; Lobashevsky A et al., Transplantation 1996 62 : 1332 ; Hawke NA et al., J Immunol 1996 156 : 2458 ; Lim SH et al., Cancer Immunol Immunother 1996 42 : 69).Des réactions d'extension des amplicons sont ensuite réalisées en utilisant d'une part des oligonucléotides TCR-BC fluorescents, et d'autre part des oligonucléotides TCR-BJ fluorescents (13 J beta). Ces produits d'amplicons marqués à la fluorescence présentent des tailles différentes du fait des réarrangements des gènes spécifiques de chaque clone T Vβ " x " et sont analysés sur gel d'électrophorèse, et la fluorescence associée à chaque bande sur le gel est lue et quantifiée à l'aide d'un Phosphorlmager.

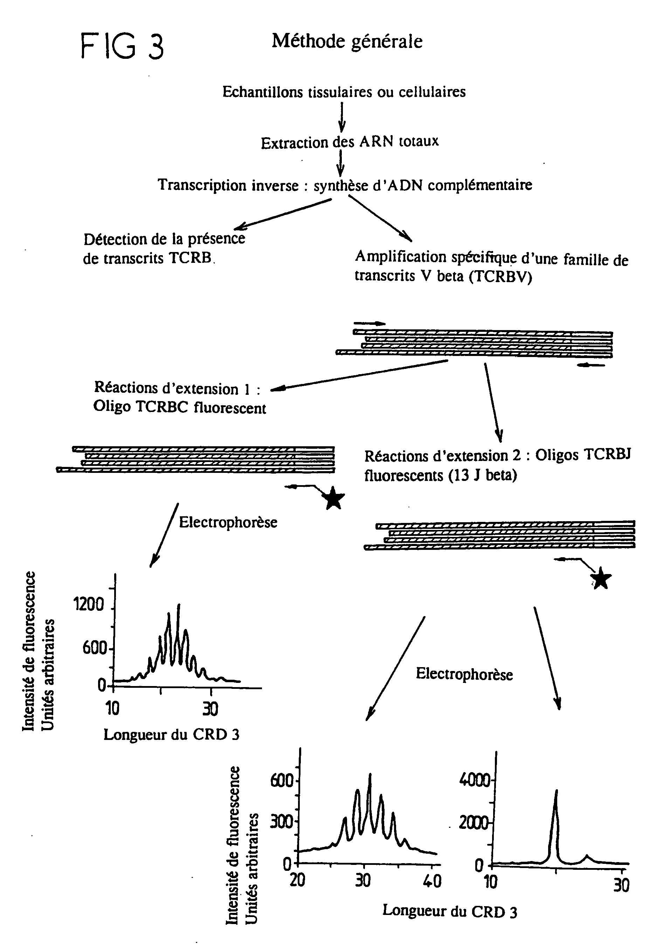

Ainsi on obtient des graphes dont le profil traduit l'amplification d'une famille de Vβ présente à la surface des cellules T (figure 3). Lorsque l'activation de la cellule T est de type polyclonal, plusieurs pics sont observés sur un même graphe, traduisant la présence de nombreuses molécules de la famille Vβ à la surface des cellules T.

### Exemple 6 : Détection des cytokines produites.

Les cytokines inflammatoires (IL-6, TNF-α et INF-γ) ont été mesurées à l'aide de kits ELISA d'immunocapture Optalia (nom commercial) de chez Pharmingen-Becton-Dickinson, sur plaques de titration de 96 puits selon les recommandations du fabricant. Une référence constituée d'une gamme de dilutions de cytokine recombinante de concentration connue (en pg/ml) est incluse sur chaque plaque d'essai. La densité optique est mesurée au spectrophotomètre à la longueur d'onde 405 nm pour la lecture du chromogène ABTS (Boehringer Mannheim). La recherche des cytokines est effectuée dans 50 µl de surnageants de culture 24 heures après la mise en contact pour 8 donneurs stimulés avec LBSEP ou LBTE et dans les surnageants de cultures 24 heures et 72 heures après la mise en contact pour 6 donneurs stimulés avec LES ou GRE.

Les résultats sont exprimés en pg/ml de surnageant, correspondant à la production de 2 x 10⁶ cellules.

**Tableau 2**

| | **LBSEP** | **LBTE** |
|---|---|---|
| **IL-6 (n=6)** | 714 | 220 |
| **INF-y (n = 3)** | 716 | 383 |
| **TNF-α (n = 6)** | 83 | 54 |

### Exemple 7 : Estimation du pourcentage de cellules en apoptose.

Le pourcentage de lymphocytes entrés en apoptose a été mesuré dans les populations de PBL mises en culture pendant 24 heures avec LBSEP ou LBTE- pour 8 donneurs, d'une part, et dans les populations mises en culture avec LES et GRE pour 9 donneurs d'autre part.

L'apoptose a été estimée au cytofluorimètre en prenant en compte les caractéristiques de taille et de granulométrie existant entre les cellules vivantes et les cellules en apoptose, comme décrit pour les lymphocytes Jurkat (Thoulouze *et al* J. Virol.71, 7372-7380, 1997).

Le pourcentage d'apoptose dans les cultures de lymphocytes stimulés avec LBSEP est significativement supérieur à celui obtenu dans les cultures de lymphocytes stimulés par LBTE. (moyenne du pourcentage d'apoptose de 38,35 pour LBSEP contre 28% pour LBTE).

### Exemple 8 : Détection d'antigènes viraux dans les cultures de lymphocytes.

Deux mélanges d'anticorps ont été utilisés pour détecter la présence d'antigènes viraux dans les lymphocytes humains cultivés en présence d'extraits de SC de plexus choroïdes LES et GRE dans les mêmes conditions que pour l'analyse de l'expansion des familles TCR (récepteur des cellules T) Vβ.

On a réalisé (i) un premier pool (pool 1) d'anticorps polyclonaux, obtenus chez le lapin avec 1 µl de chacun des trois anticorps polyclonaux, et (ii) un deuxième pool (pool 2) d'anticorps monoclonaux obtenus après immunisation de souris avec 1 µl de chacun des trois anticorps monoclonaux,. Ces anticorps polyclonaux et monoclonaux sont dirigés contre les protéines codées par les séquences *env, gag* et *pol* de MSRV-1 décrites dans les demandes de brevet WO-A-98/23755 et WO-A-99/02666.

Des lymphocytes humains provenant de donneurs sains (10⁶ cellules par coloration) sont mis en contact 24 heures avec des surnageants ultracentrifugés de cellules de plexus choroïdes LES ou GRE. Les lymphocytes sont ensuite lavés dans du milieu RPMI. Le culot cellulaire est repris dans 500 µl de tampon de fixation (tampon phosphate 3% de paraformaldéhyde) et incubé pendant 20 min. à 4°C. Après deux lavages dans un tampon de perméabilisation (tampon phosphate contenant 1% de sérum de veau foetal, 0,1% d'azide de sodium, 0,1% de saponine), les cellules fixées sont incubées pendant 30 min. à 4°C avec 3 µl de chacun des pools anti-MSRV-1, dans un volume final de 50 µl de tampon de perméabilisation. Après un lavage dans du tampon de perméabilisation, les cellules incubées avec les anticorps anti-MSRV-1 du pool 1 sont incubées 30 min. à 4°C avec des anticorps biotinylés dirigés contre les immunoglobulines de lapin (Byosis, dilution 1/2000), lavées, puis incubées 30 min. à 4°C avec de la strepavidine couplée à de l'isothiocyanate de fluoréscéine (Strep-FITC 1/50°, Immunotech Coulter-Beckman). Les cellules incubées avec les anticorps anti-MSRV-1 du pool 2 sont incubées avec des anticorps couplés à la biotine dirigés contre les anticorps de souris (Amersham, 1/500), puis, après lavage, avec de la Strep-FITC. Les cellules resuspendues dans 250µl de Cell-fix sont ensuite analysées par cytofluorimétrie.

### Exemple 9 : Analyse des répertoires Vβ des lymphocytes CD3.

L'analyse des répertoires des huit donneurs est réalisée par double marquage sur des cellules mises en contact 24 heures, soit avec LBSEP, soit avec LBTE. Ces mêmes donneurs ont été testés en présence de LES et GRE.

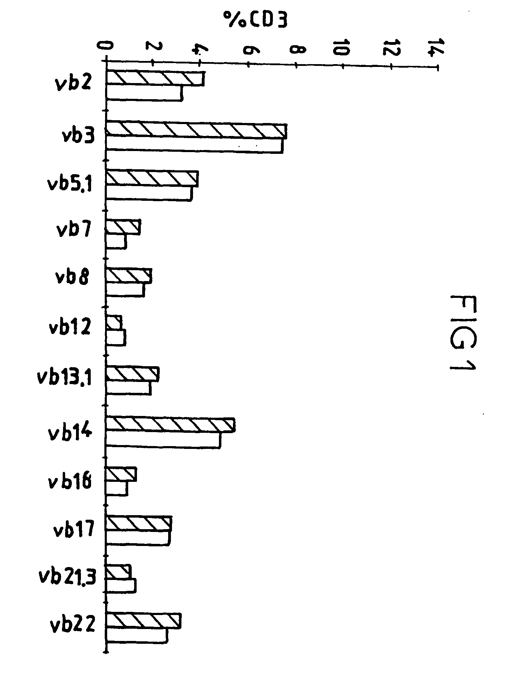

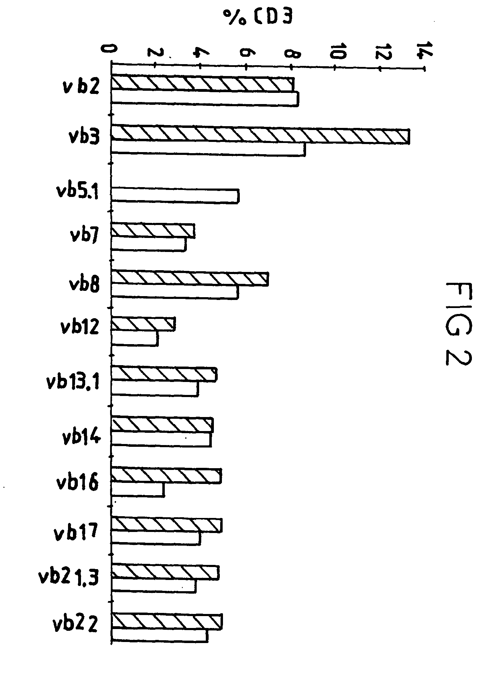

Le pourcentage de cellules exprimant un Vβ particulier parmi les lymphocytes CD3 dans les cultures de lymphocytes stimulées par LBSEP est comparé à celui obtenu dans les cultures stimulées par LBTE. Les répertoires Vβ utilisés par deux donneurs (donneur 1 et donneur 5) en réaction à LBSEP (histogrammes sombres) et à LBTE (histogrammes clairs) sont présentés sur les Figures 1 (donneur 1) et 2 (donneur 5). Pour le donneur 1, les pourcentages de Vβ utilisés pour répondre à LBSEP sont identiques à ceux utilisés pour répondre à LBTE, à l'exception de Vβ 7. En revanche, pour le donneur 5, le pourcentage de Vβ 3 et de Vβ 16 recrutés par LBSEP est différent de ceux recrutés par LBTE (respectivement 8.2 et 13.6 pour Vβ 3 et 2.1 et 4.2 pour Vβ 16).

Une augmentation de 31 % d'une famille Vβ par rapport au pourcentage obtenu dans les cultures de lymphocytes LBTE est considérée comme la marque d'une expansion significative d'une famille de Vβ particulier par LBSEP.

L'analyse comparative utilisant ce critère est présenté dans le tableau 3 ci dessous.

**Tableau 3**

| N° | HLA | Vβ2 | Vβ3 | Vβ5 | Vβ7 | Vβ8 | Vβ12 | Vβ13 | Vβ14 | Vβ16 | Vβ17 | Vβ21 | Vβ22 |
|---|---|---|---|---|---|---|---|---|---|---|---|---|---|
| 1 | DR 2/4 | | | | X | | | | | | | | |
| 4 | DR 13/8 | | | | | | X | | | X | | | |
| 5 | DR 13/14 | | X | | | | | | | X | | | |
| 7 | DR 3/12 | | | | | | | | | | X | | |
| 8 | DR 3/7 | | X | | | | | | | X | | | |
| 9 | DR 3/13 | | | | | X | | | | X | | | |
| 10 | DR 4/5 | | X | | | | X | | X | X | | | X |
| 11 | DR 4/4 | X | | | | | X | | | X | | | |
| | % age | 12,5 | 37,5 | 0 | 13 | 13 | 37,5 | 0 | 12,6 | 75 | 12,5 | 0 | 12,5 |

Après un contact avec les LBSEP, on observe que :
75 % (6/8) des donneurs présentent une expansion de la famille Vβ 16,
37,5 % (3/8) des donneurs présentent une expansion de la famille Vβ 12,
37,5 % (3/8) des donneurs présentent une expansion de la famille Vβ 3, et
12,5 % (1/8) des donneurs présentent une expansion de l'une des familles Vβ 2, 7, 8, 14, 17 ou 22.

Les résultats obtenus avec les préparations LES et GRE peuvent être illustrés par l'analyse des répertoires obtenus avec les donneurs 10 et 11. GRE induit l'expansion de Vβ 16. Les expansions obtenues avec GRE sont du même ordre et de même nature que celles obtenues avec LBSEP.

L'expansion Vβ 16 n'est pas liée à la présence d'un DR particulier, puisqu'elle peut être obtenue aussi bien dans un environnement DR3 que DR4, DR5, DR8, DR13 ou DR14, c'est à dire avec 6 des 8 haplotypes exprimés par les 6 donneurs.

LBSEP n'induit généralement chez les donneurs l'expansion majoritaire que d'une ou deux familles Vβ, sauf dans deux cas : un donneur présente à la fois une expansion des Vβ 3,12,14,16 et 22, un autre à la fois de Vβ 12 et Vβ 16. Les haplotypes de ces deux donneurs sont DR4/4 et DR4/5 respectivement.

### Exemple 10 : Analyse du répertoire Vβ des lymphocytes T par biologie moléculaire.

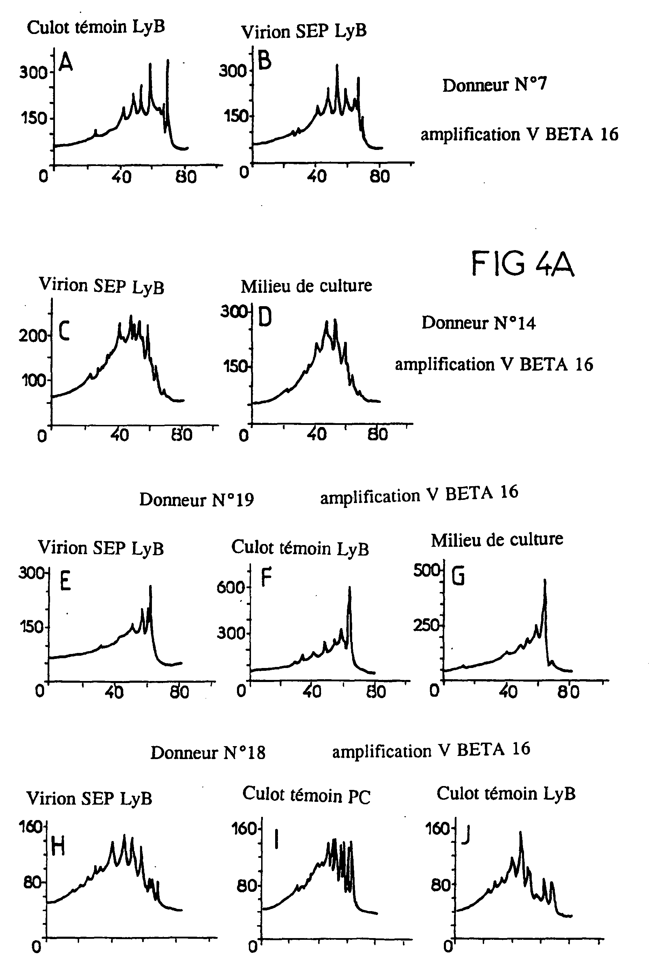

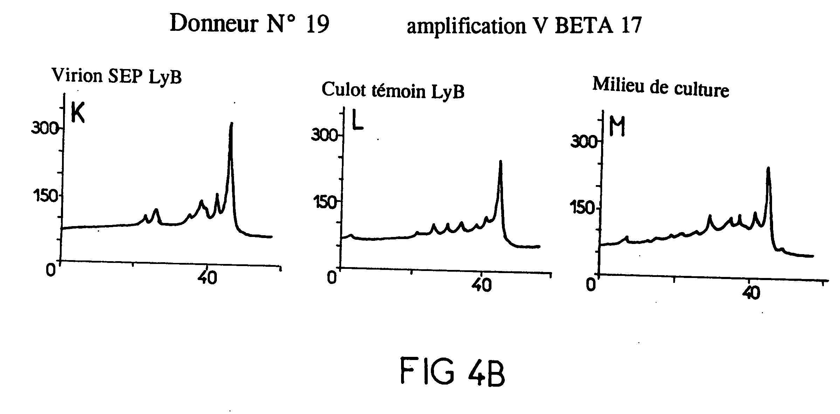

L'expérimentation est réalisée comme décrite dans l'exemple 5. Des cellules PBL de donneurs sains différents (donneur 7, 14, 19 et 18) sont récupérées et mises en contact avec des culots centrifugés de surnageants de cellules B issues de patients SEP ou d'individus sains. Des amplification du déterminant Vβ16 ou Vβ17 sont réalisées (voir figures 4A et 4B).

### Amplification du déterminant Vβ16

A : Donneur sain 7 et cellules B de témoin sains
B : Donneur sain 7 et cellules B de patients SEP
C : Donneur sain 14 et cellules B de patients SEP
D : Donneur sain 14 et milieu de culture
E : Donneur sain 19 et cellules B de patients SEP
F : Donneur sain 19 et cellules d'individu sain
G : Donneur sain 19 et milieu de culture
H : Donneur sain 18 et cellules B de patient SEP
I : Donneur sain 18 et cellules d'individu sain
J : Donneur sain 18 et cellules d'individu sain

### Amplification du déterminant Vb17

A : Donneur sain 17 et cellules B de patient SEP
B : Donneur sain 17 et cellules B d'individu sain
C : Donneur sain 17 et milieu de culture

Les graphes nous montrent que nous avons un profil différent de la même famille de Vβ16, voire Vβ17 amplifiée après incubation avec les cellules B de patients SEP ou avec les cellules B d'individu sain, pour un même donneur sain de PBL. Ces profils sont polyclonaux. Cela traduit bien l'activation polyclonale des cellules du donneur sain après une incubation avec des culots concentrés de surnageants de cellules B de patients SEP, différente de celle avec des culots concentrés de surnageants de cellules B d'individu sain. Cette activation polyclonale traduit la présence d'un ou plusieurs superantigène(s) ou superantigène-like dans les culots concentrés de SEP. Cette analyse par biologie moléculaire de l'expansion des cellules T de déterminant Vb16 suite à l'incubation en présence de culots concentrés de SEP confirme l'observation réalisée après analyse par immuno-détection en détectant directement les déterminants Vβs membranaires par des anticorps spécifiques, comme décrit dans l'exemple 9.

### Exemple 11 : Mise en évidence de l'expansion ou de la perte des lymphocytes porteurs de Vβ associée à la SEP.

Des suspensions de PBL 'Peripheral Blood Lymphocytes' prélevés à partir de donneurs sains (donneurs 5, 7, 14, 15, 19) sont mis en contact avec des culots concentrés de surnageants de lignées cellulaire B (ACX, FEX, BUX) établies à partir de prélèvements sanguins de patients SEP, comme décrit dans l'exemple 3. En parallèle ces suspensions sont mises en contact avec des culots concentrés de surnageants de lignées cellulaires B (T8, T9) établies à partir de prélèvements sanguins de patients sains. Après incubation, le répertoire Vβ des cellules CD3 est analysé par immuno-détection comme décrit dans l'exemple 4. Une prolifération majeure des cellules T présentant le déterminant Vβ 16 est observée, ainsi qu'une prolifération de cellules T avec Vβ 7, 14, 17 (tableau 4); ainsi comme décrit précédemment, une activité superantigénique est mise en évidence induite par les culots concentrés de surnageants de lignées B issues de patients SEP. Les pourcentages de prolifération des cellules T *in vitro* est estimé comme décrit précédemment. Parallèlement, il est clairement montré dans cet exemple, qu'il peut y avoir également, avec des donneurs sains différents, une diminution des sous populations de cellules T portant ces mêmes Vβs 16, 7, 14, 17 pour d'autres donneurs. Cette diminution peut refléter une mise en anergie ou en apoptose des cellules suite à l'effet superantigénique induit par les culots concentrés de surnageants cellulaires issus de patients SEP. II est à noter que 100% des suspensions PBL saines testées répondent soit par une expansion cellulaire soit par une diminution du nombre de cellules T présentant le déterminant Vb16, suite à une incubation avec les culots concentrés de surnageants de cellules B issues de patients SEP, et possédant une activité superantigénique.

Les résultats sont présentés dans le tableau ci-dessous

**Tableau 4**

| Donneur | HLA DR | Vβ2 | Vβ7 | Vβ14 | Vβ16 | Vβ17 |
|---|---|---|---|---|---|---|
| 5 | 13/14 | + 7,6 | **- 38** | - 28 | **- 49** | **- 31** |
| 7 | 3/12 | -6 | - 21 | **+ 37** | **+ 315** | -24 |
| 14 | 4/14 | | | | **+ 37** | + 5,13 |
| 15 | 3/13 | + 11 | - 24 | **+ 65** | **- 72** | - 2 |
| 19 | 4/15 | | | | **+ 56** | |

### Exemple 12 : Stimulation de la production de cytokines.

Les cultures de lymphocytes stimulés par LBSEP se distinguent par des productions significativement supérieures d'IL-6 et d'INF-γ comparées à celles stimulées par LBTE. Les titres de TNF-α sont très faibles en revanche et équivalents pour les deux types de cultures. Les résultats, exprimés en pg/ml de culture correspondant à 2 x 10 ⁶ cellules, sont présentés dans le tableau 5.

**Tableau 5**

| | **LBSEP** | **LBTE** |
|---|---|---|
| **IL-6 (n=6)** | 714 | 220 |
| **INF-γ (n = 3)** | 716 | 383 |
| **TNF-α (n = 6)** | 83 | 54 |

### Exemple 13 : Détection d'antigènes viraux dans les cultures de lymphocytes.

La présence d'antigènes rétroviraux spécifiques a été recherchée à l'aide des deux pools d'anticorps dirigés contre des protéines de MSRV-1. Les résultats d'immunofluorescence sur les cultures inoculées avec du SC ultracentrifugé de LES ou GRE, obtenus avec les deux pools (pool 2 = monoclonaux de souris, pool 1 =polyclonaux de lapin) sont présentés dans le tableau 6 pour les donneurs 10 et 11. Les chiffres représentent les pourcentages de cellules présentant des intensités de fluorescence comprises entre les canaux 100 et 1000.

**Tableau 6**

| | Donneur 10 | | Donneur 11 | |
|---|---|---|---|---|
| | LES | GRE | LES | GRE |
| Pool1 | 0.6 | 22.6 | 0.4 | 4.76 |
| Pool 2 | 7.11 | 30.77 | 0.12 | 4.69 |

Les résultats sont présentés comme des pourcentages de population exprimant une intensité de fluorescence comprise entre les canaux 100 et 1000.

L'expansion, parallèle à ces résultats concernant les antigènes MSRV-1, d'un nombre restreint de familles Vβ chez une majorité de donneurs en l'absence de restriction HLA indique que la réponse s'apparente à une réponse de type superantigène. La production de cytokines de type inflammatoire (IL-6, IFN-γ) conforte l'existence d'un environnement favorable au recrutement lymphocytaire.

L'absence de production de TNF-α est un argument en faveur de l'absence de contamination par des superantigènes bactériens ou du LPS.

### Exemple 14 : Production d'anticorps monoclonaux.

La production d'anticorps monoclonaux par ascite impose une compatibilité du système H-2 entre l'hybridome et la souris productrice. Des souris femelles Balb/c, âgées de 6 semaines, subissent une injection de 0.5ml de Pristane (2-6-10-14 acide tétraméthylpentadécane) dans leur cavité péritonéale, pour la production d'ascite (Porter et al., 1972). Une semaine à 10 jours plus tard, 5.10⁶ à 10.10⁶ hybridomes dilués dans environ 0.5ml de tampon stérile NaCl 0.145M, Na2HP04 10 mM, KCL 2.7 mM, KH2PO4 1.5 mM à pH 7.4. sont injectés par voie intrapéritonéale. L'ascite apparaît une à deux semaines plus tard. Les liquides d'ascites présents dans la cavité péritonéale sont alors recueillis avec une seringue après incision du péritoine. Le liquide recueilli est centrifugé à 3000g pendant 15 minutes à température ambiante, filtré sur gaze pour éliminer le gras, puis tamponné en ajoutant 1/20^{ème} de son volume de tris-HCL 1M à pH 8.0. Cette méthode permet d'obtenir des quantités d'anticorps 10 fois supérieures à celles obtenues par culture d'hybridomes.

Les immunoglobulines présentes dans le liquide d'ascite sont relarguées par les sels (sulfate d'ammonium ou sulfate de sodium). Le liquide d'ascite est précipité par le sulfate d'ammonium 40%. Après 20 minutes au froid la solution est centrifugée 15 minutes 8000g à 4°C. Le précipité est lavé et resuspendu à froid dans une solution de sulfate d'ammonium 40% puis de nouveau centrifugé. Le nouveau précipité enrichi en IgG est remis en solution dans du tampon PBS et dialysé la nuit contre le tampon Tris-HCl 25 mM, NaCl 150 mM pH 7.4. Parallèlement une colonne d'agarose-Protéine A (ou protéine G) (commercialisée sour forme lyophilisée, Pierce) est lavée extensivement avec le tampon Tris-HCl 25 mM, NaCl 150mM pH7.4. La solution enrichie en IgG est déposé sur la colonne puis la colonne est lavée. Les IgG retenues par la colonne sont éluées à pH acide (glycine 200 mM pH 2.8). Les fractions éluées sont neutralisées avec un volume de Tris-Base 1M pH 10.5. Le contenu en immunoglobulines de chaque fraction recueillie est quantifiée par lecture d'absorbance à 280 nm (e 1%,1cm = 14.0 Prahl et Porter 1968). Les fractions riches sont poolées. Le degré de purification des IgGs poolées est analysé par électrophorèse en gel d'acrylamide en présence de SDS. Les IgGs purifiées sont dialysées une nuit contre le tampon Tris-HCI 25 mM, NaCI 150mM pH7.4, filtrées stérilement, aliquotées et conservées à -20°C. Leur concentration finale est déterminée par lecture de l'absorbance à 280 nm ou par dosage micro-BCA.

Mais, il est à la portée de l'homme du métier de définir d'autres protocoles pour la production d'anticorps monoclonaux, par exemple à partir des techniques décrites par Köhler et Milstein et par Galfre G. *et al.* précédemment cités ou des techniques dérivées de celles ci.

### Exemple 15 : Mesure de l'activité des cellules T par prolifération cellulaire (Sredni et al., 1981).

Les cellules T sont lavées deux fois en milieu de culture pour éliminer toute trace d'IL2 présente dans le milieu initial de culture. Des lymphocytes B (EBV-LCL) ou des monocytes/macrophages pris comme cellules présentatrices de l'antigène, sont irradiées à 10000 rads, lavées deux fois avec du milieu de culture (RPMI). 2.10⁴ cellules T (2.10⁵ cellules /ml) et 2.10⁴ cellules B autologues irradiées (2.10⁵ cellules /ml) sont incubées ensemble en présence d'une gamme de concentration croissante de l'antigène sous un volume final de 200 µl dans des micropuits. Après 48 heures de culture à 37°C, 1 µCi de 3H- thymidine dans 50 µl de milieu RPMI est ajouté dans chaque puits. Les cellules T, seules à se diviser, incorporent la thymidine tritiée dans l'ADN. Après 18 heures de culture, les cellules de chaque micropuits sont récoltées sur des pastilles de laine de verre par aspiration. Après lyse osmotique des cellules, la radioactivité incorporée dans l'ADN est absorbée sur les pastilles (cell Harvester 530, Inotech). Chaque pastille séchée est placée dans un tube plastique qui contient 2 ml de scintillant ; la radioactivité b adsorbée sur chacune des pastilles est quantifiée dans un compteur beta à scientillation liquide (LKB Rackbeta 1217). Les résultats sont exprimés comme moyenne arithmétique de cpm /culture ('coups par minute').

### Exemple 16 : Protocole de détection de l'association entre peptides et molécules d'histocompatibilité (approche APC transformées par un peptide se fixant aux molécules du CMHI ou CMHII)

### 1) Matériel (exemple pour la molécule du CMHI):

Les sources de molécules d'histocompatibilité sont actuellement de deux types principaux : les cellules mutantes et les molécules d'histocompatibilité purifiées.

La cellule mutante utilisée est la cellule humaine T2 qui et un variant de la lignée T1 produite par fusion du lymphome T CEM et du lymphome B 721.174 (Salter and Cresswell Embo J 1986, 5: 943-949). Cette cellule qui est dépourvue de transporteurs de peptides contient des chaines lourdes de molécules de classe I libres de peptides qui vont pouvoir accepter de peptides exogènes.

Des molécules d'histocompatibilité de classe I purifiées par chromatographie d'affinité à partir de lignées de cellules B humaines transformées par l'EBV peuvent être également utilisées. Dans ce cas les peptides endogènes doivent être éliminés par un traitement avec de l'urée1.5 M et de la soude 12.5 mM (pH 11.7) pendant 1 heure à 4°C,suivi de leur élimination par une colonne de désalage (PDLO, Pharmacia). Les molécules d'histocompatibilité sont immédiatement remises en présence des peptides à tester dans un tampon PBS avec 0.05% Tween 20, 2 mM EDTA, 0.1% NP40 et6mM CHAPS, en présence de 2µg/ml B2m pour faciliter la réassociation (Gnjatic et al., Eur J lmmunol 1995 25 : 1638-1642). Les peptides testés ont en général 8 à 10 résidus, parfois 11 ou 12. Ils ont été synthétisés par Néosystems (Strasbourg), ou par Chiron mimotopes (Victoria, Australie). Ils sont utilisés à des concentrations variant de 100 µM à0.1 nM.

### 2) Protocole de l'assemblage (Connan et al., Eur J lmmunol 1994, 24 : 777 ; Couillin et al. Eur J Immunol 1995, 25 : 728-732).

Des aliquotes de 8.10⁵ cellules dans un volume de 64 µl, répartis dans des tubes microfuge Eppendorf, sont mis en présence d'un tampon de lyse contenant 10 mM PBS, pH 7.5 1% NP40, des inhibiteurs de protéases (1 mM PMSF, 100 µM iodoacétamide, 2 µg /ml aprotinine, 10 µM leupeptine, 10 µM pepstatine et 10 µg/ml inhibiteur de trypsine). La lyse se fait en présence des peptides à tester pendant 30 minutes ou 1 heure à 37°C. Après élimination du matériel non solubilisé par une centrifugation à 15 000 tours /minute à 4°C, le surnageant et additionné de 140 µl de PBS contenant 0.05% de Tween 20, 3 mM d'azide de sodium, 1 mM PMSF et 10 mg /ml d'albumine bovine. Chaque échantillon est incubé pendant 20 heures à 4°C dans 2 puits d'une plaque à microtitration de type Nunc,Maxisorb, préalablement recouverts d'un anticorps monoclonal (10 µg /ml en PBS) qui reconnaît les molécules d'histocompatibilité ayant une(des) conformation(s) conforme(s) pour la présentation de peptides et semblable(s) à celle(s) présente(s) à la surface des cellules. La plaque recouverte d'anticorps est préalablement saturée par de l'albumine bovine à 10 mg /ml dans du PBS-Tween avant la mise de l'échantillon. Le second anticorps permet la détection de l'assemblage des molécules d'histocompatibilité ciblées. II est couplé soit à la biotine (NHS-LC biotin, Pierce) soit à la phosphatase alcaline (P-552, Sigma) et est incubé pendant une heure à 37°C. Dans le cas de l'emploi de la biotine, une incubation de 45 minutes à 20-25°C avec de la streptavidine couplée à la phosphatase alcaline (E-2636, Sigma) est réalisée. L'activité de la phosphatase alcaline est mesurée en utilisant comme substrat le 4-méthyl-umbelliféryl-phosphate (M-8883, Sigma) à 100 µM dans de la diéthanolamine 50 mM, pH 9.5 avec du MgCl2 1 mM. La lecture est faite à 340/460 nm à l'aide d'un cytofluorimètre.

### 3) Stabilité des complexes HLA/peptides :

La stabilité des complexes précités a été étudiée car elle conditionne la bonne présentation de l'antigène et l'induction de la réponse T. A cet effet a été utilisé soit du HLA purifié, soit le lysat de la cellule. Avec le HLA purifié, les peptides endogènes ont été éliminés comme décrit en 2) puis mis en présence du peptide à tester en tube Eppendorf à 37°C, pendant des temps variables de quelques minutes à plusieurs jours. La phase suivante d'incubation sur plaque de 96 puits (comme décrit en 2) avec l'anticorps anti-HLA se fait pendant une heure à 37°C. La révélation est effectuée de manière classique. Avec le lysat de la cellule, toutes les incubations sont également faites à 37°C, après ajout de tous les inhibiteurs de protéases.

### Exemple 17: La protéine d'enveloppe MSRV reproduit la stimulation dominante de la population de lymphocytes T porteurs des chaînes Vβs16, observée préalablement avec l'infection par la fraction contenant les particules virales.

L'analyse de l'expansion ou de la déplétion (perte) des sous populations de lymphocytes T a été effectuée par FACS sur des cellules mononucléées sanguines de donneurs sains globalement selon les protocoles décrits dans les exemples 2, 3, 4, 9 et 11.

Le tableau 7 montre les résultats obtenus avec un nouveau lot de virion SEP produit dans des cultures de plexus choroïdes de SEP, testé sur une nouvelle série de différents donneurs sains. II montre la reproductibilité de la stimulation majoritaire Vβ16 chez la plupart des donneurs sains (Don 12, 15, 16, 19 et 20). La faible réactivité de deux donneurs (Don 14 et 18) correspond à un faible pourcentage de donneurs « non répondeurs » régulièrement observé dans les séries successives. La baisse parallèle de tous les Vβs chez le donneur 13 évoque un problème de viabilité de ses cellules stockées dans l'azote et décongelées pour la culture. En conséquence, ce dernier donneur n'a plus été utilisé par la suite.

**Tableau 7**

| | Vβ2 | Vβ14 | Vβ16 | Vβ17 |
|---|---|---|---|---|
| Don 12 | **-6** | **-8** | **50** | **-8** |
| Don 13 | **-39** | **-27** | **-20** | **-52** |
| Don 14 | **-19** | **-23** | **12** | **-4** |
| Don 15 | **-30** | **0** | **75** | **-16** |
| Don 16 | **-50** | **-66** | **88** | **-51** |
| Don 18 | **-25** | **-31** | **5** | **-17** |
| Don 19 | **-9** | **-14** | **43** | **-50** |
| Don 20 | **0** | **17** | **67** | **0** |

La modification faite aux protocoles décrits dans les exemples sus nommés a consisté à remplacer l'ajout de la fraction contenant les virions produits par des cultures de cellules SEP par des protéines recombinantes produites à partir des séquences rétrovirales MSRV associées à ces mêmes sources de virions ou par le tampon de solubilisation seul.

Cette analyse a déjà permis de mettre en évidence des propriétés immunologiques de la protéine d'enveloppe du rétrovirus MSRV (env MSRV) reproduisant la stimulation majeure de la sous population lymphocytaire T Vβ16 positive et une stimulation mineure de la sous population lymphocytaire T Vβ17 positive.

Pour ce faire, un plasmide d'expression bactérienne a été obtenu avec l'insert ADN codant pour le cadre de lecture d'une protéine env MSRV, selon les techniques de biologie moléculaires bien connues de l'homme de l'art.

La séquence de l'insert est référencée en SEQ ID NO 1.

La séquence en acide aminés de la protéine recombinante exprimée à partir de ce plasmide est référencée en SEQ ID NO 2:

Le plasmide d'expression a permis de produire la protéine env MSRV, en fusion avec une terminaison "polyhistidine" (HIS-Tag) qui a permis de la purifier sur colonne d'affinité à partir de l'extrait de bactéries *E. coli* recombinantes dans lesquelles l'expression la protéine a été faite.

La protéine env recombinante a été purifiée et analysée par électrophorèse SDS PAGE, en présence de marqueurs de poids moléculaire. Les fractions d'élution spécifique de la protéine recombinante produite dans la fraction insoluble retenue par le HIS-Tag sur colonne d'affinité ont été récupérées. La correspondance avec la protéine fusionnée au HIS-Tag a été confirmée par Western blot, avec un anticorps spécifique du HIS-Tag, marquant la protéine de 60 KD retrouvée seule dans la fraction éluée, après visualisation sur un gel d'électrophorèse SDS PAGE. La fraction la plus riche (environ 0,5 mg/ml), a été utilisée pour être ajoutée aux cellules de donneurs sains dans le test immunologique faisant l'objet de cet exemple.

Les cellules mononucléées sanguines de trois donneurs (12, 15 et 16) ont été incubées parallèlement 3 jours en présence de 10 ng/ml et de 100ng/ml de la protéine env MSRV recombinante, ainsi que d'une dilution équivalente du tampon de solubilisation seul.

Après ces trois jours d'incubation, l'analyse du répertoire des Vβs a montré, selon les critères d'analyses déjà décrits dans les exemples précédents, que les donneurs 12 et 16 on répondu significativement, par une expansion de Vβ16 et Vβ17, à la présence de la protéine env MSRV. Le donneur 16 a de plus montré une expansion Vβ2. Une réponse Vβ orientée n'a pas été retrouvée chez le donneur 15.

Les résultats sont résumés dans le tableau 8.

**Tableau 8**

| Don 12 | Vβ2 | Vβ7 | Vβ14 | Vβ16 | Vβ17 |
|---|---|---|---|---|---|
| 10 ng/ml | **13** | **-7** | **7** | 159 | 151 |
| 100 ng/ml | **9** | **-9** | **8** | 212 | 156 |
| | | | | | |

| Don 15 | Vβ2 | Vβ7 | Vβ14 | Vβ16 | Vβ17 |
|---|---|---|---|---|---|
| 10 ng/ml | **10** | **4** | **32** | **-30** | **24** |
| 100 ng/ml | **7** | **2** | **1** | **-4** | **6** |

| Don 16 | Vβ2 | Vβ7 | Vβ14 | Vβ16 | Vβ17 |
|---|---|---|---|---|---|
| 10 ng/ml | **80** | **-60** | **ND** | 52 | 156 |

Ceci indique que la protéine env MSRV peut être responsable de la stimulation dominante des sous populations Vβs 16 observée avec la fraction virale totale contenant les particules MSRV-1 utilisée dans les exemples précédents, mais aussi des co-stimulations ou stimulations alternatives de quelques autres sous populations (ex. Vβs 17 et 2) observées avec une fréquence moindre sur l'ensemble des donneurs sains testés à ce jour.

Ces résultats indiquent aussi que les séquences MSRV-1 codant pour la protéine env sont susceptibles d'être prises parmi les séquences nucléotidiques décrites précédemment et que les variants protéiques env MSRV-1 sont susceptibles d'être pris parmi les séquences décrites auparavant, étant donné que la variabilité des séquences rétrovirales n'exclue pas la stabilité d'une propriété biologique particulière sur une majorité de variants.

### Exemple 18: Abolition de la stimulation de type superantigénique des sous populations de lymphocytes T, par addition de médicaments anti-viraux.

L'analyse de l'expansion ou de la déplétion des sous populations de lymphocytes T a été effectuée par FACS sur des cellules mononucléées sanguines de donneurs sains selon la description faite dans les exemples 2, 3, 4, 9 et 11.

La modification faite au protocole décrit dans les exemples sus nommés a consisté à inoculer en parallèle, dans des puits contenant les cellules mononucléées sanguines d'un même donneur sain: 1) une fraction contenant les virions produits par des cultures de cellules SEP, 2) la même fraction virale en présence d'AZT 12,5 µM (Sigma), 3) la même fraction virale en présence de DDI (Sigma) 20 µM. Ces cultures ont été effectuées sur les cellules d'un donneur dont les cellules mononucléées sanguines avaient été congelées par aliquots et dont un ou plusieurs aliquots avaient montré un effet de type superantigénique en présence de virions provenant de cultures SEP (cf. tableau 7).

Par ailleurs des tests de toxicité avec l'AZT (Azido Deoxythymidine) seul et le DDI (DiDeoxylnosine) seul, ont été effectués sur les mêmes cellules et n'ont montré aucune mortalité cellulaire significative.

L'étude avec les médicaments anti-rétroviraux a déjà permis de mettre en évidence l'action inhibitrice de ce type de médicament sur l'induction d'une stimulation de type superantigénique par une fraction concentrée de surnageants de cultures de cellules provenant de patients atteints de SEP et contenant des particules rétrovirales associées à l'ARN de MSRV-1. Les résultats correspondant sont présentés dans le tableau 10 ci-dessous. La ligne intitulée « medium » correspond au milieu contenant le virion SEP sans anti-rétroviraux et les lignes intitulées DDI et AZT font référence aux tets effectués en présence de ces inhibiteurs. L 'analyse au FACS réalisée selon les exemples précédents, des sous populations T dans les différents puits de culture montre:
- Une expansion significative Vβ16 des lymphocytes incubés en présence du virion "SEP" seul qui n'était pas retrouvée en présence de la fraction "témoin virion" provenant de cultures de témoins "non-SEP".
- Une absence ou une inhibition d'expansion Vβ16 des lymphocytes incubés en présence du virion "SEP" et de l'AZT.
- Une absence d'expansion Vβ16 des lymphocytes incubés en présence du virion "SEP" et du DDI.

**Tableau 10**

| Don 12 | Vβ2 | Vβ14 | Vβ16 | Vβ17 |
|---|---|---|---|---|
| AZT | **13** | **-6** | **7** | **0** |
| DDI | **-4** | **-21** | **-15** | **-11** |
| Medium | **-6** | **-8** | 50 | **-8** |
| | | | | |

| Don 20 | Vβ2 | Vβ14 | Vβ16 | Vβ17 |
|---|---|---|---|---|
| AZT | **-3** | **-15** | **-3** | **16** |
| DDI | **-5** | **-35** | **-33** | **-19** |
| Medium | **0** | **17** | 67 | **0** |

Au vu de ces résultats, il apparaît maintenant que tout médicament, molécule ou procédé thérapeutique ayant un effet inhibiteur, bloquant ou modulateur sur l'expression rétrovirale, notamment infectieuse et/ou endogène, peut être utilisé pour inhiber l'effet immunopathologique induit par le ou les agents pathogènes associés à la production de ces particules rétrovirales. Les caractéristiques générales de cet effet immunopathologique sont décrites dans la présente invention.

**Au vu de ces résultats et de ceux des exemples 8 et 17, il apparaît maintenant que ces agents ou procédés thérapeutiques peuvent être choisis parmi les composés actifs sur l'expression du rétrovirus MSRV-1.**

### Références bibliographiques sur les Vβs.

Brenden N, et al., Differential MHC expression requirements for positive selection of separate TCR Vb families,. Immunogenetics. 1999 Jan;49(1):1-6.
Hodges E, et al., T cell receptor (TCR) Vbeta gene usage in bronchoalveolar lavage and peripheral blood T cells from asthmatic and normal subjects,. Clin Exp Immunol. 1998 Jun;112(3):363-74.
Allen TM, et al., The T-cell receptor beta chain-encoding gene repertoire of a New World primate species, the cotton-top tamarin, Immunogenetics. 1996;45(2):151-60.
Yassai M, et al., Bacterial toxin superantigens stimulate all members of susceptible VB gene families, Ann N Y Acad Sci. 1995 Jul 7;756:110-2.
Donahue JP, et al., Genetic analysis of low V beta 3 expression in humans,. J Exp Med. 1994 May 1;179(5):1701-6.
Isono T, et al., Sequence and diversity of variable gene segments coding for rabbit T-cell receptor beta chains,. lmmunogenetics. 1994;39(4):243-8.
Levinson G, et al., Sequence and diversity of rhesus monkey T-cell receptor beta chain genes, Immunogenetics. 1992;35(2):75-88.
Buitkamp J, et al. , Vb6 T-cell receptor elements in artiodactyls: conservation and germline polymorphisms, Mamm Genome. 1993 Sep;4(9):504-10.
Johnston SL, et al., Diversity of alpha and beta subunits of T-cell receptors specific for the H4 minor histocompatibility antigen, Immunogenetics. 1997;46(1):17-28.
Huck S, et al., Variable region genes in the human T-cell rearranging gamma (TRG) locus: V-J junction and homology with the mouse genes, EMBO J. 1988 Mar;7(3):719-26.
Sherman LA, et al., Comparison of the H-2Kb-specific cytolytic T lymphocyte receptor repertoire in Igh recombinant strains, J Immunol. 1985 Jun;134(6):3569-73.

## Revendications

1. Procédé de détection de la sclérose en plaques ou d'une prédisposition à développer la sclérose en plaques, dans un échantillon biologique, **caractérisé en ce qu'**on détecte une activité superantigénique induite par le produit d'expression du gène *env* de MSRV-1 ou d'un fragment du gène *env* de MSRV-1, ledit gène *env* de MSRV-1 comprenant ou consistant en la séquence référencée en SEQ ID NO :1, ou **en ce qu'**on détecte une activité superantigénique induite par le produit d'expression du gène *env* d'un variant de MSRV-1 ou d'un fragment du gène *env* dudit variant, ledit gène *env* dudit variant présentant une homologie d'au moins 90% avec le gène *env* de MSRV-1, en mettant en évidence une expansion ou une perte de lymphocytes choisis parmi les lymphocytes porteurs d'un déterminant Vβ16 et/ou Vβ17 et les lymphocytes porteurs d'un déterminant Vβ7, ladite expansion ou ladite perte étant d'au moins 31 %.

2. Procédé selon la revendication 1, **caractérisé en ce que** l'on met en évidence une expansion de lymphocytes porteurs d'un déterminant Vβ16.

3. Procédé selon la revendication 1, **caractérisé en ce que** l'on met en évidence une perte de lymphocytes porteurs d'un déterminant Vβ16.

4. Procédé selon la revendication 2, **caractérisé en ce que** l'on met en évidence une expansion de lymphocytes porteurs d'un déterminant Vβ16 et une co-expansion de lymphocytes porteurs de Vβs choisis parmi au moins l'un quelconque des Vβs 2, 3, 7, 8, 12, 14, 17 et 22 et préférentiellement des Vβs 3 et 12.

5. Procédé selon la revendication 3, **caractérisé en ce que** l'on met en évidence une perte de lymphocytes porteurs d'un déterminant Vβ16 et d'une co-diminution de lymphocytes porteurs de Vβs choisis parmi au moins l'un quelconque des Vβs 2, 3, 7, 8, 12, 14, 17 et 22, de préférence au moins l'un quelconque des Vβs 7, 14 et 17 et avantageusement des Vβs 7 et 17.

6. Procédé selon l'une quelconque des revendications 1 à 5, **caractérisé en ce que** l'activité superantigénique est induite par le produit d'expression du gène *env* de MSRV-1 référencée en SEQ ID NO :1 ou d'un fragment dudit gène *env* de MSRV-1.

7. Procédé selon l'une quelconque des revendications 1 à 5, **caractérisé en ce que** l'activité superantigénique est induite par la protéine d'enveloppe de MSRV-1 référencée en SEQ ID NO : 2 ou par un fragment de ladite protéine d'enveloppe.

8. Procédé selon l'une quelconque des revendications 1 à 7, **caractérisé en ce que** l'échantillon biologique est choisi parmi les cellules mononucléées sanguines.

9. Procédé selon l'une quelconque des revendications 1 à 7, **caractérisé en ce que** le produit d'expression du gène *env* ou du fragment du gène *env* de MSRV-1 ou du variant de MSRV-1 est obtenu à partir de cellules infectées par MSRV-1 ou par le variant de MSRV-1 choisies parmi les cellules de plexus choroïdes, les cellules leptoméningées, les cellules de lignées cellulaires établies, telles que les cellules des lignées cellulaires PLI-2 déposée à l'ECACC le 22 juillet 1992, sous le numéro 92072201 et LM7PC déposée à l'ECACC le 8 janvier 1993, sous le numéro 93010817, conformément aux dispositions du Traité de Budapest ou à partir d'un vecteur d'expression comprenant le gène *env* ou un fragment du gène *env* de MSRV-1 ou du variant de MSRV-1 ou **en ce que** le produit d'expression du gène *env* ou du fragment du gène *env* de MSRV-1 ou du variant de MSRV-1 est une molécule polypeptidique choisie parmi une protéine ou un fragment de protéine consistant ou comprenant SEQ ID NO : 2.

10. Procédé selon l'une quelconque des revendications 1 à 9, **caractérisé en ce que** :
(i) on prélève un surnageant de culture de cellules mononucléées sanguines ou de cellules de plexus choroïdes ou de cellules leptoméningées, lesdites cellules provenant de patients atteints de sclérose en plaques ou suspectés d'avoir une prédisposition à développer la sclérose en plaques, ou d'une lignée cellulaire établie, telles que les cellules des lignées cellulaires PLI-2 déposée à l'ECACC le 22 juillet 1992, sous le numéro 92072201 et LM7PC déposée à l'ECACC le 8 janvier 1993, sous le numéro 93010817, conformément aux dispositions du Traité de Budapest, et
(ii) on met en contact ledit surnageant de culture ou une partie du surnageant de culture avec une série de cultures, de préférence au moins trois, de cellules mononucléées sanguines provenant de donneurs sains, et
(iii) on détecte ladite expansion et éventuellement une co-expansion ou ladite perte et éventuellement co-diminution des cellules mononucléées sanguines de l'étape (ii).

11. Procédé selon la revendication 10, **caractérisé en ce que** les cellules mononucléées sanguines provenant de patients atteints de sclérose en plaques (SEP) sont choisies parmi les monocytes et les lymphocytes B et les cellules mononucléées sanguines provenant de donneurs sains sont choisies parmi les lymphocytes T.

12. Procédé selon l'une quelconque des revendications 1 à 9, **caractérisé en ce que** :
(i) on prélève des cellules mononucléées sanguines, lesdites cellules provenant de patients atteints de sclérose en plaques ou de patients suspectés d'avoir une prédisposition à développer la sclérose en plaques, et d'individus sains,
(ii) on met en contact lesdites cellules mono nucléées sanguines provenant des patients ou d'individus sains avec des surnageants de culture ou une fraction de surnageant de culture de cellules choisies parmi les cellules mononucléées sanguines, les cellules de plexus choroïdes et les cellules leptoméningées, les cellules issues de lignées cellulaires établies, telles que les cellules des lignées cellulaires PLI-2 déposée à l'ECACC le 22 juillet 1992, sous le numéro 92072201 et LM7PC déposée à l'ECACC le 8 janvier 1993, sous le numéro 93010817, conformément aux dispositions du Traité de Budapest, et
(iii) on détecte ladite expansion et éventuellement co-expansion ou ladite perte et éventuellement co-diminution à partir des cellules mononucléées sanguines de l'étape (i).

13. Procédé selon les revendications 10, 11 et 12, **caractérisé en ce que** ladite expansion et éventuellement co-expansion est mise en évidence par utilisation de ligands, chaque ligand étant spécifique d'un déterminant choisi parmi les Vβs 16, 2, 3, 7, 8, 12, 14, 17 et 22, de préférence des Vβs 16, 3 et 12, et **en ce que** ladite perte et éventuellement co-diminution est mise en évidence par utilisation de ligands, chaque ligand étant spécifique d'un déterminant choisi parmi les Vβs 16, 2, 3, 7, 8, 12, 14, 17 et 22, de préférence les Vβs 16, 7, 14 et 17.

14. Procédé selon la revendication 13, **caractérisé en ce que** le ligand est un anticorps, de préférence un anticorps monoclonal ou un fragment d'anticorps.

15. Procédé selon la revendication 10, 11 et 12, **caractérisé en ce que** pour mettre en évidence ladite expansion et éventuellement co-expansion ou ladite perte et éventuellement co-diminution on réalise
(i) une extraction des ARN totaux des cellules mononucléées sanguines mises en présence de surnageant ou d'une fraction de surnageant de culture SEP et en présence de surnageant ou d'une fraction de surnageant de culture témoin,
(ii) une transcription inverse desdits ARN,
(iii) une amplification spécifique de chaque famille de Vβs à l'aide d'un couple d'amorces déterminé,
(iv) un marquage par tout marqueur approprié des produits d'amplification obtenus,
(v) une électrophorèse desdits produits d'amplification et analyse des profils d'electrophorèse obtenus, à l'aide d'un détecteur approprié.

16. Procédé selon la revendication 15, **caractérisé en ce que** les cellules mononucléées sanguines provenant de patients atteints de SEP sont choisies parmi les lymphocytes.

17. Procédé selon l'une quelconque des revendications 1 à 16, **caractérisé en ce que** l'on met en outre en évidence au moins l'un des paramètres suivants :
une stimulation de la production de cytokines, telles que l'interleukine-6 (IL-6) et interféron-γ (INF-γ)
une induction d'apoptose cellulaire.

18. Procédé selon la revendication 17, **caractérisé en ce que** l'on détecte les deux paramètres en association.

19. Procédé selon l'une quelconque des revendications 1 à 9, **caractérisé en ce que**
(i) on produit ou synthétise une protéine recombinante telle qu'identifiée en SEQ ID NO :2, ou un fragment de ladite protéine,
(ii) on met en contact ladite protéine ou fragment avec une série de cultures, de préférence au moins trois, de cellules mononucléées sanguines provenant de donneurs sains, et
(iii) on détecte ladite expansion et éventuellement une co-expansion ou ladite perte et éventuellement co-diminution des cellules mononucléées sanguines de l'étape (ii).

20. Procédé selon l'une quelconque des revendications 1 à 9, **caractérisé en ce que** :
(i) on prélève des cellules mononucléées sanguines, lesdites cellules provenant de patients atteints de la SEP ou de patients suspectés d'avoir un risque de développer la SEP, et d'individus sains,
(ii) on met en contact lesdites cellules mononucléées sanguines provenant des patients ou d'individus sains avec un polypeptide ou une protéine recombinante, telle qu'identifiée en SEQ ID NO :2, ou un fragment dudit polypeptide ou de ladite protéine, et
(iii) on détecte ladite expansion et éventuellement co-expansion ou ladite perte et éventuellement co-diminution à partir des cellules mononucléées sanguines de l'étape (i).

21. Procédé selon la revendication 20, **caractérisé en ce que** ledit polypeptide ou ladite protéine est codé(e) par un gène *env,* tel qu'identifié en SEQ ID NO : 1.

22. Procédé d'évaluation de l'efficacité d'un agent ou d'une composition pour inhiber dans un échantillon biologique une activité superantigénique induite parle rétrovirus MSRV-1 ou un variant de MSRV-1, par l'expression de son gène *env* ou d'un fragment dudit gène *env,* ledit gène env de MSRV-1 comprenant la séquence référencée en SEQ ID NO :1 et le gène *env* dudit variant présentant une homologie d'au moins 90% avec le gène env de MSRV-1, **caractérisé en ce que**
i) on met en contact un surnageant de culture de cellules mononucléées sanguines ou de cellules de plexus choroïdes ou de cellules leptoméningées, lesdites cellules provenant de patients atteints d'une maladie auto-immune, ou de cellules d'une lignée cellulaire établie, telles que les cellules des lignées PLI-2 et LM7PC, avec des cellules mononucléées sanguines provenant de donneurs sains en présence dudit agent ou de ladite composition à des doses prédéterminées, et
(ii) on détecte l'inhibition de ladite expansion et éventuellement co-expansion ou l'inhibition de ladite perte et éventuellement co-diminution des lymphocytes porteurs d'au moins un déterminant choisi parmi les Vβs 16, 7 et 17, par utilisation d'un ligand comme décrit dans la revendication 14 ou d'une amplification associée à une électrophorèse comme décrit dans la revendication 15.

23. Procédé selon la revendication 22, **caractérisé en ce que** les cellules proviennent d'un patient atteint de sclérose en plaques.

24. Procédé selon la revendication 22 ou 23, **caractérisé en ce que** les cellules mononucléées sanguines provenant de patients atteints de SEP sont choisies parmi les lymphocytes B et les monocytes.

25. Procédé d'évaluation de l'efficacité prophylactique et/ou thérapeutique d'un agent ou d'une composition vis-à-vis de la sclérose en plaques, et/ou d'une prédisposition à la sclérose en plaques, **caractérisé en ce que** l'on met en évidence une inhibition d'une activité superantigénique dans un échantillon biologique comme décrit dans les revendications 22 à 24.

26. Procédé selon la revendication 25, **caractérisé en ce que** l'on met en évidence l'inhibition de la perte majoritaire de lymphocytes porteurs d'un déterminant Vβ16 et de la co-diminution de lymphocytes porteurs de Vβs7 et 17.

27. Procédé selon la revendication 25, **caractérisé en ce que** l'on met en évidence l'inhibition de l'expansion majoritaire de lymphocytes porteurs d'un déterminant Vβ16 et de la co-expansion de lymphocytes porteurs de Vβs 3 et 12.

28. Procédé selon l'une quelconque des revendications 25 à 27, **caractérisé en ce que** les cellules proviennent d'un patient atteint de sclérose en plaques.

29. Procédé selon l'une quelconque des revendications 25 à 28, **caractérisé en ce que** les cellules mononucléées sanguines provenant de patients atteints de SEP sont choisies parmi les lymphocytes B et les monocytes.

30. Procédé pour identifier des substances capables de bloquer la transcription et/ou la traduction du rétrovirus humain MSRV-1 ou d'un variant de MSRV-1, induisant une activité superantigénique dans la sclérose en plaques, par l'expression de son gène *env* ou d'un fragment dudit gène *env,* ledit gène env de MSRV-1 comprenant la séquence référencée en SEQ ID NO :1 et le gène *env* dudit variant présentant une homologie d'au moins 90% avec le gène env de MSRV-1, procédé selon lequel,
on met en contact la substance avec des cellules exprimant un polypeptide rétroviral identifié en SEQ ID NO :2, ou un fragment dudit polypeptide et ayant une activité superantigénique, et
on détecte une perte ou diminution de l'activité superantigénique comme décrit dans les revendications 1 à 5.

31. Kit pour le criblage de substances capables de bloquer l'activité superantigénique du rétrovirus MSRV-1 ou d'un variant de MSRV-1 induisant ladite activité par l'expression de son gène *env* ou d'un fragment dudit gène *env,* ledit gène env de MSRV-1 comprenant la séquence référencée en SEQ ID NO:1 et le gène *env* dudit variant présentant une homologie d'au moins 90% avec le gène env de MSRV-1, vis à vis de la sclérose en plaques, ou capable de bloquer la transcription et/ou la traduction dudit rétrovirus, ledit kit comprenant :
des cellules exprimant à leur surface des produits du MHC de classe II, transformées avec et exprimant fonctionnellement le superantigène rétroviral comprenant au moins le fragment référencé en SEQ ID NO :2,
des cellules porteuses de chaînes du récepteur d'un Vβ ou de Vβs stimulées par le superantigène rétroviral, lesdites cellules étant choisies parmi les lymphocytes porteurs d'un déterminant Vβ16 et/ou Vβ17 et les lymphocytes porteurs d'un déterminant Vβ7, et
des moyens pour détecter une perte ou diminution de l'activité superantigénique comme décrit dans les revendications 1 à 5, lesdits moyens étant choisis parmi un ligand comme décrit dans la revendication 14 et une amplification associée à une électrophorèse comme décrit dans la revendication 15.

## Claims

1. Method for detecting multiple sclerosis or a predisposition to develop multiple sclerosis, in a biological sample, **characterized in that** a super antigen activity induced by the product of expression of the MSRV-1 env gene or of a fragment of the MSRV-1 env gene is detected, the said MSRV-1 env gene comprising or consisting of the sequence designated by the reference SEQ ID NO:1, or **in that** a superantigen activity induced by the product of expression of the env gene of a variant of MSRV-1 or of a fragment of the env gene of the said variant is detected, the said env gene of the said variant exhibiting at least 90% homology with the MSRV-1 env gene, by identifying an expansion or a loss of lymphocytes chosen from the lymphocytes carrying a determinant Vβ16 and/or Vβ17 and the lymphocytes carrying a determinant Vβ7, the said expansion or the said loss being at least 31%.

2. Method according to Claim 1, **characterized in that** an expansion of lymphocytes carrying a determinant Vβ16 is identified.

3. Method according to Claim 1, **characterized in that** a loss of lymphocytes carrying a determinant Vβ16 is identified.

4. Method according to Claim 2, **characterized in that** an expansion of lymphocytes carrying a determinant Vβ16 and a co-expansion of lymphocytes carrying Vβs chosen from at least any one of the Vβs 2, 3, 7, 8, 12, 14, 17 and 22 and preferably the Vβs 3 and 12 is identified.

5. Method according to Claim 3, **characterized in that** a loss of lymphocytes carrying a determinant Vβ16 and a co-reduction of lymphocytes carrying Vβs chosen from at least any one of the Vβs 2, 3, 7, 8, 12, 14, 17 and 22, preferably at least any one of the Vβs 7, 14 and 17 and advantageously the Vβs 7 and 17 are identified.

6. Method according to any one of Claims 1 to 5, **characterized in that** the superantigen activity is induced by the product of expression of the MSRV-1 env gene designated by the reference SEQ ID NO:1 or of a fragment of the said MSRV-1 env gene.

7. Method according to any one of Claims 1 to 5, **characterized in that** the superantigen activity is induced by the MSRV-1 envelope protein designated by the reference SEQ ID NO:2 or by a fragment of the said envelope protein.

8. Method according to any one of Claims 1 to 7, **characterized in that** the biological sample is chosen from blood mononuclear cells.

9. Method according to any one of Claims 1 to 7, **characterized in that** the product of expression of the env gene or of the fragment of the env gene of MSRV-1 or of the variant of MSRV-1 is obtained from cells infected with MSRV-1 or with the variant of MSRV-1 chosen from plexus choroid cells, leptomeningeal cells, cells of established cell lines, such as the cells of the cell lines PLI-2 deposited at the ECACC on 22 July 1992, under the number 92072201 and LM7PC deposited at the ECACC on 8 January 1993, under the number 93010817, in accordance with the provisions of the Budapest Treaty or from an expression vector comprising the env gene or a fragment of the env gene of MSRV-1 or of the variant of MSRV-1 or **in that** the product of expression of the env gene or of the fragment of the env gene of MSRV-1 or of the variant of MSRV-1 is a polypeptide molecule chosen from a protein or a protein fragment consisting of or comprising SEQ ID NO:2.

10. Method according to any one of Claims 1 to 9, **characterized in that**:
(i) a culture supernatant of blood mononuclear cells or of plexus choroid cells or of leptomeningeal cells is collected, the said cells being obtained from patients suffering from multiple sclerosis or suspected of having a predisposition to develop multiple sclerosis, or from an established cell line, such as the cells of the cell lines PLI-2 deposited at the ECACC on 22 July 1992 under the number 92072201 and LM7PC deposited at the ECACC on 8 January 1993, under the number 93010817, in accordance with the provisions of the Budapest Treaty and,
(ii) the said culture supernatant or part of the culture supernatant is brought into contact with a series of cultures, preferably at least three, of blood mononuclear cells obtained from healthy donors, and
(iii) the said expansion and optionally one co-expansion or the said loss and optionally co-reduction of blood mononuclear cells of step (ii) are detected.

11. Method according to Claim 10, **characterized in that** the blood mononuclear cells obtained from patients suffering from multiple sclerosis (MS) are chosen from monocytes and B lymphocytes and blood mononuclear cells obtained from healthy donors are chosen from the T lymphocytes.

12. Method according to any one of Claims 1 to 9, **characterized in that**
(i) blood mononuclear cells are collected, the said cells being obtained from patients suffering from multiple sclerosis or from patients suspected of having a predisposition to develop multiple sclerosis, and from healthy individuals,
(ii) the said blood mononuclear cells obtained from patients or healthy individuals are brought into contact with culture supernatants or a fraction of culture supernatant of cells chosen from blood mononuclear cells, plexus choroid cells and leptomeningeal cells, cells derived from established cell lines, such as cells of the cell lines PLI-2 deposited at the ECACC on 22 July 1992, under the number 92072201 and LM7PC deposited at the ECACC on 8 January 1993, under the number 93010817, in accordance with the provisions of the Budapest Treaty, and
(iii) the said expansion and optionally co-expansion or the said loss and optionally co-reduction is detected from the blood mononuclear cells of step (i).

13. Method according to Claims 10, 11 and 12, **characterized in that** said expansion and optionally co-expansion is identified using ligands, each ligand being specific for a determinant chosen from the Vβs 16, 2, 3, 7, 8, 12, 14, 17 and 22, preferably the Vβs 16, 3 and 12, and **in that** the said loss and optionally co-reduction is identified using ligands, each ligand being specific for a determinant chosen from Vβs 16, 2, 3, 7, 8, 12, 14, 17 and 22, and preferably the Vβs 16, 7, 14 and 17.

14. Method according to Claim 13, **characterized in that** the ligand is an antibody, preferably a monoclonal antibody or an antibody fragment.

15. Method according to Claim 10, 11 and 12, **characterized in that**, to identify the said expansion and optionally co-expansion or the said loss and optionally co-reduction,
(i) the total RNA is extracted from blood mononuclear cells exposed to MS culture supernatant or supernatant fraction and exposed to control culture supernatant or supernatant fraction,
(ii) a reverse transcription of the said RNAs is carried out,
(iii) a specific amplification of each family of Vβs is carried out with the aid of a defined pair of primers,
(iv) labelling is carried out with any appropriate marker for the amplification products obtained,
(v) electrophoresis of the said amplification products is carried out and analysis of the electrophoretic profiles obtained is carried out with the aid of an appropriate detector.

16. Method according to Claim 15, **characterized in that** the blood mononuclear cells obtained from patients suffering from MS are chosen from lymphocytes.

17. Method according to any one of Claims 1 to 16, **characterized in that** at least one of the following parameters is identified:
a stimulation of the production of cytokines, such as interleukin-6 (IL-6) and γ-interferon (INF-γ) an induction of cell apoptosis.

18. Method according to Claim 17, **characterized in that** the two parameters in combination are detected.

19. Method according to any one of Claims 1 to 9, **characterized in that**
(i) a recombinant protein as identified in SEQ ID NO:2, or a fragment of the said protein, is produced or synthesized,
(ii) the said protein or fragment is brought into contact with a series of cultures, preferably at least three, of blood mononuclear cells obtained from healthy donors, and
(iii) the said expansion and optionally a co-expansion or the said loss and optionally co-reduction of blood mononuclear cells of step (ii) are detected.

20. Method according to any one of Claims 1 to 9, **characterized in that**:
(i) blood mononuclear cells are collected, the said cells being obtained from patients suffering from MS or from patients suspected of being at risk of developing MS, and from healthy individuals,
(ii) the said blood mononuclear cells obtained from patients or healthy individuals are brought into contact with a polypeptide or a recombinant protein, as identified in SEQ ID NO:2, or a fragment of the said polypeptide or of the said protein, and
(iii) the said expansion and optionally co-expansion or the said loss and optionally co-reduction are detected using the blood mononuclear cells of step (i).

21. Method according to Claim 20, **characterized in that** the said polypeptide or the said protein is encoded by an env gene, as identified in SEQ ID NO:1.

22. Method of evaluating the efficacy of an agent or a composition for inhibiting, in a biological sample, a superantigen activity induced by the MSRV-1 retrovirus or a variant of MSRV-1, by expressing its env gene or a fragment of the said env gene, the said MSRV-1 env gene comprising the sequence designated by the reference SEQ ID NO:1 and the env gene of the said variant exhibiting at least 90% homology with the MSRV-1 env gene, **characterized in that**
(i) a culture supernatant of blood mononuclear cells or of plexus choroid cells or of leptomeningeal cells, the said cells being obtained from patients suffering from an autoimmune disease, or from cells of an established cell line, such as the cell lines PLI-2 and LM7PC, is brought into contact with blood mononuclear cells obtained from healthy donors in the presence of the said agent or of the said composition at predefined doses, and
(ii) the inhibition of the said expansion and optionally co-expansion or the inhibition of the said loss and optionally co-reduction of the lymphocytes carrying at least one determinant chosen from the Vβs 16, 7 and 17 are detected using a ligand as described in Claim 14 or an amplification combined with an electrophoresis as described in Claim 15.

23. Method according to Claim 22, **characterized in that** the cells are obtained from a patient suffering from multiple sclerosis.

24. Method according to Claim 22 or 23, **characterized in that** the blood mononuclear cells obtained from patients suffering from MS are chosen from B lymphocytes and monocytes.

25. Method for evaluating the prophylactic and/or therapeutic efficacy of an agent or a composition toward multiple sclerosis, and/or a predisposition to multiple sclerosis, **characterized in that** inhibition of a superantigen activity is identified in a biological sample as described in Claims 22 to 24.

26. Method according to Claim 25, **characterized in that** the inhibition of the predominant loss of lymphocytes carrying a determinant Vβ16 and the co-reduction of lymphocytes carrying Vβs 7 and 17 is identified.

27. Method according to Claim 25, **characterized in that** the inhibition of the predominant expansion of lymphocytes carrying a determinant Vβ16 and the co-expansion of lymphocytes carrying Vβs 3 and 12 is identified.

28. Method according to any one of Claims 25 to 27, **characterized in that** the cells are obtained from a patient suffering from multiple sclerosis.

29. Method according to any one of Claims 25 to 28, **characterized in that** the blood mononuclear cells obtained from patients suffering from MS are chosen from B lymphocytes and monocytes.

30. Method for identifying substances capable of blocking the transcription and/or translation of the human retrovirus MSRV-1 or a variant of MSRV-1, inducing a superantigen activity in multiple sclerosis, by expressing its env gene or a fragment of the said env gene, the said MSRV-1 env gene comprising the sequence designated by the reference SEQ ID NO:1 and the env gene of the said variant exhibiting at least 90% homology with the MSRV-1 env gene, according to which method, the substance is brought into contact with cells expressing a retroviral polypeptide identified in SEQ ID NO:2, or a fragment of the said polypeptide and having a superantigen activity, and a loss or reduction of the superantigen activity is detected as described in Claims 1 to 5.

31. Kit for screening substances capable of blocking the superantigen activity of the MSRV-1 retrovirus or a variant of MSRV-1 inducing the said activity by expressing its env gene or a fragment of the said env gene, the said MSRV-1 env gene comprising the sequence designated by the reference SEQ ID NO:1 and the env gene of the said variant exhibiting at least 90% homology with the MSRV-1 env gene, towards multiple sclerosis, or capable of blocking the transcription and/or translation of the said retrovirus, the said kit comprising:
cells expressing, at their surface, class II MHC products, transformed with and functionally expressing the retroviral superantigen comprising at least the fragment designed by the reference SEQ ID NO:2,
cells carrying chains of the receptor of a Vβ or of Vβs stimulated by the retroviral superantigen, the said cells being chosen from lymphocytes carrying a determinant Vβ16 and/or Vβ17 and lymphocytes carrying a determinant Vβ7, and
means for detecting a loss or reduction of superantigen activity as described in Claims 1 to 5, the said means being chosen from a ligand as described in Claim 14 and an amplification combined with an electrophoresis as described in Claim 15.

## Patentansprüche

1. Verfahren zum Nachweis von Multipler Sklerose oder einer Prädisposition für die Entwicklung von Multipler Sklerose in einer biologischen Probe, **dadurch gekennzeichnet, dass** eine Superantigen-Aktivität nachgewiesen wird, die durch das Produkt der Expression des MSRV-1-env-Gens oder eines Fragments des MSRV-1-env-Gens induziert wird, wobei das MSRV-1-env-Gen die in SEQ ID NR: 1 dargestellte Sequenz umfasst oder daraus besteht, oder dass eine Superantigen-Aktivität nachgewiesen wird, die durch das Produkt der Expression des env-Gens einer Variante von MSRV-1 oder eines Fragments des env-Gens der Variante induziert wird, wobei das env-Gen der Variante eine Homologie von mindestens 90% mit dem MSRV-1-env-Gen besitzt, indem eine Vermehrung oder ein Verlust von Lymphozyten nachgewiesen wird, die aus Lymphozyten mit einer Vβ16- und/oder Vβ17-Determinante und Lymphozyten mit einer Vβ7-Determinante ausgewählt sind, wobei die Vermehrung oder der Verlust mindestens 31% beträgt.

2. Verfahren nach Anspruch 1, **dadurch gekennzeichnet, dass** eine Vermehrung von Lymphozyten mit einer Vβ16-Determinante nachgewiesen wird.

3. Verfahren nach Anspruch 1, **dadurch gekennzeichnet, dass** ein Verlust von Lymphozyten mit einer Vβ16-Determinante nachgewiesen wird.

4. Verfahren nach Anspruch 2, **dadurch gekennzeichnet, dass** eine Vermehrung von Lymphozyten mit einer Vβ16-Determinante und eine gleichzeitige Vermehrung von Lymphozyten mit einem Vβ, ausgewählt aus mindestens einem von Vβ2, 3, 7, 8, 12, 14, 17 und 22 und vorzugsweise Vβ3 und 12, nachgewiesen wird.

5. Verfahren nach Anspruch 3, **dadurch gekennzeichnet, dass** ein Verlust von Lymphozyten mit einer Vβ16-Determinante und ein gleichzeitiger Verlust von Lymphozyten mit einem Vβ, ausgewählt aus mindestens einem von Vβ2, 3, 7, 8, 12, 14, 17 und 22, vorzugsweise aus mindestens einem von Vβ7, 14 und 17 und vorteilhafterweise Vβ7 und 17, nachgewiesen wird.

6. Verfahren nach einem der Ansprüche 1 bis 5, **dadurch gekennzeichnet, dass** die Superantigen-Aktivität durch das Produkt der Expression des in SEQ ID NR: 1 dargestellten MSRV-1-*env*-Gens oder eines Fragments des MSRV-1-env-Gens induziert wird.

7. Verfahren nach einem der Ansprüche 1 bis 5, **dadurch gekennzeichnet, dass** die Superantigen-Aktivität durch das in SEQ ID NR: 2 dargestellte MSRV-1-Hüllprotein oder ein Fragment dieses Hüllproteins induziert wird.

8. Verfahren nach einem der Ansprüche 1 bis 7, **dadurch gekennzeichnet, dass** die biologische Probe aus mononukleären Blutzellen ausgewählt ist.

9. Verfahren nach einem der Ansprüche 1 bis 7, **dadurch gekennzeichnet, dass** das Produkt der Expression des env-Gens von MSRV-1 oder des Fragments des env-Gens von MSRV-1 oder der MSRV-1-Variante aus mit MSRV-1 oder der MSRV-1-Variante infizierten Zellen, die aus Plexus-choroidei-Zellen, Leptomeninx-Zellen, Zellen etablierter Zelllinien, wie den Zellen der Zelllinien PLI-2, hinterlegt bei der ECACC am 22. Juli 1992 unter der Nummer 92072201, und LM7PC, hinterlegt bei der ECACC am 8. Januar 1993 unter der Nummer 93010817 kraft des Budapester Vertrags, ausgewählt sind, oder ausgehend von einem Expressionsvektor erhalten wird, der das env-Gen oder ein Fragment des env-Gens von MSRV-1 oder der MSRV-1-Variante umfasst, oder **dadurch**, dass das Expressionsprodukt des env-Gens oder des Fragments des env-Gens von MSRV-1 oder der MSRV-1-Variante ein Polypeptidmolekül ist, das aus einem Protein oder einem Fragment des Proteins ausgewählt ist, das SEQ ID NR: 2 umfasst oder daraus besteht.

10. Verfahren nach einem der Ansprüche 1 bis 9, **gekennzeichnet durch**:
(i) Entnahme eines Kulturüberstands von mononukleären Blutzellen oder Plexus-choroidei-Zellen oder Leptomeninx-Zellen, wobei diese Zellen von Patienten stammen, die von Multipler Sklerose betroffen sind oder von denen angenommen wird, dass sie eine Prädisposition für die Entwicklung von Multipler Sklerose haben, oder von einer etablierten Zelllinie, wie Zellen der Zelllinien PLI-2, hinterlegt bei der ECACC am 22. Juli 1992 unter der Nummer 92072201, und LM7PC, hinterlegt bei der ECACC am 8. Januar 1993 unter der Nummer 93010817 kraft des Budapester Vertrags;
(ii) In-Kontakt-Bringen des Kulturüberstands oder eines Teils des Kulturüberstands mit einer Reihe mononukleären Blutzellen, die von gesunden Spendern stammen, und
(iii) Nachweisen der Vermehrung und gegebenenfalls einer gleichzeitigen Vermehrung oder des Verlusts und gegebenenfalls gleichzeitigen Verlusts der mononukleären Blutzellen des Schrittes (ii).

11. Verfahren nach Anspruch 10, **dadurch gekennzeichnet, dass** die von Patienten, die von Multipler Sklerose (MS) betroffen sind, stammenden mononukleären Blutzellen aus Monozyten und B-Lymphozyten ausgewählt sind und die von gesunden Spendern stammenden mononukleären Blutzellen aus T-Lymphozyten ausgewählt sind.

12. Verfahren nach einem der Ansprüche 1 bis 9, **gekennzeichnet durch**:
(i) Entnahme mononukleärer Blutzellen, wobei diese Zellen von Patienten, die von Multipler Sklerose betroffen sind, oder von Patienten, von denen angenommen wird, dass sie eine Prädisposition für die Entwicklung von Multipler Sklerose haben, und von gesunden Individuen stammen,
(ii) In-Kontakt-Bringen der von Patienten oder gesunden Individuen stammenden mononukleären Blutzellen mit Kulturüberständen oder einer Fraktion von Kulturüberstand von Zellen, die aus mononukleären Blutzellen, Plexus-choroidei-Zellen und Leptomeninx-Zellen, von etablierten Zelllinien stammenden Zellen, wie Zellen der Zelllinien PLI-2, hinterlegt bei der ECACC am 22. Juli 1992 unter der Nummer 92072201, und LM7PC, hinterlegt bei der ECACC am 8. Januar 1993 unter der Nummer 93010817 kraft des Budapester Vertrags, ausgewählt sind, und
(iii) Nachweisen der Vermehrung und gegebenenfalls gleichzeitigen Vermehrung oder des Verlusts und gegebenenfalls der gleichzeitigen Verringerung ausgehend von den mononukleären Blutzellen des Schrittes (i).

13. Verfahren nach den Ansprüchen 10, 11 und 12, **dadurch gekennzeichnet, dass** diese Vermehrung und gegebenenfalls gleichzeitige Vermehrung durch Verwendung von Liganden nachgewiesen wird, wobei jeder Ligand für eine aus Vβ16, 2, 3, 7, 8, 12, 14, 17 und 22, vorzugsweise Vβ16, 3 und 12, ausgewählte Determinante spezifisch ist, und dass dieser Verlust und gegebenenfalls die gleichzeitige Verringerung durch Verwendung von Liganden nachgewiesen wird, wobei jeder Ligand für eine aus Vβ16, 2, 3, 7, 8, 12, 14, 17 und 22, vorzugsweise Vβ16, 7, 14 und 17 ausgewählte Determinante spezifisch ist.

14. Verfahren nach Anspruch 13, **dadurch gekennzeichnet, dass** der Ligand ein Antikörper, vorzugsweise ein monoklonaler Antikörper, oder ein Antikörperfragment ist.

15. Verfahren nach Anspruch 10, 11 und 12, **dadurch gekennzeichnet, dass** zum Nachweis der Vermehrung und gegebenenfalls gleichzeitigen Vermehrung oder des Verlusts und gegebenenfalls der gleichzeitigen Verringerung Folgendes durchgeführt wird:
(i) eine Extraktion von Gesamt-RNA mononukleärer Blutzellen, die mit MS-Kulturüberstand oder einer Fraktion von MS-Kulturüberstand und mit Referenzkulturüberstand oder einer Fraktion von Referenzkulturüberstand zusammengebracht wurden,
(ii) eine reverse Transkription der RNA,
(iii) eine spezifische Amplifikation jeder Vβ-Familie mithilfe eines bestimmten Primer-Paars,
(iv) eine Markierung der erhaltenen Amplifikationsprodukte mit jedem geeigneten Marker,
(v) eine Elektrophorese der Amplifikationsprodukte und eine Analyse der erhaltenen Elektrophoreseprofile mithilfe eines geeigneten Detektors.

16. Verfahren nach Anspruch 15, **dadurch gekennzeichnet, dass** die von Patienten, die von MS betroffen sind, stammenden mononukleären Blutzellen aus Lymphozyten ausgewählt sind.

17. Verfahren nach einem der Ansprüche 1 bis 16, **dadurch gekennzeichnet, dass** ferner mindestens einer der folgenden Parameter nachgewiesen wird:
eine Stimulation der Produktion von Cytokinen, wie Interleukin-6 (IL-6) und Interferon-γ (INF-γ),
eine Induktion der Zellapoptose.

18. Verfahren nach Anspruch 17, **dadurch gekennzeichnet, dass** die beiden Parameter in Verbindung miteinander nachgewiesen werden.

19. Verfahren nach einem der Ansprüche 1 bis 9, **gekennzeichnet durch**
(i) Produktion oder Synthese eines rekombinanten Proteins, wie in SEQ ID NR: 2 dargestellt, oder eines Fragments des Proteins,
(ii) In-Kontakt-Bringen des Proteins oder Fragments mit einer Reihe von, vorzugsweise mindestens drei, Kulturen mononukleärer Blutzellen, die von gesunden Spendern stammen, und
(iii) Nachweisen der Vermehrung und gegebenenfalls einer gleichzeitigen Vermehrung oder des Verlusts und gegebenenfalls der gleichzeitigen Verringerung der mononukleären Blutzellen des Schrittes (ii).

20. Verfahren nach einem der Ansprüche 1 bis 9, **gekennzeichnet durch**:
(i) Entnahme mononukleärer Blutzellen, wobei diese Zellen von Patienten, die von MS betroffen sind, oder von Patienten, von denen angenommen wird, dass sie ein Risiko für die Entwicklung von MS besitzen, und von gesunden Individuen stammen,
(ii) In-Kontakt-Bringen der von Patienten oder gesunden Individuen stammenden mononukleären Blutzellen mit einem Polypeptid oder einem rekombinanten Protein, wie in SEQ ID NR: 2 dargestellt, oder einem Fragment des Polypeptids oder Proteins und
(iii) Nachweisen der Vermehrung und gegebenenfalls gleichzeitigen Vermehrung oder des Verlusts und gegebenenfalls der gleichzeitigen Verringerung ausgehend von den mononukleären Blutzellen des Schrittes (i).

21. Verfahren nach Anspruch 20, **dadurch gekennzeichnet, dass** das Polypeptid oder Protein von einem env-Gen, wie in SEQ ID NR: 1 dargestellt, codiert wird.

22. Verfahren zur Untersuchung der Wirksamkeit eines Mittels oder einer Zusammensetzung zur Hemmung einer Superantigen-Aktivität in einer biologischen Probe, die durch das MSRV-1-Retrovirus oder eine MSRV-1-Variante durch Expression seines/ihres env-Gens oder eines Fragments des env-Gens induziert wird, wobei das MSRV-1-env-Gen die in SEQ ID NR: 1 dargestellte Sequenz umfasst und das env-Gen der Variante eine Homologie von mindestens 90% mit dem MSRV-1-env-Gen aufweist, **gekennzeichnet durch**:
i) In-Kontakt-Bringen eines Kulturüberstands von mononukleären Blutzellen oder Plexus-choroidei-Zellen oder Leptomeninx-Zellen, wobei diese Zellen von Patienten stammen, die von einer Autoimmunkrankheit betroffen sind, oder von Zellen einer etablierten Zelllinie, wie Zellen der Zelllinien PLI-2 und LM7PC, mit von gesunden Spendern stammenden mononukleären Blutzellen in Gegenwart des Mittels oder der Zusammensetzung in zuvor bestimmten Dosen und
(ii) Nachweisen der Hemmung der Vermehrung und gegebenenfalls gleichzeitigen Vermehrung oder der Hemmung des Verlusts und gegebenenfalls der gleichzeitigen Verringerung von Lymphozyten mit mindestens einer Determinante, ausgewählt aus Vβ16, 7 und 17, unter Verwendung eines Liganden nach Anspruch 14 oder einer Amplifikation in Verbindung mit einer Elektrophorese nach Anspruch 15.

23. Verfahren nach Anspruch 22, **dadurch gekennzeichnet, dass** die Zellen von einem von Multipler Sklerose betroffenen Patienten stammen.

24. Verfahren nach Anspruch 22 oder 23, **dadurch gekennzeichnet, dass** die von Patienten, die von MS betroffen sind, stammenden mononukleären Blutzellen aus B-Lymphozyten und Monozyten ausgewählt sind.

25. Verfahren zur Untersuchung der prophylaktischen und/oder therapeutischen Wirksamkeit eines Mittels oder einer Zusammensetzung gegen Multiple Sklerose und/oder eine Prädisposition für Multiple Sklerose, **dadurch gekennzeichnet, dass** eine Hemmung einer Superantigen-Aktivität in einer biologischen Probe, wie in den Ansprüchen 22 bis 24 beschrieben, nachgewiesen wird.

26. Verfahren nach Anspruch 25, **dadurch gekennzeichnet, dass** die Hemmung des hauptsächlichen Verlusts von Lymphozyten mit einer Vβ16-Determinante und der gleichzeitigen Verringerung von Lymphozyten mit Vβ7 und 17 nachgewiesen wird.

27. Verfahren nach Anspruch 25, **dadurch gekennzeichnet, dass** die Hemmung der hauptsächlichen Vermehrung von Lymphozyten mit einer Vβ16-Determinante und der gleichzeitigen Vermehrung von Lymphozyten mit Vβ3 und 12 nachgewiesen wird.

28. Verfahren nach einem der Ansprüche 25 bis 27, **dadurch gekennzeichnet, dass** die Zellen von einem von Multipler Sklerose betroffenen Patienten stammen.

29. Verfahren nach einem der Ansprüche 25 bis 28, **dadurch gekennzeichnet, dass** die von Patienten, die von Multipler Sklerose betroffen sind, stammenden mononukleären Blutzellen aus B-Lymphozyten und Monozyten ausgewählt sind.

30. Verfahren zur Identifikation von Substanzen, die die Transkription und/oder Translation des humanen MSRV-1-Retrovirus oder einer MSRV-1-Variante blockieren können, das/die bei Multipler Sklerose eine Superantigen-Aktivität durch Expression seines/ihres env-Gens oder eines Fragments des env-Gens induziert, wobei das MSRV-1-env-Gen die in SEQ ID NR: 1 dargestellte Sequenz umfasst und das env-Gen der Variante eine Homologie von mindestens 90% mit dem MSRV-1-env-Gen besitzt, umfassend:
In-Kontakt-Bringen der Substanz mit Zellen, die ein durch SEQ ID NR: 2 dargestelltes retrovirales Polypeptid oder ein Fragment des Polypeptids mit Superantigen-Aktivität exprimieren, und
Nachweisen eines Verlusts oder einer Verringerung der Superantigen-Aktivität, wie in den Ansprüchen 1 bis 5 beschrieben.

31. Kit zum Screening von Substanzen, die die Superantigen-Aktivität des MSRV-1-Retrovirus oder einer MSRV-1-Variante blockieren können, das/die diese Aktivität durch Expression seines/ihres env-Gens oder eines Fragments des env-Gens induziert, wobei das MSRV-1-env-Gen die in SEQ ID NR: 1 dargestellte Sequenz umfasst und das env-Gen der Variante eine Homologie von mindestens 90% mit dem MSRV-1-env-Gen besitzt, gegen Multiple Sklerose, oder die die Transkription und/oder Translation des Retrovirus blockieren können, wobei das Kit Folgendes umfasst:
Zellen, die an ihrer Oberfläche MHC-Klasse-II-Produkte exprimieren und mit dem retroviralen Superantigen, das mindestens das in SEQ ID NR: 2 dargestellt Fragment umfasst, transformiert sind und dieses funktionell exprimieren,
Zellen, die ein oder mehrere Rezeptor-Vβ-Ketten tragen und durch das retrovirale Superantigen stimuliert sind, wobei diese Zellen aus Lymphozyten mit einer Vβ16- und/oder Vβ17-Determinante und Lymphozyten mit einer Vβ7-Determinante ausgewählt sind, und
Mittel zum Nachweis eines Verlusts oder einer Verringerung der Superantigen-Aktivität, wie in den Ansprüchen 1 bis 5 beschrieben, wobei die Mittel aus einem Liganden nach Anspruch 14 und einer Amplifikation in Verbindung mit einer Elektrophorese nach Anspruch 15 ausgewählt sind.


### LISTE DE SEQUENCES

<110> BIO MERIEUX
<120> Procédé de détection d'une activité superantigénique dans un échantillon et composition thérapeutique ou prophylactique
<130> SEP21-SAg
<140>
   <141>
<150> FR-9903622
   <151> 1999-03-19
<150> FR-9913755
   <151> 1999-10-28
<160> 2
<170> PatentIn Ver. 2.1
<210> 1
   <211> 1629
   <212> ADN
   <213> Homo sapiens
<400> 1
   atggccctcc cttatcatac ttttctcttt actgttctct tacccccttt cgctctcact 60
   gcaccccctc catgctgctg tacaaccagt agctcccctt accaagagtt tctatggaga 120
   acgcggcttc ctggaaatat tgatgcccca tcatatagga gtttatctaa gggaaactcc 180
   accttcactg cccacaccca tatgccccgc aactgctata actctgccac tctttgcatg 240
   catgcaaata ctcattattg gacagggaaa atgattaatc ctagttgtcc tggaggactt 300
   ggagccactg tctgttggac ttacttcacc cataccagta tgtctgatgg gggtggaatt 360
   caaggtcagg caagagaaaa acaagtaaag gaagcaatct cccaactgac ccggggacat 420
   agcaccccta gcccctacaa aggactagtt ctctcaaaac tacatgaaac cctccgtacc 480
   catactcgcc tggtgagcct atttaatacc accctcactc ggctccatga ggtctcagcc 540
   caaaacccta ctaactgttg gatgtgcctc cccctgcact tcaggccata catttcaatc 600
   cctgttcctg aacaatggaa caacttcagc acagaaataa acaccacttc cgttttagta 660
   ggacctcttg tttccaatct ggaaataacc catacctcaa acctcacctg tgtaaaattt 720
   agcaatacta tagacacaac cagctcccaa tgcatcaggt gggtaacacc tcccacacga 780
   atagtctgcc taccctcagg aatatttttt gtctgtggta cctcagccta tcattgtttg 840
   aatggctctt cagaatctat gtgcttcctc tcattcttag tgccccctat gaccatctac 900
   actgaacaag atttatacaa tcatgtcgta cctaagcccc acaacaaaag agtacccatt 960
   cttccttttg ttatcagagc aggagtgcta ggcagactag gtactggcat tggcagtatc 1020
   acaacctcta ctcagttcta ctacaaacta tctcaagaaa taaatggtga catggaacag 1080
   gtcactgact ccctggtcac cttgcaagat caacttaact ccctagcagc agtagtcctt 1140
   caaaatcgaa gagctttaga cttgctaacc gccaaaagag ggggaacctg tttattttta 1200
   ggagaagaac gctgttatta tgttaatcaa tccagaattg tcactgagaa agttaaagaa 1260
   attcgagatc gaatacaatg tagagcagag gagcttcaaa acaccgaacg ctggggcctc 1320
   ctcagccaat ggatgccctg gactctcccc ttcttaggac ctctagcagc tataatattt 1380
   ttactcctct ttggaccctg tatcttcaac ttccttgtta agtttgtctc ttccagaatt 1440
   gaagctgtaa agctacaaat agttcttcaa atggaacccc agatgcagtc catgactaaa 1500
   atctaccgtg gacccctgga ccggcctgct agactatgct ctgatgttaa tgacattgaa 1560
   gtcacccctc ccgaggaaat ctcaactgca caacccctac tacactccaa ttcagtagga 1620
   agcagttag                                                        1629
<210> 2
   <211> 542
   <212> PRT
   <213> Homo sapiens
<400> 2
   Met Ala Leu Pro Tyr His Thr Phe Leu Phe Thr Val Leu Leu Pro Pro
    1               5                  10                  15
   Phe Ala Leu Thr Ala Pro Pro Pro Cys Cys Cys Thr Thr Ser Ser Ser
               20                  25                  30
   Pro Tyr Gln Glu Phe Leu Trp Arg Thr Arg Leu Pro Gly Asn Ile Asp
           35                  40                  45
   Ala Pro Ser Tyr Arg Ser Leu Ser Lys Gly Asn Ser Thr Phe Thr Ala
       50                  55                  60
   His Thr His Met Pro Arg Asn Cys Tyr Asn Ser Ala Thr Leu Cys Met
   65                  70                  75                  80
   His Ala Asn Thr His Tyr Trp Thr Gly Lys Met Ile Asn Pro Ser Cys
                   85                  90                  95
   Pro Gly Gly Leu Gly Ala Thr Val Cys Trp Thr Tyr Phe Thr His Thr
               100                 105                 110
   Ser Met Ser Asp Gly Gly Gly Ile Gln Gly Gln Ala Arg Glu Lys Gln
           115                 120                 125
   Val Lys Glu Ala Ile Ser Gln Leu Thr Arg Gly His Ser Thr Pro Ser
       130                 135                 140
   Pro Tyr Lys Gly Leu Val Leu Ser Lys Leu His Glu Thr Leu Arg Thr
   145                 150                 155                 160
   His Thr Arg Leu Val Ser Leu Phe Asn Thr Thr Leu Thr Arg Leu His
   165 170 175
   Glu Val Ser Ala Gln Asn Pro Thr Asn Cys Trp Met Cys Leu Pro Leu
   180 185 190
   His Phe Arg Pro Tyr Ile Ser Ile Pro Val Pro Glu Gln Trp Asn Asn
   195 200 205
   Phe Ser Thr Glu Ile Asn Thr Thr Ser Val Leu Val Gly Pro Leu Val
   210 215 220
   Ser Asn Leu Glu Ile Thr His Thr Ser Asn Leu Thr Cys Val Lys Phe
   225 230 235 240
   Ser Asn Thr Ile Asp Thr Thr Ser Ser Gln Cys Ile Arg Trp Val Thr
   245 250 255
   Pro Pro Thr Arg Ile Val Cys Leu Pro Ser Gly Ile Phe Phe Val Cys
   260 265 270
   Gly Thr Ser Ala Tyr His Cys Leu Asn Gly Ser Ser Glu Ser Met Cys
   275 280 285
   Phe Leu Ser Phe Leu Val Pro Pro Met Thr Ile Tyr Thr Glu Gln Asp
   290 295 300
   Leu Tyr Asn His Val Val Pro Lys Pro His Asn Lys Arg Val Pro Ile
   305 310 315 320
   Leu Pro Phe Val Ile Arg Ala Gly Val Leu Gly Arg Leu Gly Thr Gly
   325 330 335
   Ile Gly Ser Ile Thr Thr Ser Thr Gln Phe Tyr Tyr Lys Leu Ser Gln
   340 345 350
   Glu Ile Asn Gly Asp Met Glu Gln Val Thr Asp Ser Leu Val Thr Leu
   355 360 365
   Gln Asp Gln Leu Asn Ser Leu Ala Ala Val Val Leu Gln Asn Arg Arg
   370 375 380
   Ala Leu Asp Leu Leu Thr Ala Lys Arg Gly Gly Thr Cys Leu Phe Leu
   385 390 395 400
   Gly Glu Glu Arg Cys Tyr Tyr Val Asn Gln Ser Arg Ile Val Thr Glu
   405 410 415
   Lys Val Lys Glu Ile Arg Asp Arg Ile Gln Cys Arg Ala Glu Glu Leu
                       420                   425                   430
   Gln Asn Thr Glu Arg Trp Gly Leu Leu Ser Gln Trp Met Pro Trp Thr
           435                 440                     445
   Leu Pro Phe Leu Gly Pro Leu Ala Ala Ile Ile Phe Leu Leu Leu Phe
       450                 455                 460
   Gly Pro Cys Ile Phe Asn Phe Leu Val Lys Phe Val Ser Ser Arg Ile
   465                 470                 475                 480
   Glu Ala Val Lys Leu Gln Ile Val Leu Gln Met Glu Pro Gln Met Gln
                   485                 490                 495
   Ser Met Thr Lys Ile Tyr Arg Gly Pro Leu Asp Arg Pro Ala Arg Leu
               500                 505                 510
   Cys Ser Asp Val Asn Asp Ile Glu Val Thr Pro Pro Glu Glu Ile Ser
           515                 520                 525
   Thr Ala Gln Pro Leu Leu His Ser Asn Ser Val Gly Ser Ser
       530                 535                 540